# DEMANDE DE BREVET EUROPEEN

(11) **EP 1 797 867 A1**
(43) Date de publication de la demande: **20.06.2007**
(21) Numéro de dépôt: 06123821.8
(22) Date de dépôt: 10.11.2006
(51) Int. Cl.: A61K 8/72, A61K 8/81, A61Q 5/02, A61Q 19/00

(54) **Composition cosmétique ou pharmaceutique comprenant un copolymère comportant au moins un groupe ionisable, et procédé de traitement cosmétique**

(30) Priorité: 16.12.2005 FR 0553909
(71) Demandeur: L'Oréal, 75008 Paris (FR)
(72) Inventeur: Mougin, Nathalie, 75011, PARIS (FR); Chodorowski-Kimmes, Sandrine, 60300, SENLIS (FR); Schultze, Xavier, 77340, PONTAULT-COMBAULT (FR)
(74) Mandataire: Dodin, Catherine

(57) **Abrégé**

La présente invention concerne une composition cosmétique ou pharmaceutique comprenant, dans un milieu physiologiquement acceptable, au moins un (co)polymère comportant un squelette polymérique et au moins un groupe de jonction lié audit squelette polymérique et capable d'établir des liaisons H avec un ou plusieurs groupes de jonction partenaires, chaque appariement d'un groupe de jonction faisant intervenir au moins 3 liaisons H, ledit (co)polymère comportant au moins un groupe ionisable.

L'invention concerne également un procédé de traitement cosmétique employant ladite composition.

## Description

La présente invention concerne de manière générale une composition cosmétique de soin, de traitement et/ou de maquillage des matières kératiniques, présentant une persistance accrue d'au moins un effet cosmétique et/ou de soin apporté après application par la composition, une bonne adhésion après application sur les matières kératiniques, et permettant un démaquillage rapide, total et sélectif.

En particulier, cette composition peut être filmogène, et conduire après application à la filmification de la composition au cours du séchage,
En cosmétique, en effet, on cherche à obtenir un dépôt sur les cheveux, la peau, les cils et les ongles, souvent filmogène et apportant par exemple soit la mise en forme de la coiffure (cheveux); soit de la couleur (cheveux, maquillage); soit de la brillance, ou de la brillance et de la couleur (rouge à lèvres, mascaras, eye-liners et vernis à ongle); soit de la couleur et de la matité (fond de teint par exemple); soit du soin, si le dépôt contient un actif de soin tel qu'un hydratant, une protection contre les UV s'il contient des filtres solaires.
On cherche donc pour une persistance de l'effet apporté (couleur, brillance, matité, tenue, soin) une grande rémanence du dépôt cosmétique qui doit, en particulier résister aux agressions mécaniques telles que frottements, transferts par contact d'un autre objet ; résister à l'eau, à la sueur, aux larmes, à la pluie ; et résister au sébum et aux huiles.
Ceci est particulièrement vrai en maquillage pour les rouges à lèvres où l'on recherche la tenue prolongée de la couleur et de la brillance, et le non transfert de la couleur ; les vernis à ongles où l'on souhaite la brillance et la tenue du film; les fonds de teint, fards à paupière et poudres où l'on recherche également la tenue de la couleur apportée, en maintenant le plus longtemps possible la matité de la teinte initiale malgré la sécrétion de sébum et de sueur, ainsi que le non transfert.
En particulier, dans le cas des compositions de maquillage, il serait extrêmement souhaitable de disposer de polymères de nature chimique différente ou identique, facilement solubles ou dispersibles dans les huiles, les solvants cosmétiques et/ou les milieux aqueux, à concentration variable. Il serait également souhaitable que ces compositions, présentant une persistance accrue des effets apportés (notamment couleur, brillance, matité, soin), favorisent l'adhésion sur les matières kératiniques.
En coiffage plus particulièrement, la composition doit permettre la mise en forme du cheveu avec une bonne tenue dans le temps et dans les conditions de température et d'humidité de la vie quotidienne.

Pour résoudre ces problèmes, on a proposé l'utilisation dans des compositions cosmétiques de polymères capables d'induire au moins entre eux, une réticulation physique et, notamment des polymères comportant des groupes susceptibles de former des liaisons hydrogène.
Ainsi, la demande de brevet international WO02/098377 décrit une composition cosmétique de soin, de traitement, de maquillage des matières kératiniques comprenant, dans un milieu physiologiquement acceptable, un polymère linéaire, ramifié, cyclique ou dendrimère comportant un squelette polymérique et un groupe de jonction susceptible de former des liaisons hydrogène. Les squelettes polymériques peuvent comporter des motifs poly(oxyde d'éthylène) (POE).
Egalement, la demande de brevet international WO03/032929 décrit une composition de traitement capillaire contenant un polymère comprenant au moins deux groupes susceptibles de former des liaisons hydrogène.
Le brevet US5919441 décrit, quant à lui, des compositions cosmétiques comprenant une huile de silicone et comme agent gélifiant un polymère comprenant des groupements siloxanes et des groupes à liaisons hydrogène.

Véhiculer dans les milieux aqueux, notamment dans l'eau ou dans un milieu comportant de l'eau, des polymères tout en conservant les propriétés visées des compositions, et, plus particulièrement, celles qu'induisent lesdits polymères ajoutés, s'avère particulièrement difficile. Ceci est particulièrement le cas dans le domaine du coiffage, où un apport suffisant de coiffant par les polymères véhiculés par le milieu aqueux est nécessaire, tout en maintenant une bonne résistance dans le temps de l'effet.
Véhiculer dans les milieux non aqueux, notamment solvants organiques et huiles cosmétiques, des polymères s'avère également difficile, en particulier dans le domaine des produits de maquillage de type fond de teint ou rouge à lèvres, et également de type vernis à ongles.
Par l'expression "véhiculer dans un milieu", aqueux ou non aqueux, un polymère, on entend selon la présente invention que ledit polymère est soluble et/ou dispersible dans ledit milieu, à 25°C, à une concentration d'au moins 1% en poids.

Il existe donc le besoin de compositions cosmétiques qui conduisent après application sur les matières kératiniques à des dépôts permettant de concilier la persistance de l'effet cosmétique et/ou de soin, une bonne adhésion de la composition sur les matières kératiniques et un démaquillage complet, et qui comprennent des polymères qui sont aisément véhiculables dans le milieu physiologiquement acceptable de la composition.

L'invention a donc pour but de proposer une composition cosmétique, notamment de soin, de traitement cosmétique et/ou de maquillage des matières kératiniques, permettant de pallier les inconvénients des formulations traditionnelles.

La demanderesse a trouvé de manière surprenante qu'une composition cosmétique comprenant un polymère particulier comportant un squelette polymérique comprenant au moins deux motifs de répétition, au moins un groupe de jonction lié au squelette polymérique, ce groupe de jonction étant capable d'interagir avec tout groupe de jonction partenaire via la formation d'au moins 3 liaisons H (liaisons hydrogène), présentait une tenue améliorée sur un support kératinique, notamment sur les ongles.
Par ailleurs, cette composition permet d'obtenir la persistance d'au moins un effet cosmétique et/ou de soin apporté après application sur les matières kératiniques par la composition, voire d'accroître cet effet.

L'invention a donc pour objet une composition cosmétique comprenant, dans un milieu physiologiquement acceptable, au moins un (co)polymère comportant:
(a) un squelette polymérique -POL-,
(b) au moins un groupe de jonction (A) lié audit squelette polymérique et capable d'établir des liaisons H avec un ou plusieurs groupes de jonction partenaires, de nature chimique identique ou différente, chaque appariement d'un groupe de jonction faisant intervenir au moins trois liaisons H, de préférence au moins 4 liaisons H, préférentiellement 4 liaisons H, caractérisé en ce que ledit (co)polymère comprend au moins un groupe ionisable.

On a constaté que les (co)polymères selon l'invention étaient aisément solubles et/ou dispersibles dans le milieu de la composition et notamment dans les milieux aqueux et/ou huileux et/ou solvant organique usuellement employés en cosmétique.
Par ailleurs, le polymère selon l'invention permet d'obtenir une composition cosmétique dont l'élimination est facilitée, par rapport aux compositions de l'art antérieur, notamment décrites dans WO02/98377. Ceci est particulièrement important dans le cas des compositions capillaires, qui doivent être facilement éliminables au shampoing.
En outre, les compositions selon l'invention ne présentent pas de caractère collant.
Enfin, on a constaté que les compositions comprenant des polymères selon l'invention pouvaient présenter une faible viscosité, bien que comprenant des quantités importantes de polymères; ceci est notamment vrai pour les compositions en milieu aqueux. Ces compositions conservaient une bonne sprayabilité et permettaient l'obtention d'un dépôt filmifiable sur le support.

Par "squelette polymérique" aussi appelé POL, on entend au sens de l'invention, un homopolymère ou copolymère, désigné ci-après par (co)polymère, comportant au moins deux motifs de répétition identiques ou différents, liés de manière covalente. Ledit (co)polymère peut être linéaire, cyclique, ramifié, notamment en étoile, dendrimère ou greffé, ou bien réticulé; il peut s'agir d'un homopolymère ou d'un copolymère qui peut être statistique, alterné, séquencé ou autre.

Par "groupe de jonction" aussi appelé A, on entend au sens de l'invention, tout groupe fonctionnel comportant des groupes donneurs ou accepteurs de liaisons H, et capable d'établir au moins trois liaisons H, de préférence au moins 4 liaisons H, préférentiellement 4 liaisons H, avec un groupe de jonction partenaire, identique ou non.
Les groupes de jonction (A) peuvent être latéraux au squelette polymérique (en ramification latérale), et/ou aux extrémités dudit squelette polymérique, et/ou dans la chaîne formant ledit squelette polymérique.
Ils peuvent être répartis de façon aléatoire ou contrôlée dans la chaîne.

Par " groupe de jonction partenaire", on entend au sens de l'invention, tout groupe de jonction d'un (co)polymère selon l'invention pouvant établir des liaisons H avec un ou plusieurs groupes de jonction d'un même ou d'un autre (co)polymère selon l'invention. Les groupes de jonction peuvent être de nature chimique identique ou différente. S'ils sont identiques, ils peuvent alors établir des liaisons H entre eux et sont alors appelés groupes de jonction auto-complémentaires. S'ils sont différents, ils sont choisis de telle façon qu'ils soient complémentaires vis à vis des interactions H.
L'utilisation de tels (co)polymères dans une composition cosmétique conduit, après application de cette composition sur les matières kératiniques, à la formation d'un polymère supramoléculaire.

Par "polymère supramoléculaire", on entend au sens de l'invention, une chaîne
ou un réseau polymérique formé de l'assemblage d'un (co)polymère selon l'invention avec au moins un autre (co)polymère selon l'invention, identique ou différent, chaque assemblage comprenant au moins une paire de groupes de jonction appariés, identiques ou différents.

Par "paire de groupes de jonction appariés", on entend au sens de l'invention, deux groupes de jonction dont chacun peut être porté ou non par un même (co)polymère selon l'invention, les deux groupes étant reliés ensemble via au moins trois liaisons H, de préférence au moins 4 liaisons H, préférentiellement 4 liaisons H.
Ainsi le polymère supramoléculaire présentera des points de réticulations physiques assurés par les liaisons H entre ces paires de groupes de jonction. La réticulation physique assurera le maintien et la persistance de l'effet cosmétique et/ou de soin de manière analogue à la réticulation chimique, tout en permettant la réversibilité, c'est à dire la possibilité d'éliminer totalement le dépôt soit par un démaquillant spécifique, soit par la température, soit par tout autre moyen, ce que ne permet pas la réticulation chimique.

Par "groupe ionisable", on entend au sens de l'invention, tout groupe qui, soit par sa nature chimique propre, soit en fonction du milieu et/ou du pH du milieu
dans lequel il se trouve, peut être sous forme ionique. Selon sa nature chimique, il peut être cationisable, anionisable, ou amphotère. Ceci inclut également les groupes ioniques de type ammonium quaternaire tétra N-substitués.

Les (co)polymères selon l'invention peuvent être définis comme résultant de l'homopolymérisation ou de la copolymérisation de monomères de formule (I) et facultativement de monomères de formule (II) selon le schéma réactionnel : 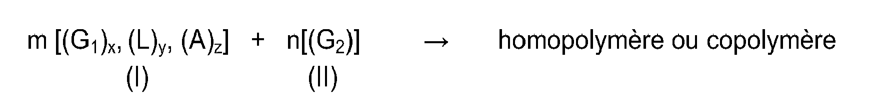 dans lequel :
- les groupements G₁, identiques ou différents, sont des groupements (co)polymérisables capables de former un lien covalent avec un autre groupement (co)polymérisable G₁ d'un autre monomère (I) et/ou avec un groupement (co)polymérisable porté par un monomère G2;
- les groupes A, identiques ou différents, sont des groupes de jonction capables de former au moins trois liaisons H, de préférence au moins 4 liaisons H, préférentiellement 4 liaisons H;
- les bras L, identiques ou différents, sont des bras de liaison divalents, y compris une liaison covalente simple, reliant un groupe de jonction A à un groupement G₁ ;
- x est un entier supérieur ou égal à 1, de préférence variant de 1 à 12, et mieux x est égal à 1 ou 2 ;
- y est un entier supérieur ou égal à 1, de préférence variant de 1 à 12, et mieux y est égal à 1 ou 2 ;
- z est un entier supérieur ou égal à 1, de préférence variant de 1 à 6, et mieux z est égal à 1 ;
- G₂, identiques ou différents, sont des monomères exempts de groupe de jonction A et comportant au moins un groupement (co)polymérisable capable de former un lien covalent avec un groupement (co)polymérisable G₁ d'un monomère (I) et/ou avec un groupement (co)polymérisable porté par un autre monomère G2, identique ou différent;
- m est le nombre de moles de monomères, identiques ou différents, de formule (I), homopolymérisés ou copolymérisés et est un entier allant de 1 à 12, de préférence compris entre 2 et 8, mieux entre 2 et 6 ;
- n est le nombre de moles de monomères, identiques ou différents, de formule (II), homopolymérisés ou copolymérisés, et est un entier allant de 0 à 20000, de préférence de 1 à 10000 et mieux de 1 à 5000 ; et m+n ≥ 2, de préférence ≥ 4; étant entendu que l'un au moins des groupes A et/ou groupements G1 et/ou bras L et/ou monomère G2, comporte au moins un groupe ionisable.

Il est clair que, dans les monomères de formule (I), identiques ou différents, lorsque ceux-ci comportent plusieurs groupements G₁ et/ou plusieurs bras de liaison L et/ou plusieurs groupes de jonction A, ceux-ci peuvent être identiques ou différents.

De préférence, les (co)polymères selon l'invention peuvent être formés par réaction d'au moins un monomère de formule G₁-L-A et/ou G₁-L-A-L-G₁ et/ou G₁-L(A)-G₁ avec au moins un monomère de formule G₂, les groupements G₁, les bras de liaison L, les groupes de jonction A et les monomères G2 pouvant être identiques ou différents et étant définis comme précédemment.
Dans cette définition, G₁-L(A)-G₁ désigne spécifiquement des monomères dans lesquels le groupe de jonction A est lié par une seule extrémité au groupe de liaison L et G₁-L-A-L-G₁ désigne spécifiquement des monomères dans lesquels le groupe de jonction A est lié à deux bras de liaison L, identiques ou différents, à ses deux extrémités.

Lorsque les (co)polymères selon l'invention sont formés par réaction d'un mélange de monomères (I) et (II), ils résultent de préférence de la copolymérisation d'au moins 2 moles de monomères (I) et d'au moins 1 mole de monomère (II).
Dans le cas des (co)polymères résultant de l'homo- ou copolymérisation de monomères (I), ces (co)polymères comportent de préférence une mole de groupe de jonction (A) par unité monomère.
Dans le cas de copolymérisation d'un mélange de monomères de formule (I) ou de copolymérisation d'un mélange de monomères de formule (I) et de formule (II), ces copolymères comportent de préférence deux ou plus de deux moles de groupes de jonction (A), de préférence 4 et mieux 6 moles de groupes de jonction (A) par chaîne de copolymères.
De manière avantageuse, les (co)polymères ont un squelette polymérique ayant un degré de polymérisation allant de 2 à 20000, et de préférence de 5 à 10000 et mieux de 10 à 5000.

De préférence également, la masse moléculaire moyenne en nombre (Mn) des (co)polymères selon l'invention est comprise entre 1000 et 3 000 000, notamment 5 000 et 1 000 000, et de préférence entre 8 000 et 500 000.

On a constaté que l'utilisation de tels (co)polymères, seuls ou en mélange avec des (co)polymères selon l'invention, comportant au moins un groupe de jonction, dans une composition cosmétique peut conduire, après application de la composition sur les matières kératiniques, soit à la formation d'un (co)polymère supramoléculaire sous forme de réseau tridimensionnel réticulé physiquement, soit à la formation d'un (co)polymère supramoléculaire linéaire sous forme d'une longue chaîne polymère, généralement de masse moléculaire élevée, résultant de la connection physiques des (co)polymères de l'invention entre eux.

### Structure générale des (co)polymères selon l'invention

Les (co)polymères selon l'invention peuvent avantageusement se présenter sous l'une des structures suivantes:
- (co)polymère linéaire et fonctionnalisé en α, ω avec des groupes de jonction (A);
- (co)polymère linéaire comportant plus de deux groupes de jonction, situés dans la chaîne et/ou à l'une ou aux deux extrémités et/ou en ramifications; et/ou
- (co)polymère ramifié avec des groupes de jonction dans la chaîne et/ou en ramification et/ou à l'une ou aux deux extrémités.

Il est clair que les (co)polymère selon l'invention peuvent présenter une seule de ces structures, ou un mélange de ces structures, en toutes proportions.
Les (co)polymères préférés selon l'invention sont les (co)polymères comportant 2 ou plus de 2 groupes de jonction, de préférence comportant 4 ou au moins 4 groupes et mieux 6 ou au moins 6 groupes de jonction.

### Définition générale des groupes de jonction A

Selon la présente invention, un groupe de jonction A est un groupe chimique, notamment carboné, capable de former au moins trois liaisons H, de préférence au moins 4 liaisons H, préférentiellement 4 liaisons H, et comportant de préférence au moins 3 hétéroatomes, identiques ou différents, de préférence au moins 4 hétéroatomes, voire 4 hétéroatomes, identiques ou différents, choisis dans le groupe constitué de O, N, S, P et F, de préférence parmi O, S et N.

Ces groupes de jonction peuvent comprendre par exemple au moins 3 groupes fonctionnels, de préférence au moins 4, voire 4 groupes fonctionnels, choisis parmi :

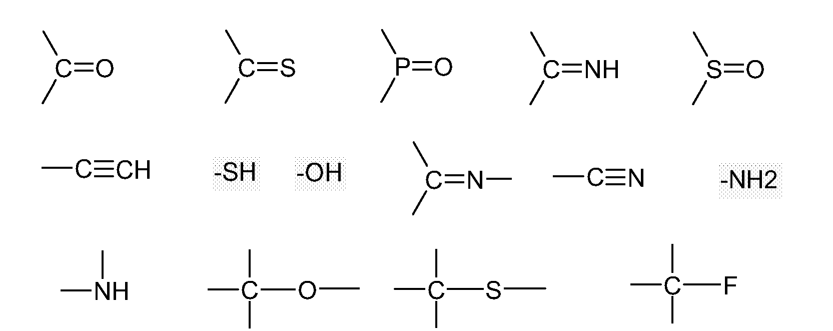

Ces groupes fonctionnels peuvent être classés en deux catégories :
- les groupes fonctionnels donneurs de liaisons H tels que les groupes : 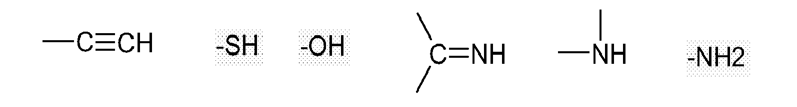
- les groupes fonctionnels accepteurs de liaisons H tels que les groupes :

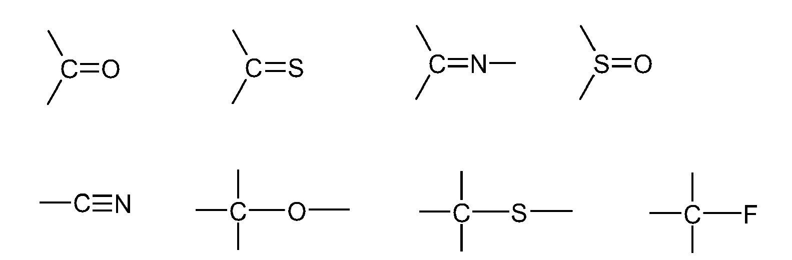

Les groupes de jonction A forment un élément structural de base comportant au moins 3 groupes, de préférence au moins 4 groupes, voire 4 groupes, capables d'établir des liaisons H.
Les éléments structuraux de base capables d'établir 3 ou 4 liaisons H peuvent être schématisés de la manière suivante : 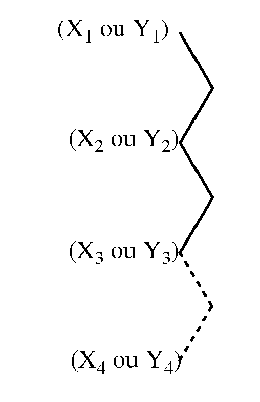 où Xᵢ est un groupe fonctionnel accepteur de liaisons H et Yᵢ est un groupe fonctionnel donneur de liaisons H.
Ainsi, chaque élément structural doit pouvoir établir des liaisons H avec un ou plusieurs éléments structuraux partenaires, identiques (c'est-à-dire autocomplémentaires) ou différents, de telle sorte que chaque appariement de deux éléments structuraux partenaires se fasse par formation d'au moins trois liaisons H, de préférence au moins 4 liaisons H, préférentiellement 4 liaisons H.
Un accepteur de protons X s'appariera avec un donneur de protons Y.
Plusieurs possibilités sont offertes, par exemple :
Appariement de :
XXXX avec YYYY ;
XXXY avec YYYX ;
XXYX avec YYXY ;
XYYX avec YXXY ;
XXYY avec YYXX autocomplémentaire ou non ;
XYXY avec YXYX autocomplémentaire ou non.

De préférence les groupes de jonction A peuvent établir 4 liaisons H avec un groupe partenaire identique (ou autocomplémentaire) parmi lesquelles 2 liaisons donneur (par exemple NH) et 2 liaisons accepteur (par exemple CO et - C=N-).
De préférence, les groupes de jonction comportent des cycles à 5 ou 6 atomes (cycles aromatiques ou hétérocycles insaturés) très souvent constitués d'atomes de C et/ou N et avec des doubles liaisons conjuguées pour stabiliser et diriger les interactions H.
De préférence encore, les groupes de jonction sont engagés dans des cycles à 6 atomes comprenant des C et/ou N et avec des doubles liaisons conjuguées pour stabiliser et diriger les interactions H.

De manière préférée, les groupes de jonction (A) sont capables d'établir au moins quatre liaisons H avec un même groupe de jonction partenaire (auto-complémentaire).

Les groupes de jonction (A) capables de former 3 ou 4 liaisons H peuvent être choisis parmi les familles suivantes, étant entendu que toutes les formes tautomériques sont inclues :
- (i) les aminopyrimidones de formule : 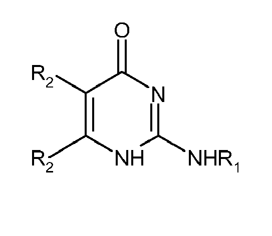
- (ii) les ureïdopyrimidones de formule : 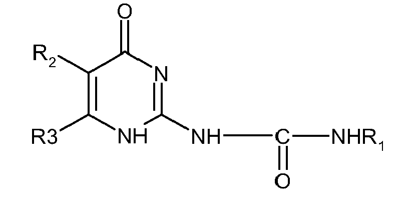
- (iii) les acylaminopyridines et notamment :
   - les monoacylaminopyridines de structure : 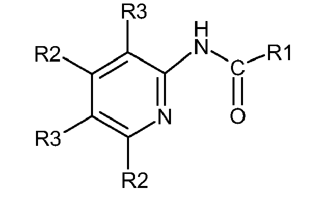
   - les di(acylamino)pyridines et plus particulièrement les 2,6-di(acylamino)pyridines de structure : 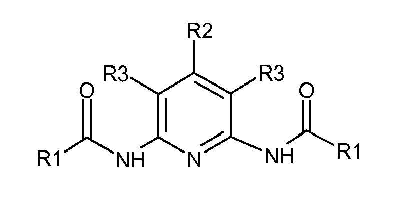
- (iv) les aminopyrimidines, et notamment :
   - les composés aminopyrimidines : 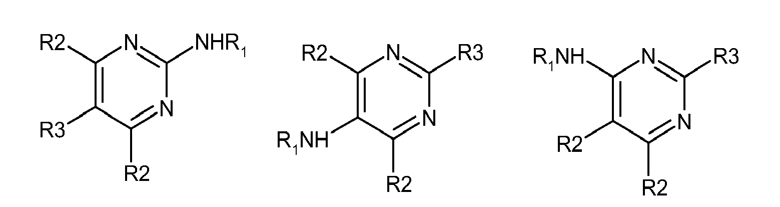
   - les composés diaminopyrimidines de structure : 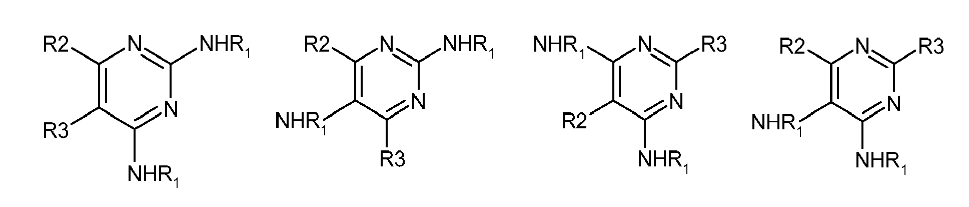
   - les composés triaminopyrimidines;
- (v) les uréïdotriazines, et notamment les mono-, di- et tri-uréïdotriazines, et en particulier les uréïdoaminotriazines de structure : 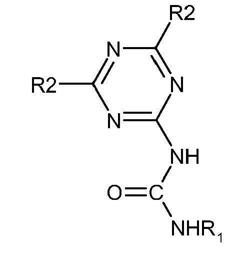
- (vi) les (acylamino)triazines, et notamment les mono-, di- et tri-acylamino triazines, éventuellement amino (mono-, di- ou tri-amino) et en particulier :
   - les di(acylamino)triazines de structure : 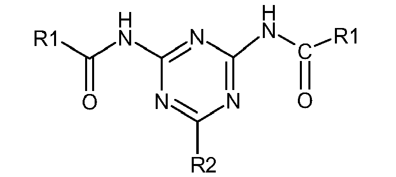
   - les acylamino, amino-triazines, (mono ou di- acylamino, et mono- ou di-amino) et notamment les composés de structure : 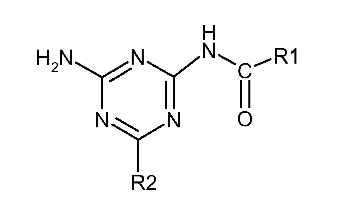
   - les acylaminotriazines de structure : 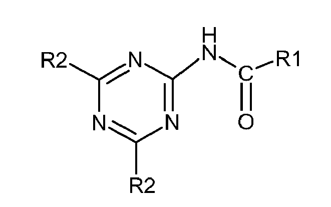
   - les tri-acylamino triazines,
- (vii) les aminotriazines et notamment :
   - les monoaminotriazines,
   - les 2,6-diamino-s-triazines de structure : 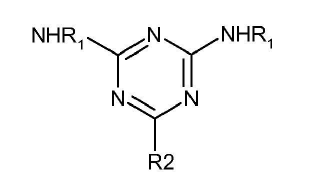
   - les composés triamino-s-triazines de structure : 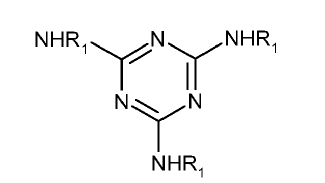
- (viii) les acylaminotriazoles de structure : 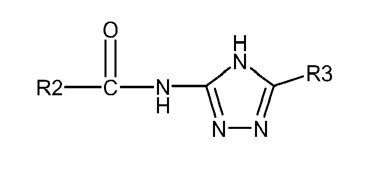
- (ix) les composés de la famille de l'acide urazoyl benzoïque de structure : 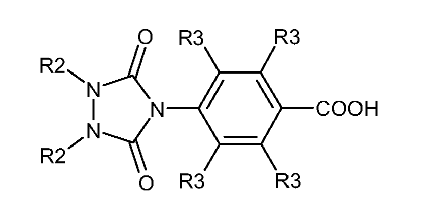
- (x) les phtalhydrazides de structure : 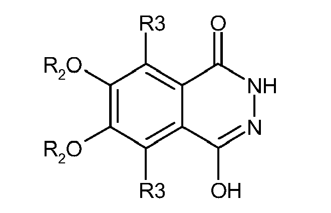
- (xi) les uraciles de structure : 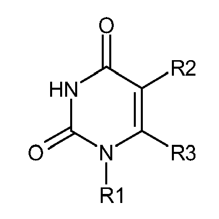
- (xii) les thymines de structure : 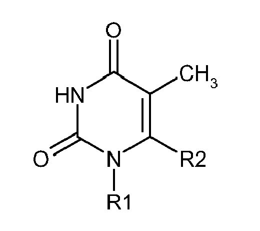
- (xiii) les succinimides de structure : 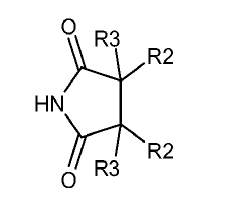
- (xiv) les glutarimides de structure : 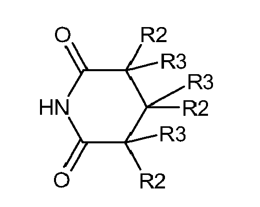
- (xv) les composés de la famille de l'acide cyanurique de structure : 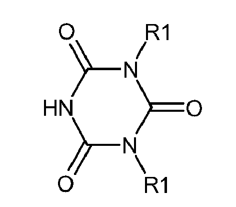
- (xvi) les maléimides : 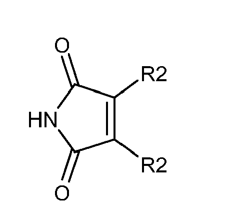
- (xvii) les composés de la famille de l'acide barbiturique, de structure : 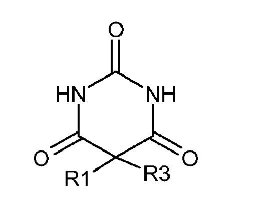
- (xviii) les composés de structure : 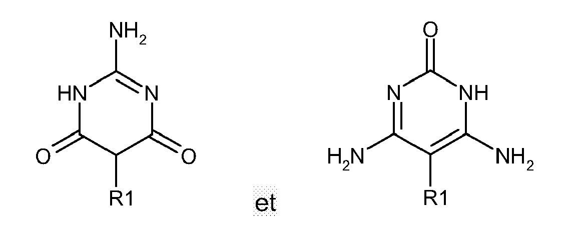
- (xix) les composés de la famille de l'acide triméllitique, de formule : 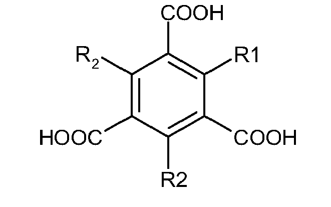
- (xx) les uréïdopyridines, notamment les mono- ou di-uréïdopyridines, et en particulier ceux de formule : 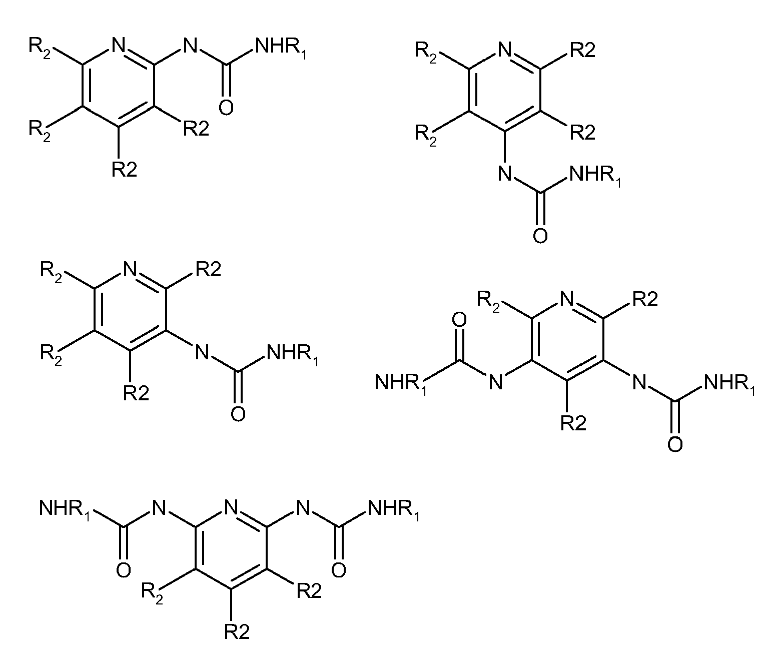
- (xxi) les carbamoylpyridines, de formule : 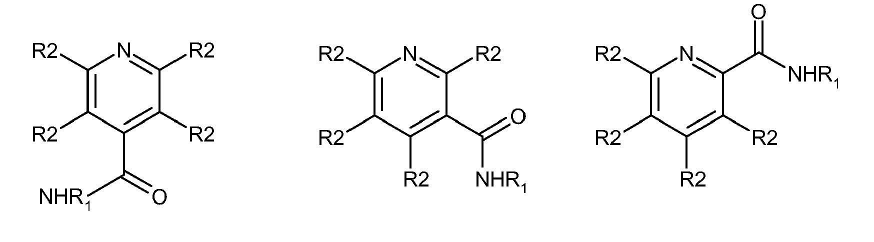
- (xxii) les adénines de formule : 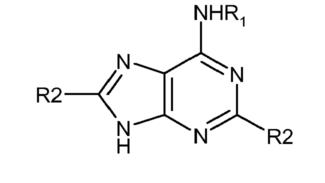
- (xxiii) les guanines de formule : 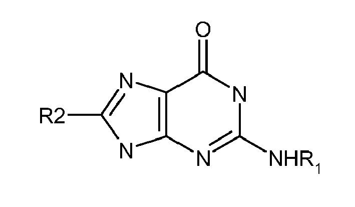
- (xxiv) les cytidines de formule :

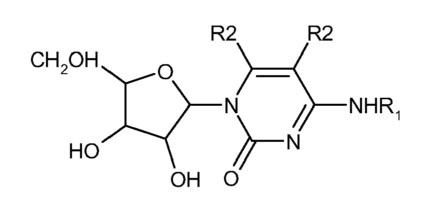

Dans l'ensemble de ces formules, la signification des radicaux est la suivante :
- (a) les radicaux R¹ identiques ou différents, représentent H, halogène et/ou un groupe carboné (notamment alkyle) monovalent, linéaire, ramifié ou cyclique, en C₁-C₆₀₀₀, saturé ou non, éventuellement aromatique, pouvant contenir un ou plusieurs hétéroatomes tels que O, S, N, P, Cl, Br, Cl, Br, F; ou une combinaison de ces significations.
   Le radical R¹ peut notamment être un groupe cycloalkyle en C₄-C₁₂; un groupe alkyle linéaire ou ramifié en C₁-C₃₀ ou un groupe aryle en C₄-C₁₂; éventuellement substitués par une fonction amino, ester et/ou hydroxy.
   De préférence, R₁ est un groupement : -C₄H₉, -phényle ; 1,4-nitrophényle, ou 1,2-éthylène, 1,6-héxylène, 1,4-butylène, 1,6-(2,4,4-triméthylhexylène), 1,4-(4-méthylpentylène), 1,5-(5-méthylhexylène), 1,6-(6-méthylheptylène), 1,5-(2,2,5-triméthylhexylène) ou 1,7-(3,7-diméthyloctylène); -isophorone-, 4,4'-méthylène biscyclohexylène, tolylène , 2-méthyl-1,3-phénylène, 4-méthyl-1,3-phénylène, 4,4-biphénylèneméthylène, et de préférence : -isophorone-, -(CH₂)₂-, -(CH₂)₆-, -CH₂CH(CH₃)-CH₂-C(CH₃)₂-CH₂-CH₂, 4,4'-méthylène biscyclohexylène, 2-méthyl-1,3-phénylène.
- (b) les radicaux R2, identiques ou différents au sein d'une même formule, représentent H, halogène (-BR, -Cl, -F), -OH, -N(R)2 (avec R étant H ou un radical alkyle, linéaire ou ramifié en C1-C12, de préférence en C1-C4 et mieux un radical méthyle ou éthyle); ou un groupe hydrocarboné monovalent, linéaire, ramifié ou cyclique, en C1-C6000, saturé ou non, éventuellement aromatique, pouvant contenir un ou plusieurs hétéroatomes tels que O, S, N, P, F; ou une combinaison de ces significations;
   Les radicaux R² peuvent notamment être CN, NH₂ ou bien :
   - un groupe alkyle en C₁-C₃₀ ;
   - un groupe cycloalkyle en C₄-C₁₂ ;
   - un groupe aryle en C₄-C₁₂ ;
   - un groupe aryl(C₄-C₁₂) alkyle en C₁-C₃₀
   - un groupe alcoxy en C₁-C₄ ;
   - un groupe arylalcoxy, en particulier un groupe aryle (C₁-C₄) alcoxy ;
   - un hétérocycle en C₄-C₁₂
   - un groupe thioalcoxy,
   - un groupe sulfoxy,
      ou leurs mélanges, ces groupes étant éventuellement substitués par une fonction amino, ester et/ou hydroxy.
      De préférence R² représente H, CH₃, C₁₃H₂₇, C₇H₁₅ ou phényle.
- (c) les radicaux R³, identiques ou différents au sein d'une même formule, représentent H ou un groupe hydrocarboné monovalent, linéaire, ramifié ou cyclique, en C₁-C₆₀₀₀, saturé ou non, éventuellement aromatique, pouvant contenir un ou plusieurs hétéroatomes tels que O, S, N, P, F; ou une combinaison de ces significations;
   Le radical R³ peut notamment être un groupe cycloalkyle en C₄-C₁₂; un groupe alkyle linéaire ou ramifié en C₁-C₃₀ ou un groupe aryle en C₄-C₁₂; éventuellement substitués par une fonction amino, ester et/ou hydroxy.

Dans l'ensemble de ces formules, il est bien entendu qu'au moins un, notamment un ou deux, des groupes R¹ et/ou R² est le point d'accroche du groupe de jonction A sur le squelette polymérique -POL-.
De préférence, ledit point d'accroche est porté par R¹ et/ou R² et de préférence lorsque le point d'accroche est unique, il est porté par le groupe R¹.

Les groupes de jonction (A) peuvent notamment être choisis parmi :
(a) les groupes de jonction (A) complémentaires et identiques c'est-à-dire auto-complémentaires, et notamment :
   - les aminopyrimidones, les uréïdopyrimidones,
   - les composés de la famille de l'acide triméllitique, ou de l'acide urazoyl benzoïque,
   - les acylaminopyridines, les uréïdopyridines, les carbamoylpyridines,
   - les acylaminotriazines, les uréidotriazines et notamment les uréïdoaminotriazines, les diamino-triazines,
   - les acylaminotriazoles,
   - les phtalhydrazides,
   - les composés de formule : 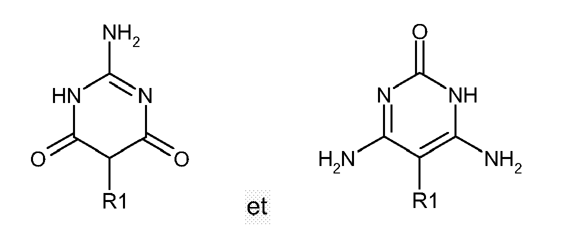 dans lesquelles R¹ est H ou un groupe hydrocarboné monovalent, linéaire, ramifié ou cyclique, en C₁-C₆₀₀₀, saturé ou non, éventuellement aromatique, pouvant contenir un ou plusieurs hétéroatomes tels que O, S, N, P, F.
(b) les groupes de jonction (A) complémentaires mais différents, et notamment:
   - adénine complémentaire de guanine,
   - cytidine complémentaire de thymine,
   - triamino-s-triazine complémentaire de uracile ou de succinimide ou de glutarimide ou d'acide cyanurique ou de thymine ou de maléimide ou de (di)aminopyrimidine ou d'acide barbiturique;
   - acylamino-amino-s-triazine complémentaire de uracile ou de succinimide ou de glutarimide ou d'acide cyanurique ou de thymine ou de maléimide ou de (di)aminopyrimidine ou d'acide barbiturique.

De manière préférée, les groupes de jonction A sont choisis parmi les groupes capables d'établir au moins trois liaisons H avec eux-mêmes (auto-complémentaire), notamment au moins quatre liaisons H avec eux-mêmes. Parmi ces groupes, on peut en particulier citer :
- les uréïdopyrimidones;
- les uréïdopyridines, les carbamoylpyridines;
- les acylamino-s-triazines et notamment les acyl-diamino-s-triazines;
- les uréidotriazines ;
- les phtalhydrazides;
- les composés de formule : 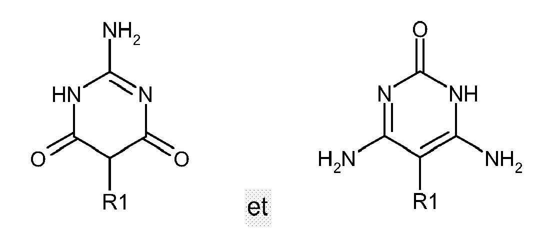
Dans lesquels les radicaux R1, R2 et R3 ont les significations données ci-dessus, en particulier les significations données en préférence.

Encore mieux, on peut citer à titre d'exemple préféré de groupes de jonction capables d'établir au moins 3 liaisons H avec eux mêmes, les groupes suivants:
- la 2-uréïdopyrimidone ;
- la 6-méthyl, 2-uréïdopyrimidone ;
- la diacyl de 2,6-diamino-s-triazine ;
- l'uréido-s-triazine ;
- les composés de formule : 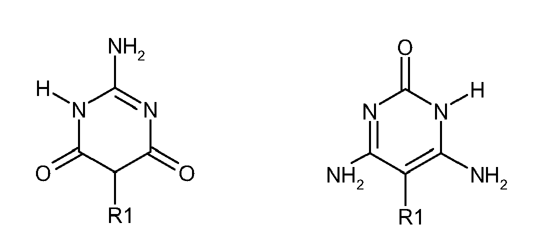
dans lesquels les radicaux R1, R2 et R3 ont les significations données ci-dessus, en particulier les significations données en préférence.

Comme indiqué précédemment, les groupes de jonction A sont liés aux groupements (co)polymérisables G₁ par l'intermédiaire d'au moins un bras de liaison L, de préférence un seul bras de liaison L.

Ledit bras de liaison L peut être une liaison covalente simple.
Ledit bras de liaison L se forme généralement lors de la réaction :
- soit entre une fonction réactive liée au groupe de jonction A avec une fonction réactive portée par le groupement (co)polymérisable G₁ ;
- soit entre une fonction réactive liée à un précurseur du groupe de jonction A avec une fonction réactive portée par le groupement (co)polymérisable G₁ pour former simultanément le groupe de jonction A et l'entité A-L-G₁;
les deux fonctions réactives étant bien entendu capables de réagir entre elles, et pouvant être liées directement ou par un segment divalent audit groupe de jonction A et/ou audit groupement G1 et/ou audit précurseur dudit groupe A.

Dans la présente invention, on entend par A le groupe de jonction sans sa fonction réactive.

Les fonctions réactives peuvent de préférence être choisies parmi les fonctions :
- isocyanate -N=C=O ;
- isothiocyanate -N=C=S ;
- acide ou ester carboxylique -COOR avec R étant H ou un radical alkyle, linéaire ou ramifié en C₁-C₁₂, de préférence en C₁-C₄ et mieux un radical méthyle ou éthyle;
- hydroxyle -OH;
- amine primaire, secondaire, ou tertiaire -N(R)₂, où R identique ou différent, est tel que défini ci-dessus;
- éthylénique -CR=C(R)₂ où R identique ou différent, est tel que défini ci-dessus;
- une fonction choisie parmi : 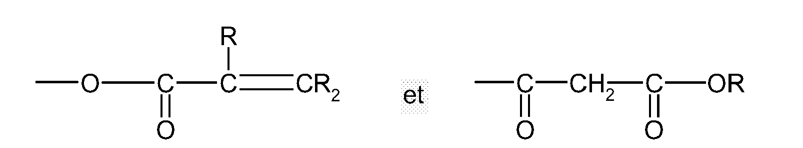 où R identique ou différent, est tel que défini ci-dessus;
- leurs formes salifiées ou activées; par exemple le groupe réactif OH peut être activé sous forme O-tosylate; le groupe acide carboxylique peut être activé sous forme de chlorure d'acide ou d'anhydride d'acide; on peut encore citer les activations par le carbonyldiimidazole tel que : 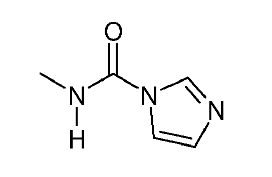
De préférence la fonction réactive précurseur du bras de liaison L est une fonction isocyanate, hydroxy ou amine, préférentiellement isocyanate.

Lorsque la fonction réactive est reliée par un segment divalent à A et/ou G1 et/ou au précurseur de A, ce segment peut être choisi parmi :
- les radicaux alkylènes, linéaires ou ramifiés, éventuellement substitués;
- les radicaux cycloalkylènes, éventuellement substitués ;
- les radicaux arylènes, éventuellement substitués ;
- les radicaux amino (-NH- ou -NR-) ou encore -O-, -SO-, -SO₂- ou -C(O)-;
ainsi que leurs combinaisons de même catégorie et/ou de catégories différentes, conduisant notamment aux radicaux cycloalkylènesalkylènes, biscycloalkylènes, biscycloalkylènesalkylènes, arylènealkylènes, bisphénylènesalkylènes, oxyalkylènes et aminoalkylènes.
Ces radicaux peuvent éventuellement être substitués, notamment par un ou plusieurs groupes alkyles en C₁-C₁₂, comprenant éventuellement des hétéroatomes choisis parmi O, N, S, F et P, et leurs combinaisons.

Parmi les radicaux alkylènes éventuellement substitués préférés, on peut citer les radicaux alkylènes en C₁-C₃₀, par exemple le radical méthylène -CH₂- et les radicaux poly(méthylène) de formule -(CH₂)ₙ- (n ≥ 2), tels que les radicaux éthylène, butylène, notamment 1,4-butylène, et 1,6-hexylène et les radicaux alkylènes ramifiés en C₃-C₁₀ tels que les radicaux 1,4-(4-méthyl pentylène), 1,6-(2,2,4-triméthyl hexylène), 1,5-(5-méthyl hexylène), 1,6-(6-méthyl heptylène), 1,5-(2,2,5-triméthyl hexylène), 1,7-(3,7-diméthyl octylène), 2,2-(diméthylpropylène) et 1,6-(2,4,4-triméthyl hexylène).
Parmi les radicaux cycloalkylènes éventuellement substitués préférés, on peut citer les radicaux cyclopentylène et cyclohexylène, éventuellement substitués notamment par des groupes alkyles.
Parmi les cycloalkylènesalkylènes préférés, on peut citer le radical isophorone de formule :

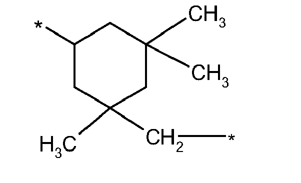

Parmi les radicaux biscycloalkylènealkylènes éventuellement substitués préférés, on peut citer les radicaux de formule : 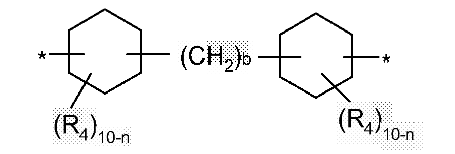 où b est un entier de 0 à 3, n un entier de 0 à 4; et R4, identique ou différent, représente H ou un radical alkyle en C1-C12, notamment un radical méthyle; on peut notamment citer la 4,4'-méthylène biscyclohexylène.

Parmi les radicaux arylènes éventuellement substitués préférés, on peut citer le radical phénylène, les radicaux tolylènes, notamment les radicaux 2,4- et 2,6-tolylène, et les radicaux naphtylènes, notamment 2,4-naphtylène ou 2,6-naphtylène.

Parmi les radicaux arylènealkylènes éventuellement substitués préférés, on peut citer les radicaux phénylène-alkylènes tels que le radical benzylène : 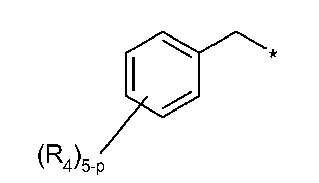 dans lequel p est un entier de 0 à 5.

Parmi les radicaux bisphénylènes alkylènes éventuellement substitués préférés, on peut citer (i) les radicaux de formule : 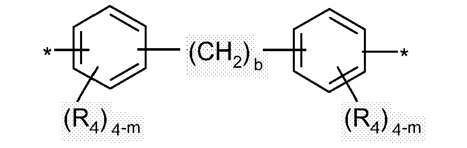 où b est un entier de 0 à 3, et m un entier de 0 à 4; tel que le radical bisphénylène et le radical 4,4'-méthylène bisphénylène,
et (ii) les radicaux de formule : 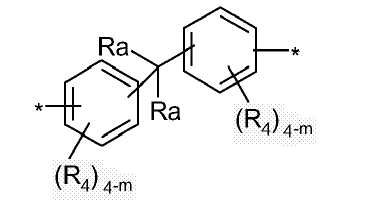 dans laquelle m un entier de 0 à 4, et les radicaux Rₐ, identiques ou différents, représentent H ou un radical alkyle en C₁-C₄, de préférence un radical méthyle.

Parmi les radicaux oxyalkylènes éventuellement substitués préférés, on peut citer les radicaux oxyde d'alkylène de formule -O-(R'O)_{y}- où R' identique ou différent, représente un radical alkylène, linéaire ou ramifié, en C₂-C₄, notamment éthylène ou propylène; et y est un entier de 1 à 500, de préférence de 1 à 200 et plus préféré de 5 à 100.

Dans un mode particulier de réalisation de l'invention, A peut porter au moins un groupe ionisable tel que défini ci-après.
En particulier, l'un au moins des radicaux R1, R2 et/ou R3 peut comporter un groupe ionisable.

### Définition des groupements (co)polymérisables G₁

Le groupement (co)polymérisable G₁ est un groupement chimique capable de réagir avec un groupement chimique, identique ou différent, porté par un autre groupement G₁ d'un autre monomère (I), identique ou non, et/ou avec un groupement chimique d'un monomère (II) de formule G₂, pour former un (co)polymère comportant au moins deux motifs de répétition.

Par "comportant au moins deux motifs de répétition", on entend selon la présente invention, une unité constitutive d'un homopolymère ou copolymère résultant de l'homopolymérisation ou copolymérisation d'au moins deux motifs monomères ou oligomères identiques ou différents.
Les fonctions homopolymérisables et/ou copolymérisables des groupements G₁ peuvent notamment être choisies parmi les fonctions (co)polymérisables par voie radicalaire ou anionique ou cationique, par polyaddition, par polycondensation, par ouverture de cycle, ou par tout autre mécanisme de (co)polymérisation.
Parmi les fonctions (co)polymérisables par voie radicalaire, anionique ou cationique, on peut citer les doubles liaisons éthyléniques, activées ou non, telles que les fonctions oléfiniques, les fonctions vinyliques, allyliques, (méth)acryliques, (méth)acrylamides et leurs combinaisons.
Parmi les fonctions polymérisables par polyaddition ou par polycondensation, on peut citer les fonctions hydroxyles, amines primaires et secondaires, esters, acides carboxyliques et isocyanates, activées ou non.
Parmi les fonctions polymérisables par ouverture de cycle anionique ou cationique, on peut citer les esters cycliques, les amides cycliques, les carbonates cycliques et les éthers cycliques.
On peut citer également les réactions entre les halogénures et les amines tertiaires.

Dans un mode particulier de réalisation de l'invention, G1 peut porter au moins un groupe ionisable tel que défini ci-après.

### Définition des bras de liaison L

Les bras de liaison L, identiques ou différents, sont d'une manière générale des groupes divalents reliant les groupes de jonction (A) aux groupements (co)polymérisables G₁.

Ces bras de liaison peuvent être une liaison covalente simple.

Ils peuvent également être des groupes carbonés divalents, saturés ou insaturés, linéaires, ramifiés ou cycliques (y compris aromatiques), notamment alkyles, ainsi que leurs combinaisons, ayant de 1 à 6000 atomes de carbone, notamment 1 à 30, et pouvant éventuellement comprendre un ou plusieurs, de préférence 1 à 5, hétéroatomes identiques ou différents choisis parmi O, N, S, P, F, de préférence O, N et S.
Le ou les hétéroatome(s), quand il(s) est(sont) présent(s), peut(vent) être dans la chaîne du bras de liaison L ou peuvent participer à un groupement de substitution sur la chaîne dudit bras de liaison, par exemple un groupement hydroxyle, ester, thiol, amine (NR₂ avec R identiques ou différents, représentant un alkyle linéaire en C₁-C₁₂, de préférence en C1-C4 et notamment un groupe méthyle ou éthyle), ou POE (polyoxyde d'éthylène).

Généralement, les bras de liaison L sont des groupes carbonés, notamment alkylènes (alkyles divalents) en C1-C30, pouvant comprendre des groupements fonctionnels choisis parmi :

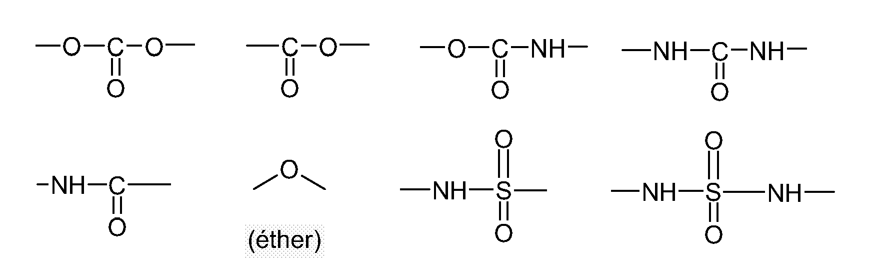

Ainsi, les groupements G-L-A-L-G sont de préférence du type : 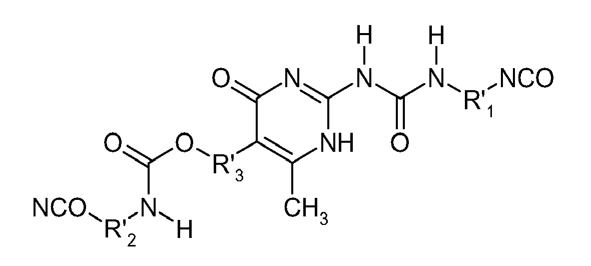 et les groupements A-L-G sont du type : 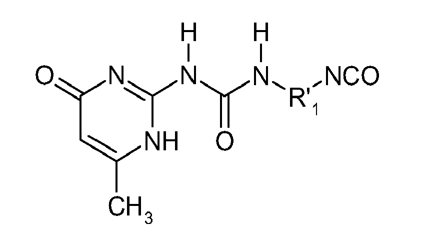 les radicaux R'₁, R'₂ et R'₃, identiques ou différents, représentant un groupe carboné divalent, notamment susceptible d'être choisi parmi 1,2-éthylène, 1,6-héxylène, 1,4-butylène, 1,6-(2,4,4-triméthylhexylène), 1,4-(4-méthylpentylène), 1,5-(5-méthylhexylène), 1,6-(6-méthylheptylène), 1,5-(2,2,5-triméthylhexylène), 1,7-(3,7-diméthyloctylène); -isophorone-, 4,4'-méthylènebiscyclohexylène, tolylène, 2-méthyl-1,3-phénylène, 4-méthyl-1,3-phénylène, 4,4-bisphénylèneméthylène.
De préférence, ils représentent, indépendamment l'un de l'autre, isophorone, - (CH₂)₂-, -(CH₂)₆-, -CH₂CH(CH₃)-CH₂-C(CH₃)₂-CH₂-CH₂, 4,4'-méthylènebiscyclohexylène, 2-méthyl-1,3-phénylène.
Préférentiellement, R'₁ = R'₂ = -isophorone- et R'₃ = -(CH₂)₂- .

Préférentiellement, le groupement G-L-A-L-G peut être de formule :

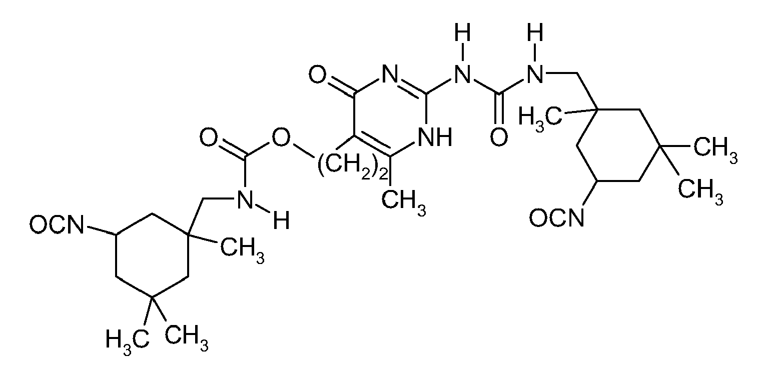

Les groupements (co)polymérisables G1 peuvent être principalement répartis en trois grandes classes, à savoir :
- les groupements (co)polymérisables comportant au moins une double liaison éthylénique capable de (co)polymériser par voie radicalaire, anionique ou cationique, par exemple un groupement CH₂=C(CH3)- ou CH₂=CH- ;
- les groupements (co)polymérisables par réaction de substitution ou d'addition, nucléophile ou électrophile, ou d'addition radicalaire et de préférence par polyaddition ou polycondensation, choisis par exemple parmi les groupements comportant une fonction hydroxyle (OH) ou hydroxyle activé tel que tosylate, thiol (SH), halogénure (Br, Cl), amine primaire ou secondaire (NH₂ ou NHR), ester (COOR), acide carboxylique (COOH), acide activé tel que COHal, isocyanate (NCO) protégé ou non, isothiocyanate (NCS), -C=C-, -C(O)H, -SiH, succinimide, oxazoline, acétal, hemiacétal, chlorotriazine, -SO₂Cl, époxyde; le radical R étant un alkyle en C1-6, de préférence méthyle.
- les groupements (co)polymérisables par ouverture, anionique ou cationique, de cycles, tels que des groupements comportant au moins un groupe éther cyclique, ester cyclique, amide cyclique ou carbonate cyclique.

Comme monomères de formule (I) particulièrement préférés, on peut citer :

- i) les monomères (co)polymérisables par voie radicalaire, anionique ou cationique qui peuvent être représentés par la formule : 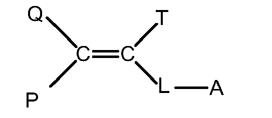 où P, Q et T représentent, indépendamment les uns des autres, un atome d'hydrogène, un radical hydrocarboné, linéaire ou ramifié, cyclique ou non, saturé ou non, ou aromatique, de préférence alkyle, comportant 1 à 12 atomes de carbone ou un groupement -L-A, L et A étant tels que définis précédemment.

De préférence les monomères (I) comportent 1 ou 2 groupements -L-A.
A titre d'exemples de tels monomères (co)polymérisables par voie radicalaire, anionique ou cationique, on peut citer les monomères de formule : 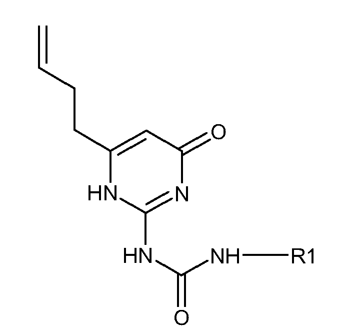 dans laquelle R₁ est tel que défini précédemment,
ainsi que l'acrylate dérivé du 6-méthyl 2-uréïdo-pyrimidone de formule :

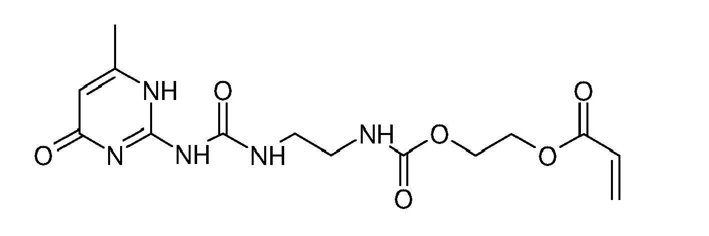

Ces monomères peuvent notamment être préparés selon l'enseignement décrit dans la demande internationale WO 98/14504.

On peut citer également les monomères de formules :

A-(CH₂)₂-CH=CH₂

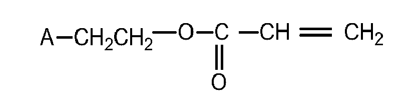

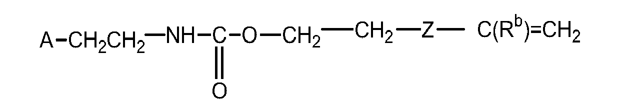

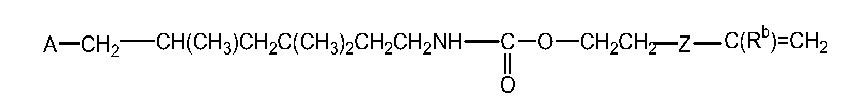

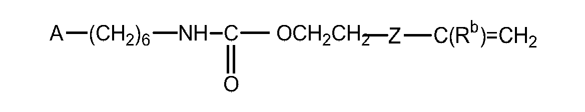

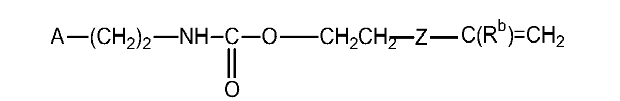

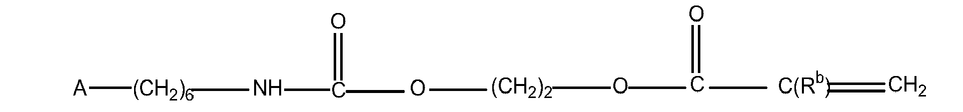

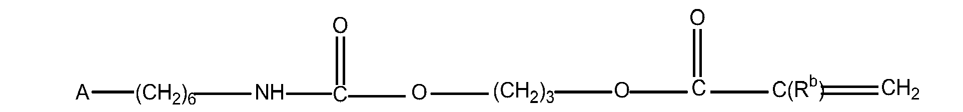

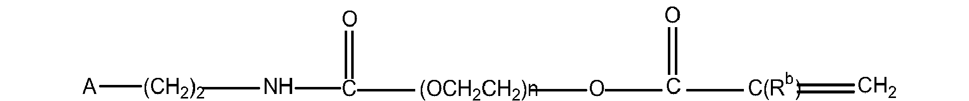

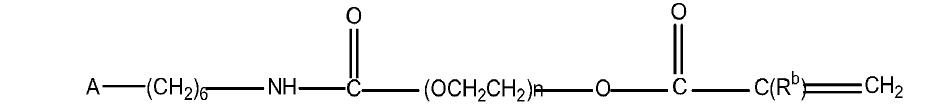

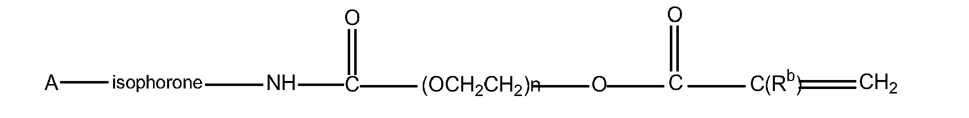

dans lesquelles :
- Z représente -O-C(O)- ou -NH-C(O)-
- n est un entier variant de 1 à 500
- R^{b} est H ou CH3 et
- A est le groupe : 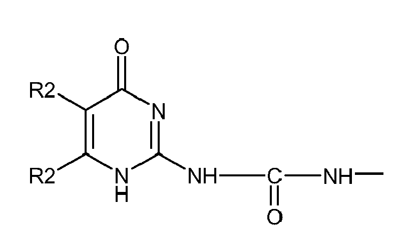 où les R₂, identiques ou différents, sont tels que définis ci-dessus.

Ces monomères peuvent notamment être préparés selon l'enseignement décrit dans la demande US 2004/0034190.

-ii) les monomères (co)polymérisables par addition ou polycondensation, comportant au moins un groupement G₁, de préférence deux groupements G₁, pouvant (co)polymériser par addition ou polycondensation.
Ces groupements G1 peuvent être identiques ou différents sur un même monomère, et peuvent être copolymérisables avec un monomère voisin étant entendu que l'homme de l'art choisira les monomères en tenant compte de leur réactivité.
Les groupements (co)polymérisables peuvent être choisis parmi les fonctions réactives mentionnées plus haut.
Les (co)polymères résultant de la polyaddition ou polycondensation peuvent alors être des polyuréthanes, des polyurées, des polyesters aliphatiques ou aromatiques, des polyamides aliphatiques ou aromatiques, ou leurs copolymères, comme par exemple des poly(uréthane/urées) et des poly(ester/amides).

De préférence les monomères comportent 1 ou 2 groupements A-L- ou -L-A-L-, de préférence un groupement A-L- ou -L-A-L-.
A titre d'exemples de tels monomères (co)polymérisables par polyaddition ou polycondensation, on peut citer les monomères de formules: 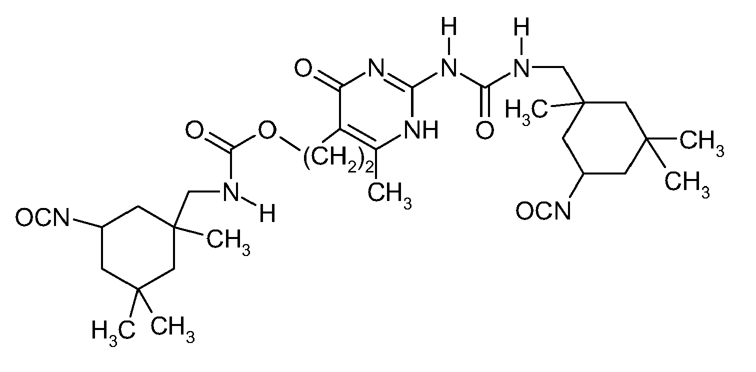 ou encore :

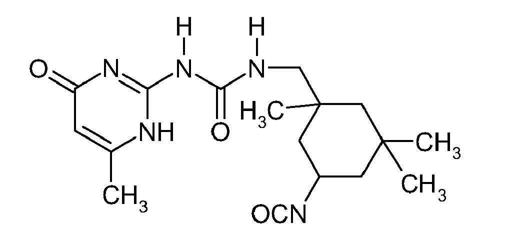

-iii) les monomères (co)polymérisant par ouverture de cycle.
Ces monomères comportent des groupes G₁ qui peuvent être choisis parmi :
- des éthers cycliques de formule : 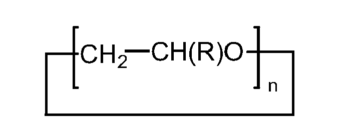 où R est H ou un radical hydrocarboné, linéaire ou ramifié, cyclique ou non, saturé ou non, ou aromatique, de préférence un alkyle, en C₁-C₁₂ et n est un entier de 1 à 3. De préférence R est H ou CH₃ et n = 1.
- des amides cycliques de formule : 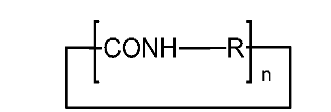 dans laquelle R est -(CH₂)ₘ- et m est un entier de 3 à 12, de préférence égal à 3 ou 5 et n est un entier de 1 à 3, de préférence égal à 1.
- des esters cycliques de formule : 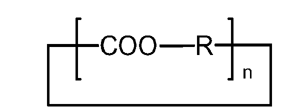 dans laquelle R est -(CH₂)ₘ- et m est un entier de 3 à 12, de préférence égal à 3 ou 5 et n est un entier de 1 à 3, de préférence égal à 1.
- des carbonates cycliques de formule : 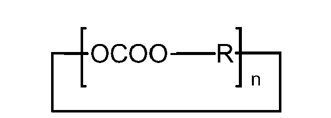 dans laquelle R est -(CH₂)ₘ- et m est un entier de 3 à 12, de préférence égal à 3 ou 5 et n est un entier de 1 à 3, de préférence égal à 1.
- des perfluoroéthers cycliques, des lactides, des oxazolines, le norbonène, ainsi que leurs dérivés;
- leurs combinaisons.

Parmi les (co)polymères résultants de la (co)polymérisation des éthers cycliques, on peut citer les poly(oxyalkylène), notamment les poly(oxyéthylène) et poly(oxypropylène) et leurs copolymères, tels que les poly(oxyéthylène /oxypropylène).
Parmi les polyamides résultants de telles polymérisations, on peut citer les polycaprolactames et les polypyrrolidones.
Parmi les polyesters résultants de telles polymérisations, on peut citer les polycaprolactones.
Parmi les polyoxazolines résultantes de telles polymérisations, on peut citer la poly(2-méthyl-oxazoline) et la poly(2-éthyloxazoline).
On peut également obtenir des poly(norbornènes) et des dérivés de ceux-ci.

Dans un mode particulier de réalisation de l'invention, L, lorsqu'il n'est pas une liaison simple, peut porter au moins un groupe ionisable tel que défini ci-après.

### Définition des monomères (II) de formule G₂

Les monomères (II) G₂ sont choisis en fonction du mécanisme de (co)polymérisation envisagé pour pouvoir se (co)polymériser avec les groupements G₁ des monomères (I).
Une classe préférée de monomères (II) G₂ est formée par les monomères éthyléniques, c'est-à-dire les monomères comportant une double liaison réactive capable de réagir dans des conditions radicalaires, anioniques ou cationiques.

Parmi les monomères éthyléniques préférés, on peut citer :

-i) les (méth)acrylates de formule CH₂=CHCOOR⁴ ou CH₂=C(CH₃)COOR⁴ dans laquelle R⁴ représente :
- un hydrogène,
- un groupe alkyle, linéaire, cyclique ou ramifié, (notamment cycloalkyle ou alkylcycloalkyle), de 1 à 30 atomes de carbone, dans lequel se trouve(nt) éventuellement intercalé(s) un ou plusieurs hétéroatomes choisis parmi O, N, S et P, ledit groupe alkyle pouvant, en outre, être éventuellement substitué par un ou plusieurs substituants choisis parmi les groupes hydroxyle, les atomes d'halogène (Cl, Br, I et F), et les groupes Si (R₇)(R₈), où R₇ et R₈, identiques ou différents, représentent un groupe alkyle en C₁ à C₆ ou un groupe phényle.
- un groupe aryle en C₃ à C₂₀ tel que le groupe phényle ;
- un groupe aralkyle ou alkylaryle, en C₄ à C₃₀ (groupe alkyle en C₁ à C₈) tels que 2-phényl-éthyle ou benzyle ;
- un groupe hétérocycloalkyle en C4-C12 contenant un ou plusieurs hétéroatomes choisis parmi O, N, P et S, le cycle étant aromatique ou non; tel qu'imidazole;
- un groupe alkylhétérocycloalkyle en C4-C30, (alkyle C₁-C₈), tels que furfurylméthyle ou tétrahydrofurfurylméthyle,
   lesdits groupes aryles, aralkyles pouvant comprendre éventuellement intercalés un ou plusieurs hétéroatomes choisis parmi O, N, S et P, et/ou pouvant être substitués par un ou plusieurs substituants choisis parmi les groupes hydroxyle, les atomes d'halogène et les groupes alkyles C₁-C₄, linéaires ou ramifiés qui eux-mêmes peuvent comprendre intercalés un ou plusieurs hétéroatomes choisis parmi O, N, S et P et/ou qui peuvent être substitués par un ou plusieurs substituants choisis parmi les groupes hydroxyle, les atomes d'halogène (Cl, Br, I et F), et les groupes Si (R₇)(R₈), où R₇ et R₈, identiques ou différents, représentent un groupe alkyle en C₁ à C₆, ou un groupe phényle.

Parmi les radicaux R⁴ préférés, on peut citer les groupes méthyle, éthyle, propyle, n-butyle, isobutyle, tertiobutyle, hexyle, éthylhexyle, octyle, lauryle, stéaryle, isooctyle, isodécyle, dodécyle; des groupes dérivés d'alkyle (c'est à dire alkyles substitués et/ou interrompus) tels que les groupes hydroxyalkyles en C1-C4 tels que 2-hydroxyéthyle, 2-hydroxybutyle et 2-hydroxypropyle; les groupes alcoxy(C1-4)alkyle(C1-4) tels que méthoxyéthyle, éthoxyéthyle et méthoxypropyle; les groupes cycloalkyles en C3 à C12 tels que isobornyle, t-butylcyclohexyle ou cyclohexyle; ou encore les groupes t-butylbenzyle, phényle, furfurylméthyle, tétrahydrofurfurylméthyle, éthyl-2-perfluorohexyle.
On peut également citer les groupes -(OC₂H₄)_{q}-OR, avec q = 5 à 500 et R = H ou alkyle de C₁ à C₃₀, par exemple -POE-méthoxy ou -POE-béhényle.

-ii) les (méth)acrylamides de formule CH₂=CHCONR⁶R⁵ ou CH₂=C(CH₃)CONR⁶R⁵ dans laquelle :
R⁵ et R ⁶ identiques ou différents, ont les mêmes significations que pour les groupes R⁴ ci-dessus.
Des exemples de monomères (méth)acrylamide sont le (méth)acrylamide, le N-éthyl(méth)acrylamide, le N-butylacrylamide, le N-t-butylacrylamide, le N-isopropylacrylamide, le N,N-diméthyl(méth)acrylamide, le N,N-dibutylacrylamide, le N-octylacrylamide, le N-dodécylcacrylamide, l'undécylacrylamide, et le N(2-hydroxypropylméthacrylamide).

-iii) les monomères vinyliques de formules : CH2=CH-R⁹, CH2=CH-CH₂-R⁹ ou CH2=C(CH3)-CH2-R⁹
dans lesquelles R⁹ est un groupe hydroxyle, halogène (Cl ou F), NH₂, acétamide (-NHCOCH3), -OR₁₀ où R₁₀ représente un groupe phényle ou un groupe alkyle en C1-C12 (éther de vinyle); -OCOR₁₁ (ester de vinyle) où R₁₁ représente:
(i) un groupe alkyle de C₂ à C₁₂, linéaire ou ramifié,
(ii) un groupe cycloalkyle en C₃ à C₁₂ tel que isobornyle, cyclohexyle,
(iii) un groupe aryle en C₃ à C₂₀ tel que phényle,
(iv) un groupe aralkyle en C₄ à C₃₀ (groupe alkyle en C₁ à C₈) tel que 2-phényléthyle et benzyle,
(v) un groupe hétérocycloalkyle saturé ou non, aromatique ou non, de 4 à 12 chaînons contenant un ou plusieurs hétéroatomes choisis parmi O, N, et S, tel que furfuryle ou tétrahydrofurfuryle,
(vi) un groupe alkylhétérocycloalkyle de C₁ à C₄, tel que furfurylméthyle ou tétrahydrofurfurylméthyle,
lesdits groupes alkyles, cycloalkyles, aryles, aralkyles, hétérocycloalkyles ou alkylhétérocycloalkyles pouvant être éventuellement substitués par un ou plusieurs substituants choisis parmi les groupes hydroxyle, les atomes d'halogène, et les groupes alkyles de C₁ à C₄, linéaires ou ramifiés dans lesquels se trouvent éventuellement intercalés un ou plusieurs hétéroatomes choisis parmi O, N, S et P, lesdits groupes alkyles pouvant, en outre, être éventuellement substitués par un ou plusieurs substituants choisis parmi les groupes hydroxyle, les atomes d'halogène (Cl, Br, I et F) et les groupes Si(R₇)(R₈) où R₇ et R₈, identiques ou différents, représentent un groupe alkyle en C₁ à C₆, ou un groupe phényle.

Des exemples de monomères vinyliques sont le vinylcyclohexane, le styrène, la N-vinyl-pyrrolidone et le N-vinylcaprolactame.
Des exemples d'esters de vinyle sont l'acétate de vinyle, le propionate de vinyle, le butyrate de vinyle, l'éthylhexanoate de vinyle, le néononanoate de vinyle et le néododécanoate de vinyle.
Parmi les éthers de vinyle, on trouve par exemple le vinyl méthyl éther, le vinyl éthyl éther, et le vinyl isobutyl éther.

-iv) D'autres monomères sont les monomères (méth)acrylates, (méth)acrylamides ou vinyliques à groupe fluoré ou perfluoré, tels que le méthacrylate d'éthylperfluorooctyle et les monomères (méth)acryliques, (méth)acrylamides ou vinyliques siliconés.

Dans un mode particulier de réalisation de l'invention, G2 peut porter au moins un groupe ionisable tel que défini ci-après.

### Définition des groupes ionisables

Selon l'invention, l'un au moins des groupements G₁ des monomères (I), des bras de liaison L des monomères (I), des groupes de jonction (A) des monomères (I) et/ou des monomères (II) G₂ éventuels, constitutifs du (co)polymère final selon l'invention, porte au moins un groupement ionisable.
De préférence, le (ou les) groupement(s) ionisable(s) sont portés par un bras de liaison L du monomère (I) lorsque L n'est pas une liaison simple, ou mieux encore par le monomère (II) G₂ lorsque celui-ci intervient dans la constitution du (co)polymère.
On rappelle que par groupe ionisable selon l'invention, on entend tout groupe qui, soit par sa nature chimique propre, soit en fonction du milieu et/ou du pH du milieu dans lequel il se trouve, peut être sous forme ionique.
Selon sa nature chimique, il peut être cationisable, anionisable, ou amphotère. Ceci inclut également les groupes ioniques de type ammonium quaternaire tétra N-substitués.

Le groupe ionisable, quand il est présent, peut représenter 0,1 à 50% en poids du poids total du copolymère selon l'invention, de préférence 0,5 à 35% en poids, et notamment 1 à 15% en poids, par rapport au poids total du polymère.
Ainsi, le ou les monomères portant le/les groupes ionisables peuvent représenter 3 à 20% en poids, de préférence 6 à 17% en poids, voire 8 à 15% en poids, du poids total du copolymère final.

Parmi les groupes ionisables préférés, on peut citer :

-i) les groupes anionisables, et leurs sels, tels que les groupes comportant une fonction acide choisie parmi :
- le radical carboxylique : -COOH,
- le radical sulfonique : -SO₃H
- le radical : -OSO₃H
- le radical phosphonique : -(O)P(OH)₂
- le radical phosphorique : -OP(O)(OH)₂
- ou leurs formes salifiées organiques ou minérales.

La neutralisation des groupes acides peut être effectuée par une base minérale, telle que LiOH, NaOH, KOH, Ca(OH)₂, NH₄OH, Mg(OH)₂ ou Zn(OH)₂; ou par une base organique telle qu'une alkylamine primaire, secondaire ou tertiaire, notamment la triéthylamine ou la butylamine. Cette alkylamine primaire, secondaire ou tertiaire peut comporter un ou plusieurs atomes d'azote et/ou d'oxygène et peut donc comporter par exemple une ou plusieurs fonctions alcool; on peut notamment citer l'amino-2-méthyl-2-propanol, la triéthanolamine et la diméthylamino-2-propanol. On peut encore citer la lysine ou la 3-(diméthylamino)propylamine.

-ii) les groupes cationisables, et leurs sels, tels que les groupes comportant une fonction choisie parmi :
-a) les radicaux amine de formule -N(R₁₅)(R₁₆) ou leurs sels organiques ou minéraux, avec R₁₅ et R₁₆ représentant, indépendamment l'un de l'autre :
(i) un atome d'hydrogène,
(ii) un groupement alkyle linéaire, ramifié ou cyclique, saturé ou insaturé, éventuellement aromatique, comprenant de 1 à 30 atomes de carbone et pouvant comprendre 1 à 10 hétéroatomes choisis parmi O, N, S, et P, notamment un groupement méthyle, éthyle, propyle, isopropyle, n-butyle, t-butyle, isobutyle, lauryle, stéaryle;
(iii) un groupement oxyde d'alkylène de formule -(R₁₇O)ᵣR₁₈ avec R₁₇ représentant un alkyle linéaire ou ramifié en C₂-C₄, R₁₈ est l'hydrogène ou un radical alkyle, linéaire ou ramifié, en C₁ à C₃₀ et r est compris entre 1 et 250 inclus ;
(iv) R₁₅ et R₁₆ peuvent former avec l'atome d'azote un cycle saturé ou insaturé, éventuellement aromatique, comprenant au total 5, 6, 7 ou 8 atomes, et notamment 4, 5 ou 6 atomes de carbone et/ou 2 à 4 hétéroatomes choisi parmi O, S et N; ledit cycle pouvant en outre être fusionné avec un ou plusieurs autres cycles, saturés ou insaturés, éventuellement aromatiques, comprenant chacun 5, 6 ou 7 atomes, et notamment 4, 5, 6 ou 7 atomes de carbone et/ou 2 à 4 hétéroatomes choisi parmi O, S et N ;
-b) un groupement -R'₁₅-N-R'₁₆- dans lequel R'₁₅ et R'₁₆ forment avec l'atome d'azote un cycle, saturé ou insaturé, éventuellement aromatique, comprenant au total 5, 6, 7 ou 8 atomes, et notamment 4, 5 ou 6 atomes de carbone et/ou 2 à 4 hétéroatomes choisi parmi O, S et N; ledit cycle pouvant être fusionné avec un ou plusieurs autres cycles, saturés ou insaturés, éventuellement aromatiques, comprenant chacun 5, 6 ou 7 atomes, et notamment 4, 5, 6, 7 ou 8 atomes de carbone et/ou 2 à 4 hétéroatomes choisi parmi O, S et N, par exemple, le groupe ionisable peut constituer un cycle aromatique ou non comportant un groupement amine tertiaire cationisable ou peut représenter un hétérocycle aromatique ou non, contenant un azote tertiaire, cationisable.
-c) un groupement guanidino ou amidino respectivement de formule :

| | |
|---|---|
| 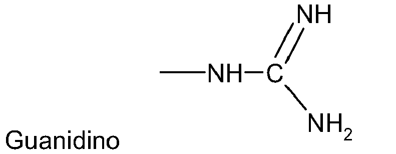 | 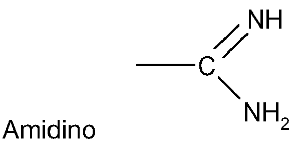 |

-d) les radicaux ammonium quaternaire de formule : -N⁺(R¹²)₃, Z⁻ où R¹² est un radical alkyle, identique ou différent, linéaire ou ramifié, en C₁ à C₂₀ ; et Z est un atome d'halogène tel que Br, Cl ou bien -OSO₃CH₃ ;
-e) et leurs mélanges.

Parmi ces radicaux préférés, on peut citer les radicaux pyridinyle, indolyle, isoindolinyle, imidazolyle, imidazolinyle, piperidinyle, pyrazolyle, quinolinyle, pyrazolinyle, piperazinyle, pyrrolidinyle, quinidinyle, thiazolinyle, morpholinyle, guanidino, amidino, et leurs combinaisons.

Les motifs amines peuvent éventuellement être neutralisés. Parmi les sels, on peut citer les sels d'acides minéraux, tels que l'acide sulfurique, l'acide chlorhydrique, l'acide phosphorique. On peut également citer les sels d'acides organiques, qui peuvent comporter un ou plusieurs groupes acide carboxylique, sulfonique, ou phosphonique. Il peut s'agir d'acides aliphatiques linéaires, ramifiés
ou cycliques ou encore d'acides aromatiques. Ces acides peuvent comporter, en outre, un ou plusieurs hétéroatomes choisis parmi O et N, par exemple sous la forme de groupes hydroxyle. On peut notamment citer l'acide propionique, l'acide acétique, l'acide téréphtalique, l'acide citrique, l'acide tartrique et l'acide lactique.

Il est à noter que la neutralisation des motifs acides ou amines, de même que la quaternisation, peut être totale ou partielle.

Plus particulièrement, ces groupes ionisables peuvent être choisis parmi :
- les groupes anionisables suivants : les groupes monovalents -COOH, - CH₂COOH, -(CH₂)₂COOH, -(CH₂)₃COOH, -(CH₂)SO₃H, -(CH₂)₂SO₃H, - (CH₂)₃SO₃H, -O(CH₂)₃SO₃H; les groupes divalents -C(COOH)(CH₃)- et -CH₂-C(COOH)(CH₃)-CH₂- ;
   pour lesquels les neutralisants peuvent être choisis parmi NaOH, KOH, Ca(OH)₂, NH₄OH, la triéthylamine, la butylamine, l'amino-2-méthyl-2-propanol, la triéthanolamine, la diméthylamino-2-propanol, la lysine ou la 3-(diméthylamino)propylamine;
- les groupes cationisables suivants : les groupes monovalents -(CH₂)₂-N(CH₃)₂, -N(CH₃)₂, -(CH₂)₃-N(CH₃)₂, -O-(CH₂)₃-N(CH₂CH₃)₂, -(CH₂)₂-N(CH₂CH₃)₂; les groupes divalents -(CH₂)₂-N(CH₃)-(CH₂)₂-et -(CH₂)₃-N(CH₃)-(CH₂)₃-.
   pour lesquels les neutralisants peuvent être choisis parmi HCl, l'acide propionique, l'acide acétique, l'acide citrique et l'acide tartrique.

Parmi les groupements G₁ porteurs de groupes ionisables, on peut citer les groupements CH₂=C(COOH)-COO- et HOC(CH₃)(COOH)-.

Ainsi, à titre de groupement G1-L-A, on peut avoir :
- le groupement CH₂=C(COOH)-COO-CH₂OCONH(CH₂)₆uréidopyrimidone de formule : 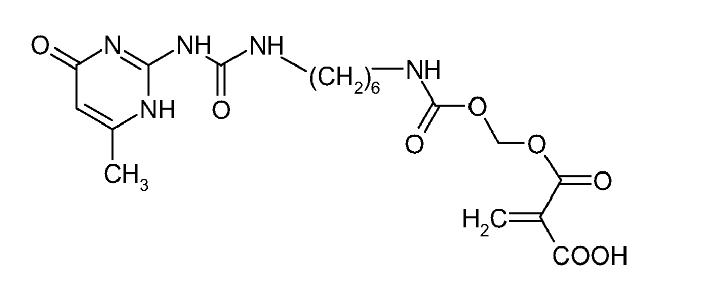
- le groupement HOC(CH₃)(COOH)-CH₂CH₂OC(O)-NH(CH₂)₆uréidopyrimidone de formule :

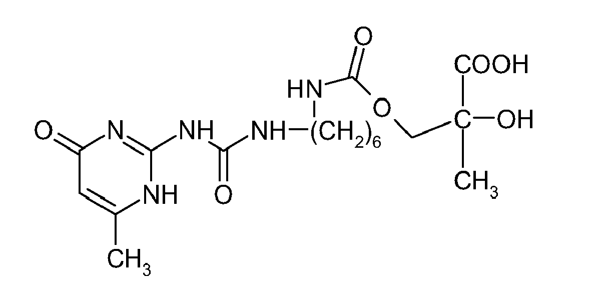

Parmi les monomères G₂ comprenant une double liaison polymérisable par voie anionique, cationique ou radicalaire et comportant un groupe ionisable, notamment anionisable ou cationisable, on peut citer :

-(i) les monomères à insaturation(s) éthylénique(s) comprenant au moins une fonction acide carboxylique (COOH), phosphonique (PO₃H₂) ou sulfonique (SO₃H) comme par exemple ceux de formule suivante :

CH₂=C(R₁₉)-(Z1)_{z1}-(Z2)_{z2}-Y

dans laquelle
- R₁₉ est un atome d'hydrogène ou un radical hydrocarboné, linéaire, cyclique ou ramifié, de type CₚH₂ₚ₊₁, avec p étant un entier compris entre 1 et 12, inclus; notamment R₁₉ peut représenter un radical méthyle, éthyle, propyle, butyle. De préférence, R₁₉ représente l'hydrogène ou un radical méthyle ou éthyle ;
- Z1 est un groupement divalent choisi parmi -COO-, -CONH-, -CONCH₃-, - OCO- et -O-, de préférence Z₁ est choisi parmi -COO- et -CONH- ;
- z₁ est 0 ou 1, de préférence 1 ;
- Z2 est un radical divalent carboné, saturé ou insaturé, éventuellement aromatique, linéaire, ramifié ou cyclique, de 1 à 30 atomes de carbone, pouvant comprendre 1 à 30 hétéroatomes choisis parmi O, N, S, et P;
- z₂ est 0 ou 1, de préférence 1 ; et
- Y est un groupement choisi parmi -COOH, -SO₃H, -OSO₃H, -PO(OH)₂ et - OPO(OH)₂.
Dans le radical Z2, le ou les hétéroatomes, quand ils sont présents, peuvent être intercalés dans la chaîne dudit radical Z2, ou bien ledit radical Z2 peut être substitué par un ou plusieurs groupes les comprenant tels que hydroxy ou amino (NH₂, NHR' ou NR'R" avec R' et R" identiques ou différents représentant un alkyle linéaire ou ramifié en C₁-C₂₂, notamment méthyle ou éthyle).
Notamment Z2 peut être :
- un radical alkylène tel que méthylène, éthylène, propylène, n-butylène, isobutylène, tertiobutylène, n-hexylène, n-octylène, n-dodécylène, n-octadécylène, n-tétradécylène, n-docosanylène;
- un radical phénylène -C₆H₄-(ortho, méta ou para) éventuellement substitué, par un radical alkyle en C₁-C₁₂ comprenant éventuellement 1 à 8 hétéroatomes choisis parmi O, N, S, et P; ou bien un radical benzylène -C₆H₄-CH₂- éventuellement substitué, par un radical alkyle en C₁-C₁₂ comprenant éventuellement 1 à 8 hétéroatomes choisis parmi O, N, S, et P;
- un radical de formule -CH₂-CH(OH)-, -CH₂-CH₂-CH(OH)-, -CH₂-CH₂-CH(NH₂)-, -CH₂-CH(NH₂)-, -CH₂-CH₂-CH(NHR')-, -CH₂-CH(NHR')-, -CH₂-CH₂-CH(NR'R")-, -CH₂-CH(NR'R")-, -CH₂-CH=CH- avec R' et R" représentant un alkyle linéaire ou ramifié en C₁-C₁₈, notamment méthyle ou éthyle.

Parmi les monomères G2 anionisables plus particulièrement préférés, on peut citer notamment l'acide acrylique, l'acide méthacrylique, l'acide crotonique, l'acide itaconique, l'acide fumarique, l'acide maléique, l'acide diacrylique, l'acide diméthylfumarique, l'acide citraconique, l'acide acrylamidopropanesulfonique, l'acide 2-acrylamido 2-méthylpropanesulfonique, l'acide styrène sulfonique, l'acide vinylbenzoïque, l'acide vinylphosphorique, l'acide vinylsulfonique, l'acide vinylbenzène sulfonique, l'acide acrylamidoglycolique de formule CH2=CH-CONHCH(OH)COOH, l'acide vinylphosphonique; le (méth)acrylate de 2-carboxyéthyle, le méthacrylate ou l'acrylate de sulfopropyle (CH₂=C(CH₃)CO₂(CH₂)₃SO₃H), le méthacrylate ou l'acrylate de sulfoéthyle et la vinylméthylsulfone, le 2-(méthacryloyloxy)éthylphosphate de formule CH₂=C(CH₃)COOC₂H₄OP(O)(OH)₂; le maléate de diallyle de formule C₃H₅-CO₂-CH=CH-CO₂-C₂H₅; les anhydrides carboxyliques porteurs d'une liaison vinylique tels que l'anhydride maléique, ainsi que leurs sels; et leurs mélanges.

-(ii) les monomères à insaturation(s) éthylénique(s) comprenant au moins une fonction amine primaire, secondaire ou tertiaire, notamment ceux de formule suivante : 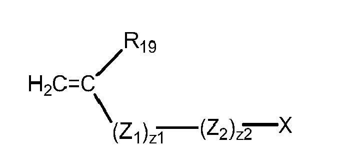 dans laquelle
- R₁₉, Z1, Z2, z₁ et z₂ ont les mêmes significations que dans la formule précédente; et
- X est un groupe de formule -N-R₁₇R₁₈ avec R₁₇ et R₁₈ représentant, indépendamment l'un de l'autre,

(i) un atome d'hydrogène ;
(ii) un groupement alkyle linéaire, ramifié ou cyclique, saturé ou insaturé, éventuellement aromatique, comprenant de 1 à 30 atomes de carbone, pouvant comprendre 1 à 10 hétéroatomes choisis parmi O, N, S, et P; notamment un groupement méthyle, éthyle, propyle, isopropyle, n-butyle, t-butyle, isobutyle, lauryle, stéaryle;
(iii) un groupement oxyde d'alkylène de formule -(R₂₀O)_{y}R₂₁ avec R₂₀ représentant un alkyle linéaire ou ramifié en C₂-C₄, R₂₁ est l'hydrogène ou un radical alkyle, linéaire ou ramifié, en C₂-C₃₀ et y est compris entre 1 et 250 inclus ;
(iv) R₁₇ et R₁₈ peuvent former avec l'atome d'azote un cycle saturé ou insaturé, éventuellement aromatique, comprenant au total 5, 6, 7 ou 8 atomes, et notamment 4, 5 ou 6 atomes de carbone et/ou 2 à 4 hétéroatomes choisi parmi O, S et N; ledit cycle pouvant en outre être fusionné avec un ou plusieurs autres cycles, saturés ou insaturés, éventuellement aromatiques, comprenant chacun 5, 6 ou 7 atomes, et notamment 4, 5, 6 ou 7 atomes de carbone et/ou 2 à 4 hétéroatomes choisi parmi O, S et N ;
ou bien X représente (b) un groupement -R'₁₅-N-R'₁₆- dans lequel R'₁₅ et R'₁₆ forment avec l'atome d'azote un cycle, saturé ou insaturé, éventuellement aromatique, comprenant au total 5, 6, 7 ou 8 atomes, et notamment 4, 5 ou 6 atomes de carbone et/ou 2 à 4 hétéroatomes choisi parmi O, S et N; ledit cycle pouvant être fusionné avec un ou plusieurs autres cycles, saturés ou insaturés, éventuellement aromatiques, comprenant chacun 5, 6 ou 7 atomes, et notamment 4, 5, 6, 7 ou 8 atomes de carbone et/ou 2 à 4 hétéroatomes choisi parmi O, S et N.

Par exemple, X peut constituer un cycle aromatique ou non comportant un groupement amine tertiaire cationisable ou peut représenter un hétérocycle aromatique ou non, contenant un azote tertiaire, cationisable. Parmi les radicaux X préférés, on peut citer les radicaux de type pyridine, indolyle, isoindolinyle, imidazolyle, imidazolinyle, piperidinyle, pyrazolynyle, pyrazolye, quinoline, pyrazolinyle, pyridinyle, piperazinyle, pyrrolidinyle, quinidinyle, thiazolinyle, morpholine, guanidino, amidino, et leurs mélanges.

Parmi les monomères G2 cationisables préférés, on peut citer, seuls ou en mélange :
- la 2-vinylpyridine, la 4-vinylpyridine, l'allylamine et l'allylpyridine;
- les (méth)acrylates d'aminoalkyles, tels que les (méth)acrylates de [N,N-di(C₁-C₄) alkylamino](C₁-C₆)alkyle ou les (méth)acrylates de [N-(C₁-C₄)alkylamino](C1-C6)alkyle et notamment le (méth)acrylate de N,N-diméthylaminoéthyle, le (méth)acrylate de N,N-diéthylaminoéthyle, le (méth)acrylate de 2-aminoéthyle, le (méth)acrylate de 2-(N-tertbutylamino)éthyle;
- les (méth)acrylamides d'aminoalkyles, tels que les (méth)acrylamides de [N,N-di(C₁-C₄)alkylamino](C₁-C₆)alkyle ou les (méth)acrylamides de [N-(C₁-C₄)alkyle amino](C₁-C₆)alkyle, et notamment le (méth)acrylamide de N,N-diméthylaminopropyle, le (méth)acrylamide de N,N-diméthylaminoéthyle; le (méth)acrylamide de 3-aminopropyle;
- la vinylamine, la vinylimidazole, le 2-(diéthylamino)éthylstyrène;
- la N-vinylimidazole, la N-vinyl-2-méthylimidazole, la N-vinylcarbazole;
ainsi que leurs sels et/ou leurs formes quaternisées.

-(iii) les monomères de formule : 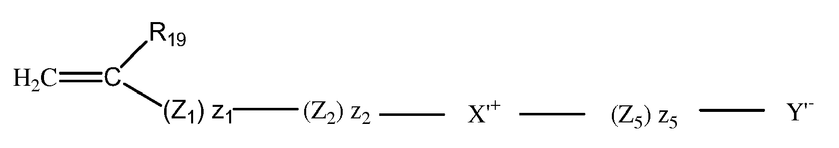 dans laquelle :
- R₁₉, Z₁, Z₂, z₁, z₂, ont la même signification que dans la formule précédente,
- Z₅ a la même signification que celle donnée pour Z₂, mais peut être différent de Z2
- Z₅ =0 ou 1,
- X'⁺ est un groupe divalent de formule -N⁺(R₆)(R₇)- avec R₆ et R₇ représentant, indépendamment l'un de l'autre, soit (i) un atome d'hydrogène, soit (ii) un groupement alkyle linéaire, ramifié ou cyclique, éventuellement aromatique, comprenant de 1 à 25 atomes de carbone, pouvant comporter de 1 à 20 hétéroatomes choisis parmi O, N, S et P ; soit (iii) R₆ et R₇ peuvent former avec l'atome d'azote un premier cycle, saturé ou insaturé, éventuellement aromatique, comprenant au total 5, 6, 7 ou 8 atomes, et notamment 4, 5, 6 ou 7 atomes de carbone et/ou 2 ou 3 hétéroatomes choisis parmi O, N et S; ledit premier cycle pouvant être fusionné avec un ou plusieurs autres cycles, saturés ou insaturés, éventuellement aromatiques, comprenant chacun 5, 6, 7 ou 8 atomes, et notamment 4, 5, 6 ou 7 atomes de carbone et/ou 2 ou 3 hétéroatomes choisis parmi O, N et S ; R₆ et R₇ étant notamment choisis parmi l'hydrogène, un groupement méthyle, éthyle, propyle, isopropyle, n-butyle, t-butyle ou isobutyle ; et
- Y'⁻ est un groupement choisi parmi -COO⁻, -SO₃⁻, -OSO₃⁻, -PO₃²⁻, -OPO₃²⁻.

Notamment, Z₂ et Z₅ peuvent être sélectionnés parmi les groupements alkylènes linéaires, saturés ou non, ramifiés ou cycliques (aromatiques ou non) comprenant 1-30 atomes de carbone, incluant ou non un ou plusieurs hétéroatomes tels que O, N, S et P. De préférence, Z₂ et Z₅ sont sélectionnés parmi les groupes alkylènes en C₁-C₃₀, phénylène, benzylène, -(CH₂-CH=CH)-, -(CH₂-CH₂-CHOH)- ou -CH₂-CH₂-CH(NR₂₀R₂₁)- avec R₂₀ et R₂₁ sélectionnés parmi H et alkyles en C₁-C₁₈, de préférence, R₂₀ et R₂₁ étant choisis parmi H, CH₃ et C₂H₅. Préférentiellement, Z₂ et Z₅ sont sélectionnés parmi les groupes alkylènes en C₁-C₆, phénylène, benzylène.
On peut également peut citer la N,N-dimethyl-N-(2-methacryloyloxyethyl)-N-(3-sulfopropyl) ammonium bétaine (notamment la SPE de la société Raschig); la N,N-dimethyl-N-(3-methacrylamidopropyl)-N-(3-sulfopropyl) ammonium bétaine (SPP de Raschig), et la 1-(3-sulfopropyl)-2-vinylpyridinium bétaine (SPV de Raschig).

Parmi les monomères G₂ pouvant (co)polymériser par polyaddition ou polycondensation et comportant un groupe ionisable, on peut citer, par exemple, les monomères de formule HX"-B-X"H, dans laquelle :
- X", identique ou différent, représente O, S, NH ou NR, avec R représentant un groupe alkyle en C₁-₆,
- B représente un radical alkylène ayant de 1 à 6000 atomes de carbone, linéaire, ramifié ou cyclique, saturé ou insaturé, éventuellement aromatique, comprenant éventuellement un ou plusieurs hétéroatomes choisis parmi O, S, P et N, et/ou éventuellement substitué par un ou plusieurs atomes de fluor et/ou silicium;
sous réserve qu'au moins un des radicaux B porte un groupe ionisable tel que défini ci-dessus.

Notamment, B peut être :
- un radical alkylène ayant 1 à 40 atomes de carbone ou cycloalkylène ayant 3 à 16 atomes de carbone, éventuellement substitué par un radical alkyle en C1-C12 et/ou comprenant éventuellement 1 à 8 hétéroatomes choisis parmi O, N, S, F, Si et P; tel que méthylène, éthylène, propylène, n-butylène, isobutylène, tertiobutylène, n-hexylène, n-octylène, n-dodécylène, n-octadécylène, n-tétradécylène, n-docosanylène; ethyl-2-hexyl, cyclohexylène, cyclohexyleméthylène, isophorone ;
- un radical arylène en C1-C30 tel qu'un radical phénylène -C₆H₄-(ortho, méta ou para);
- un radical alkylarylène ou arylalkylène en C₁ à C₃₀, de préférence en C₂ à C₁₂, éventuellement substitué par un radical alkyle en C₁-C₁₂ comprenant éventuellement 1 à 25 hétéroatomes choisis parmi O, N, S, F, Si et P; tel qu'un radical benzylène -C₆H₄-CH₂- éventuellement substitué, par un radical alkyle en C₁-C₁₂ comprenant éventuellement 1 à 8 hétéroatomes choisis parmi O, N, S, F, Si et P.

Le ou les hétéroatomes, quand ils sont présents dans B, peuvent être intercalés dans la chaîne dudit radical, ou bien ledit radical peut être substitué par un ou plusieurs groupes les comprenant tels que hydroxy ou amino (NH₂, NHR' ou NR'R" avec R' et R" identiques ou différents représentant un alkyle linéaire ou ramifié en C₁-C₂₂, comprenant éventuellement 1 à 12 hétéroatomes choisis parmi O, N, S, F, Si et P, notamment méthyle ou éthyle).
En particulier, B peut comprendre :
- un radical de formule -O-CO-O-, -CO-O-, -OCO -, -O-CO-NH-, anhydride, - NH-CO-NH-, NHCO;
- un radical -Si(R₄)(R₅)O- où R₄ et R₅, identiques ou différents représentent H ou un radical hydrocarboné, linéaire ou ramifié, cyclique ou non, saturé ou non, ou aromatique, de préférence un alkyle en C₁-C₁₂ pouvant éventuellement comporter un ou plusieurs, de préférence 1 à 5, hétéroatomes identiques ou différents choisis parmi O, N, S, P, F et Si, de préférence O, N et S ; et/ou
- un radical oxyalkylène ou aminoalkylène, notamment un radical oxyde d'alkylène de formule -(R"O)_{y}R"₁ avec R" représentant un alkyle linéaire ou ramifié en C₂-C₄, R"₁ représentant H ou un radical alkyle, linéaire ou ramifié, en C₁à C₃₀ et y est compris entre 1 et 500 inclus.

Parmi les monomères HX-B-XH préférés pouvant (co)polymériser par polyaddition ou polycondensation, et comportant des groupes ionisables, on peut citer l' acide diméthylolpropionique, l'acide diméthylaminopropionique, la N-éthylsulfoniquediméthanolamine, la N-éthylsulfoniquediéthanolamine, l'acide diol benzène sulfonique, la diaminopyridine, la N-méthyldiéthanolamine, la N-éthyldiéthanolamine, la N-tert-butyldiéthanolamine, et leurs mélanges.

On a constaté que les (co)polymères selon la présente invention étaient avantageusement solubles ou dispersibles dans les milieux aqueux, les huiles carbonées, les huiles de silicone et/ou les solvants cosmétiques, tels que les alcanols, notamment l'éthanol et les esters d'alkyle tels que les acétates d'alkyle.
De façon particulièrement préférée, les (co)polymères de l'invention sont solubles ou dispersibles dans l'eau.
Le (co)polymère est dit soluble dans le milieu lorsqu'il est solubilisé, c'est-à-dire forme une solution limpide, à raison d'au moins 1% en poids dans le milieu à 25°C.

Le (co)polymère est dit dispersible s'il forme dans le milieu, à concentration de 1 % en poids à 25°C, une suspension ou dispersion stable de fines particules, généralement sphériques. Par « stable » on entend que la suspension ne précipite pas et ne présente donc pas de dépôt visible. La taille moyenne des particules constituant la suspension ou dispersion est de préférence inférieure à 1 µm et, plus généralement, varie entre 5 et 400 nm, de préférence 10 à 250 nm. Ces tailles de particules sont mesurées par toute méthode classique de diffusion de lumière.

Les (co)polymères selon l'invention plus particulièrement préférés peuvent appartenir à l'une des catégories suivantes, sous réserve qu'ils comprennent un groupe ionisable :
- (i) les (co)polymères éthyléniques et notamment vinyliques, comme par exemple les copolymères (méth)acryliques, les copolymères (méth)acrylamides, les copolymères allyliques, les copolyoléfines tels que les polydiènes hydrogénés ou non et leurs mélanges.
   Ainsi conviennent à l'invention les copolymères vinyliques/(méth)acrylates, vinyliques/(méth)acrylamides, vinyliques/(méth)acrylates/méthacrylamides, oléfiniques/vinyliques et les (méth)acrylates /(méth)acrylamides.
   A titre illustratif de ces copolymères, on peut plus particulièrement citer les copolymères à base d'acétate de vinyle, de styrène, de vinylpyrrolidone, de vinylcaprolactame, de (méth)acrylate de polyoxyde d'éthylène, de (méth)acrylate de stéaryle, de (méth)acrylate de lauryle, de laurate de vinyle, de (méth)acrylate de butyle, de (méth)acrylate d'éthylhexyle, d'acide crotonique, d'acide (méth)acrylique, d'anhydride maléique, d'acide styrène sulfonique, de diméthyldiallylamine, de vinylpyridine, de (méth)acrylate de diméthylaminoéthyle, de diméthylaminopropyl(méth)acrylamide et leurs sels.
   Dans ce cas les (co)polymères obtenus comporteront en général les motifs de jonction (A) en greffons le long du squelette du (co)polymère.
- (ii) les polycondensats de polyuréthanne et/ou de polyurées, de polyesters aliphatiques, ou aromatiques, polyamides aliphatiques ou aromatiques ou de leurs copolymères, comme par exemple polyuréthanne/urée, polyester/amide, polyester/polyuréthanne/urée.
- (iii) les (co)polymères obtenus par ouverture de cycle, comme les polyéthers de type polyoxyde d'éthylène, polyoxyde de propylène, et leurs copolymères polyoxyde d'éthylène/polyoxyde de propylène; les polylactides, les polyesters comme la polycaprolactone; les polyoxazolines tels que la poly(2-méthyloxazoline), ou la poly(2-éthyloxazoline),
- (iv) les (co)polymères de siloxane, tels que par exemple les polydiméthylsiloxanes (PDMS), et polyméthylphénylsiloxanes ;
- (v) les polythioéthers, les polycarbonates, les polyacétals, les perfluoropolyéthers,
- (vi) les (co)polymères obtenus par métathèse comme le poly(norbornène) et ses copolymères,
- (vii) les copolymères de ces différents types de polymères, comme par exemple les copolymères polysiloxane/polyoxyde d'éthylène, polysiloxane/polyuréthanne/urée, poly(éthylène-butylène)/polyuréthanne et polybutadiène hydrogéné/polyuréthanne;
- (viii) leurs sels et leurs dérivés ; et leurs mélanges.

Les (co)polymères selon l'invention trouvent une application toute particulière dans le domaine de la cosmétique. Ils peuvent être présents dans la composition sous forme solubilisée, par exemple dans un milieu solvant qui peut comprendre de l'eau et/ou un solvant organique, ou bien sous forme de dispersion aqueuse ou organique.

Ils peuvent être utilisés dans les compositions notamment cosmétiques ou pharmaceutiques à raison de 0,01 à 90% en poids de matière sèche, notamment 0,05 à 70% en poids, voire 0,1 à 60% en poids, encore mieux 1 à 50% en poids, par rapport au poids total de la composition.

Bien évidemment les compositions selon l'invention peuvent comprendre un mélange de (co)polymères selon l'invention.
Les (co)polymères POL-(A)i d'un mélange de (co)polymères peuvent comporter deux à deux un nombre différent de groupes de jonction (A) et des groupes de jonction (A) de nature différente, étant entendu que la condition d'appariement est satisfaite.

Avantageusement, les compositions selon l'invention peuvent également comprendre 0,01 à 90% en poids de matière sèche, notamment 0,05 à 70% en poids, voire 0,1 à 60% en poids, encore mieux 1 à 50% en poids, par rapport au poids total de la composition, de (co)polymères tels que décrits dans la demande WO02/98377, c'est-à-dire d'au moins un polymère linéaire, ramifié ou cyclique, ou dendrimère, comportant un squelette polymérique -POL- comprenant au moins deux motifs de répétition, et au moins deux groupes de jonction (A) fixés sur le squelette polymérique, et capables d'établir au moins trois liaisons H avec un même groupe de jonction partenaire.

Les compositions cosmétiques ou pharmaceutiques selon l'invention comprennent, outre lesdits (co)polymères, un milieu physiologiquement acceptable, notamment cosmétiquement ou dermatologiquement acceptable, c'est-à-dire un milieu compatible avec les matières kératiniques telles que la peau du visage ou du corps, les cheveux, les cils, les sourcils et les ongles.

Le milieu physiologiquement acceptable est avantageusement un milieu ne nuisant pas aux propriétés de persistance accrue d'au moins un effet cosmétique et/ou de soin, d'adhésion sur les matières kératiniques et de facilité de démaquillage apportées par la composition après application.

De préférence, le milieu physiologiquement acceptable comprend un milieu solvant des (co)polymères selon l'invention, qui peut comprendre au moins un composé choisi parmi l'eau, les alcools, les polyols, les esters, les huiles carbonées, les huiles de silicone, les huiles de silicone fluorées, et leurs mélanges.

Ainsi, le milieu solvant des compositions selon l'invention peut comprendre de l'eau ou un mélange d'eau et de solvant(s) organique(s) hydrophile(s) comme les alcools et notamment les monoalcools, linéaires ou ramifiés en C1-C6, comme l'éthanol, le tert-butanol, le n-butanol, l'isopropanol ou le n-propanol, ou le 2-butoxy-éthanol; et les polyols comme la glycérine, la diglycérine, l'éthylène glycol, le propylène glycol, le sorbitol, le pentylène glycol, et les polyéthylène glycols, ou bien encore les éthers de glycols notamment en C₂ tel que le monoéthyléther et le monométhyléther de diéthylèneglycol, et les aldéhydes en C₂-C₄ hydrophiles.

Le milieu solvant peut comprendre des alcools aromatiques comme l'alcool benzylique ou le phénoxyéthanol, leurs dérivés; des esters carboxyliques, et leurs mélanges.
Parmi les esters carboxyliques, on peut citer ceux comprenant 2 à 8 atomes de carbone, notamment l'acétate d'éthyle et l'acétate de butyle, l'acétate de méthyle, l'acétate de propyle, l'acétate de n-butyle, l'acétate d'isopentyle.

Le milieu solvant peut également comprendre des solvants organiques, physiologiquement acceptables, choisis parmi les cétones liquides à température ambiante tels que méthyléthylcétone, méthylisobutylcétone, diisobutylcétone, l'isophorone, la cyclohexanone, l'acétone; les éthers de propylène glycol liquides à température ambiante tels que le monométhyléther de propylène glycol, l'acétate de monométhyléther de propylène glycol, le mono n-butyl éther de dipropylène glycol; les éthers liquides à 25°C tels que le diéthyléther, le diméthyléther ou le dichlorodiéthyléther; les alcanes liquides à 25°C tels que le décane, l'heptane, le dodécane, l'isododécane, le cyclohexane; les composés cycliques aromatiques liquides à 25°C tels que le toluène et le xylène ; les aldéhydes liquides à 25°C tels que le benzaldéhyde, l'acétaldéhyde et leurs mélanges.

Le milieu solvant peut également comprendre des huiles cosmétiques, polaires
ou apolaires, carbonées ou siliconées, d'origine animale, végétale, minérale ou synthétique.

Parmi les huiles polaires, on peut citer les huiles carbonées pouvant comporter des fonctions esters, éthers, acides et/ou alcools, telles que par exemple:
- les huiles végétales carbonées à forte teneur en triglycérides constitués d'esters d'acides gras et de glycérol dont les acides gras peuvent avoir des longueurs de chaîne variées, ces dernières pouvant être linéaires ou ramifiées, saturées ou insaturées; ces huiles sont notamment les huiles de germe de blé, de maïs, de tournesol, de karité, de ricin, d'amandes douces, de macadamia, d'abricot, de soja, de colza, de coton, de luzerne, de pavot, de potimarron, de sésame, de courge, d'avocat, de noisette, de pépins de raisin ou de cassis, d'onagre, de millet, d'orge, de quinoa, d'olive, de seigle, de carthame, de bancoulier, de passiflore, de rosier muscat; ou encore les triglycérides des acides caprylique/caprique comme ceux vendus par la société Stearineries Dubois ou ceux vendus sous les dénominations Miglyol 810, 812 et 818 par la société Dynamit Nobel;
- les huiles de synthèse de formule R²⁰COOR²¹ dans laquelle R²⁰ représente le reste d'un acide gras, linéaire ou ramifié, comportant de 7 à 19 atomes de carbone, et R²¹ représente une chaîne hydrocarbonée ramifiée contenant de 3 à 20 atomes de carbone, comme par exemple l'huile de Purcellin (octanoate de cétostéaryle), l'isononanoate d'isononyle, les benzoates d'alkyle en C₁₂-C₁₅.
- les esters et les éthers de synthèse comme le myristate d'isopropyle, le palmitate d'éthyl-2-hexyle, les octanoates, les décanoates ou les ricinoléates d'alcools ou de polyalcools,
- les esters hydroxylés comme le lactate d'isostéaryle, le malate de diisostéaryle, et les esters du pentaérythritol;
- les alcools gras en C₈ à C₂₆ comme l'alcool oléique; et
- leurs mélanges.

Parmi les huiles apolaires, on peut citer :
- les huiles de silicone volatiles ou non, linéaires ou cycliques, liquides à température ambiante, telles que les polydiméthylsiloxanes (PDMS) comportant des groupements alkyle, alcoxy ou phényle, pendants et/ou en bout de chaîne siliconée et ayant de 2 à 24 atomes de carbone; les silicones phénylées, comme les phényl triméthicones, les phényl diméthicones, les phényl triméthylsiloxydiphénylsiloxanes, les diphényl diméthicones, les diphénylméthyltrisiloxanes, les 2-phényléthyl triméthylsiloxysilicates,
- les hydrocarbures et les hydrocarbures fluorés/ou fluorocarbures, linéaires ou ramifiés d'origine synthétique ou minérale, comme les huiles volatiles, telles que les huiles de paraffine (par exemple les isoparaffines et notamment l'isododécane), ou non volatiles et leurs dérivés, tels que la vaseline, les polydécènes, le polyisobutène hydrogéné tel que le parléam, le squalane, et leurs mélanges.

Le milieu solvant peut être présent de préférence en une quantité de 1 à 90% en poids, en particulier de 5 à 70% en poids par rapport au poids total de la composition.

La solubilité des (co)polymères selon l'invention peut être contrôlée par le choix des squelettes polymériques -POL- et/ou des groupes de jonction (A).
Les (co)polymères de l'invention peuvent dans certains cas interagir entre eux physiquement (en établissant un réseau d'interactions H) dans certains solvants
ou mélanges de solvants. Cela dépend notamment de la nature et des proportions de solvants ou mélanges de solvants utilisés. Ceci peut provoquer une augmentation indésirable de la viscosité de la composition et éventuellement gêner son application (par exemple lotion, aérosol, etc....).
Pour s'affranchir de ce problème de viscosité, il est possible :
- de solubiliser le (co)polymère selon l'invention dans un solvant volatil pouvant établir des interactions H avec des groupes de jonction (A), par exemple en utilisant des alcools courts en C1-C4, des polyols volatils, de l'eau et/ou un mélange de ces solvants, ou
- d'utiliser un milieu solvant biphasique, comme par exemple une émulsion eau-dans-huile (E/H) ou huile-dans-eau (H/E). et un couple de (co)polymères selon l'invention sélectifs dont les groupes de jonction et les squelettes polymériques sont de nature chimique différentes, chaque polymère étant dissous dans une phase différente de l'autre (l'un dans l'eau, l'autre dans l'huile).
Dans ce dernier cas, le couple de (co)polymères A₁-POL₁-A₁ et A₂-POL₂-A₂ peut être avantageusement choisi de telle manière que :
- chacun des groupes de jonction (A₁) n'établit pas d'interaction H avec lui-même mais seulement avec (A₂),
- chacun des groupes de jonction (A₂) n'établit pas d'interaction H avec lui-même mais seulement avec (A₁),
- les groupes de jonction (A₁) et (A₂) n'établissent des interactions H que lorsqu'ils sont mis en contact, et
- les squelettes polymériques -POL₁- et -POL₂- sont choisis de manière telle que les (co)polymères A₁-POL₁- A₁et A₂-POL₂- A₂ peuvent être chacun véhiculés dans une phase distincte de l'émulsion, de sorte qu'ils ne puissent pas réagir dans l'émulsion.
L'interaction entre les deux (co)polymères ne se fera qu'à l'application, à condition toutefois que les milieux solvants soient des solvants volatils ou puissent pénétrer dans le support kératinique.

La composition de l'invention peut en outre comprendre les adjuvants habituellement employés dans les domaines cosmétique ou pharmaceutique, dans la mesure où l'adjuvant n'altère pas les propriétés recherchées pour la composition de l'invention, tels que les cires, les gommes, les tensioactifs, les épaississants, les gélifiants hydrophiles ou lipophiles, les actifs cosmétiques hydrophiles
ou lipophiles, les conservateurs, les antioxydants, les parfums, les agents nacrants, les charges, les neutralisants, les (co)polymères autres que ceux définis préalablement, les émulsionnants et les co-émulsionnants, les pigments, les matières colorantes.
Bien entendu l'homme du métier veillera à choisir ce ou ces éventuels adjuvants et/ou leur quantité, de manière telles que les propriétés avantageuses de la composition selon l'invention ne soient pas, ou substantiellement pas, altérées par l'adjonction envisagée.
Les quantités de ces différents adjuvants sont celles classiquement utilisées dans les domaines considérés, par exemple de 0,001 à 30% du poids total de la composition. Ces adjuvants, selon leur nature, peuvent être introduits dans la phase grasse, dans la phase aqueuse, dans les vésicules lipidiques et/ou dans les nanoparticules.

Par cire au sens de la présente invention, on entend un composé lipophile, solide à température ambiante (25°C), à changement d'état solide/liquide réversible, ayant un point de fusion supérieur ou égal à 25°C pouvant aller jusqu'à 120°C. En portant la cire à l'état liquide (fusion), il est possible de la rendre miscible aux huiles éventuellement présentes et de former un mélange homogène microscopiquement, mais en ramenant la température du mélange à la température ambiante, on obtient une recristallisation de la cire dans les huiles du mélange. Le point de fusion de la cire peut être mesuré à l'aide d'un calorimètre à balayage différentiel (D.S.C.), par exemple le calorimètre vendu sous la dénomination DSC 30 par la société METTLER.
Les cires peuvent être hydrocarbonées, fluorées et/ou siliconées et être d'origine végétale, minérale, animale et/ou synthétique. En particulier, les cires présentent un point de fusion supérieur à 30°C et mieux supérieur à 45°C. Comme cire utilisable dans la composition de l'invention, on peut citer la cire d'abeilles, la cire de Carnauba ou de Candellila, la paraffine, les cires microcristallines, la cérésine ou l'ozokérite; les cires synthétiques comme les cires de polyéthylène ou de Fischer Tropsch, les cires de silicones comme les alkyl ou alkoxy-diméthicone ayant de 16 à 45 atomes de carbone.

Les gommes sont généralement des polydiméthylsiloxanes (PDMS) à haut poids moléculaire ou des gommes de cellulose ou des polysaccharides et les corps pâteux sont généralement des composés hydrocarbonés comme les lanolines et leurs dérivés ou encore des PDMS.
La nature et la quantité des cires et/ou gommes sont fonctions des propriétés mécaniques et des textures recherchées. A titre indicatif, la composition peut comprendre 0,1 à 50% en poids de cires, par rapport au poids total de la composition et mieux de 1 à 30% en poids.

La composition selon l'invention peut en outre comprendre, dans une phase particulaire, des pigments et/ou des nacres et/ou des charges habituellement utilisés dans les compositions cosmétiques.
La composition peut également comprendre d'autres matières colorantes choisies parmi les colorants hydrosolubles ou les colorants liposolubles bien connus de l'homme du métier.
Par pigments, il faut comprendre des particules de toute forme, blanches ou colorées, minérales ou organiques, insolubles dans le milieu physiologique, destinées à colorer la composition.
Par charges, il faut comprendre des particules incolores ou blanches, minérales
ou de synthèse, lamellaires ou non lamellaires, destinées à donner du corps ou de la rigidité à la composition, et/ou de la douceur, de la matité et de l'uniformité au maquillage.
Par nacres, il faut comprendre des particules de toute forme irisées, notamment produites par certains mollusques dans leur coquille ou bien synthétisées.
Les pigments peuvent être présents dans la composition à raison de 0,01 à 25% en poids de la composition finale, et de préférence à raison de 3 à 10% en poids. Ils peuvent être blancs ou colorés, minéraux ou organiques. On peut citer les oxydes de titane, de zirconium ou de cérium, ainsi que les oxydes de zinc, de fer ou de chrome, le bleu ferrique, l'hydrate de chrome, le noir de carbone, les outremers (polysulfures d'aluminosilicates), le pyrophosphate de manganèse et certaines poudres métalliques telles que celles d'argent ou d'aluminium. On peut encore citer les pigments D&C et les laques couramment employées pour conférer aux lèvres et à la peau un effet de maquillage, qui sont des sels de calcium, de baryum, d'aluminium, de strontium ou de zirconium.
Les nacres peuvent être présentes dans la composition à raison de 0,01 à 20% en poids, de préférence à un taux de l'ordre de 3 à 10% en poids. Parmi les nacres envisageables, on peut citer la nacre naturelle, le mica recouvert d'oxyde de titane, d'oxyde de fer, de pigment naturel ou d'oxychlorure de bismuth ainsi que le mica titane coloré.
Parmi les colorants, liposolubles ou hydrosolubles, qui peuvent être présents dans la composition, seul ou en mélange, à raison de 0,001 à 15% en poids, de préférence 0,01 à 5% en poids et notamment de 0,1 à 2% en poids, par rapport au poids total de la composition, on peut citer le sel disodique de ponceau, le sel disodique du vert d'alizarine, le jaune de quinoléine, le sel trisodique d'amarante, le sel disodique de tartrazine, le sel monosodique de rhodamine, le sel disodique de fuchsine, la xanthophylle, le bleu de méthylène, le carmin de cochenille, les colorants halogéno-acides, azoïques, anthraquinoniques, le sulfate de cuivre ou de fer, le brun Soudan, le rouge Soudan et le rocou, ainsi que le jus de betterave et le carotène.
La composition selon l'invention peut comprendre en outre en outre une ou plusieurs charges, notamment en une teneur allant de 0,01 % à 50% en poids, par rapport au poids total de la composition, de préférence allant de 0,02% à 30% en poids. Les charges peuvent être minérales ou organiques de toute forme, plaquettaires, sphériques ou oblongues. On peut citer le talc, le mica, la silice, le kaolin, les poudres de polyamide (Nylon®), de poly-β-alanine et de polyéthylène, les poudres de polymères de tétrafluoroéthylène (Téflon®), la lauroyllysine, l'amidon, le nitrure de bore, les microsphères creuses polymériques telles que celles de chlorure de polyvinylidène/acrylonitrile comme l'Expancel® (Nobel Industrie), de copolymères d'acide acrylique (Polytrap® de la société Dow Corning) et les microbilles de résine de silicone (Tospearls® de Toshiba, par exemple), les particules de polyorganosiloxanes élastomères, le carbonate de calcium précipité, le carbonate et l'hydrocarbonate de magnésium, l'hydroxyapatite, les microsphères de silice creuses (Silica Beads® de Maprecos), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.

La composition peut comprendre en outre un polymère additionnel tel qu'un polymère filmogène. Selon la présente invention, on entend par "polymère filmogène", un polymère apte à former à lui seul ou en présence d'un agent auxiliaire de filmification, un film continu et adhérent sur un support, notamment sur les matières kératiniques. Parmi les polymères filmogènes susceptibles d'être utilisés dans la composition de la présente invention, on peut citer les polymères synthétiques, de type radicalaire ou de type polycondensat, les polymères d'origine naturelle et leurs mélanges, en particulier les polymères acryliques, les polyuréthanes, les polyesters, les polyamides, les polyurées, les polymères cellulosiques comme la nitrocellulose.

La composition peut également comprendre avantageusement au moins un agent tensioactif qui est généralement présent en une quantité comprise entre 0,01% et 50% en poids, de préférence entre 0,1% et 40% et encore plus préférentiellement entre 0,5% et 30%, par rapport au poids total de la composition.
Cet agent tensioactif peut être choisi parmi les agents tensioactifs anioniques, amphotères, non ioniques, cationiques ou leurs mélanges.
Les tensioactifs convenant à la mise en oeuvre de la présente invention sont notamment, seuls ou en mélange, :
- les tensioactifs anioniques parmi lesquels on peut citer, seuls ou mélanges, les sels (en particulier sels de métaux alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, α-oléfine-sulfonates, paraffine-sulfonates; les alkylsulfosuccinates, les alkyléthersulfosuccinates, les alkylamidesulfosuccinates; les alkylsulfosuccinamates; les alkylsulfoacétates ; les alkylétherphosphates; les acylsarcosinates; les acyliséthionates et les N-acyltaurates, le radical alkyle ou acyle de tous ces différents composés comportant de préférence de 8 à 24 atomes de carbone, et le radical aryle désignant de préférence un groupement phényle ou benzyle.
On peut également citer les sels d'acides gras tels que les sels des acides oléique, ricinoléique, palmitique, stéarique, les acides d'huile de coprah ou d'huile de coprah hydrogénée; les acyl-lactylates dont le radical acyle comporte 8 à 20 atomes de carbone; les acides d'alkyl D galactoside uroniques et leurs sels ainsi que les acides alkyl (C₆-C₂₄) éther carboxyliques polyoxyalkylénés, les acides alkyl(C₆-C₂₄)aryl éther carboxyliques polyoxyalkylénés, les acides alkyl(C₆-C₂₄) amido éther carboxyliques polyoxyalkylénés et leurs sels, en particulier ceux comportant de 2 à 50 groupements oxyde d'éthylène, et leurs mélanges.
- les tensioactifs non ioniques parmi lesquels on peut citer, seuls ou mélanges, les alcools, les alpha-diols, les alkylphénols ou les acides gras polyéthoxylés, polypropoxylés ou polyglycérolés, ayant une chaîne grasse comportant par exemple 8 à 18 atomes de carbone, le nombre de groupements oxyde d'éthylène ou oxyde de propylène pouvant aller notamment de 2 à 50 et le nombre de groupements glycérol pouvant aller notamment de 2 à 30.
On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl (C10-C14) amines ou les oxydes de N-acylaminopropylmorpholine.
- les tensioactifs amphotères parmi lesquels on peut citer, seuls ou mélanges, les dérivés d'amines secondaires ou tertiaires aliphatiques, dans lesquels le radical aliphatique est une chaîne linéaire ou ramifiée comportant 8 à 22 atomes de carbone et comprenant au moins un groupe anionique hydrosolubilisant (par exemple carboxylate, sulfonate, sulfate, phosphate ou phosphonate); on peut citer encore les alkyl (C₈-C₂₀) bétaïnes, les sulfobétaïnes, les alkyl (C₈-C₂₀) amidoalkyl (C₁-C₆) bétaïnes telles que la cocoamidopropylbétaïne ou les alkyl (C₈-C₂₀) amidoalkyl (C₁-C₆) sulfobétaïnes.
- les tensioactifs cationiques parmi lesquels on peut citer, seuls ou mélanges,
   A) les sels d'ammonium quaternaires de la formule générale (XVI) suivante : 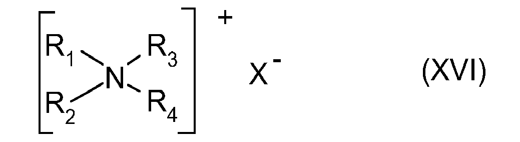 dans laquelle X est un anion choisi parmi les halogénures (chlorure, bromure ou iodure) ou alkyl(C₂-C₆)sulfates plus particulièrement méthylsulfate, des phosphates, des alkyl-ou-alkylarylsulfonates, des anions dérivés d'acide organique tel que l'acétate ou le lactate, et
      a) les radicaux R1 à R3, qui peuvent être identiques ou différents, représentent un radical aliphatique, linéaire ou ramifié, comportant de 1 à 4 atomes de carbone, ou un radical aromatique tel que aryle ou alkylaryle. Les radicaux aliphatiques peuvent comporter des hétéroatomes tels que notamment l'oxygène, l'azote, le soufre, les halogènes. Les radicaux aliphatiques sont par exemple choisis parmi les radicaux alkyle, alcoxy, alkylamide,
         R4 désigne un radical alkyle, linéaire ou ramifié, comportant de 16 à 30 atomes de carbone.
         De préférence le tensioactif cationique est un sel (par exemple chlorure) de béhényl triméthyl ammonium.
      b) les radicaux R1 et R2, qui peuvent être identiques ou différents, représentent un radical aliphatique, linéaire ou ramifié, comportant de 1 à 4 atomes de carbone, ou un radical aromatique tel que aryle ou alkylaryle. Les radicaux aliphatiques peuvent comporter des hétéroatomes tels que notamment l'oxygène, l'azote, le soufre, les halogènes. Les radicaux aliphatiques sont par exemple choisis parmi les radicaux alkyle, alcoxy, alkylamide et hydroxyalkyle, comportant environ de 1 à 4 atomes de carbone;
         R3 et R4, identiques ou différents, désignent un radical alkyle, linéaire ou ramifié, comportant de 12 à 30 atomes de carbone, ledit radical comprenant au moins une fonction ester ou amide.
         R3 et R4 sont notamment choisis parmi les radicaux alkyl(C₁₂-C₂₂)amido
         alkyle(C₂-C₆), alkyl(C₁₂-C₂₂)acétate ;
         De préférence le tensioactif cationique est un sel (par exemple chlorure) de stéaramidopropyl diméthyl (myristylacétate) ammonium.
   B) - les sels d'ammonium quaternaire de l'imidazolinium, comme par exemple celui de formule (XVII) suivante : 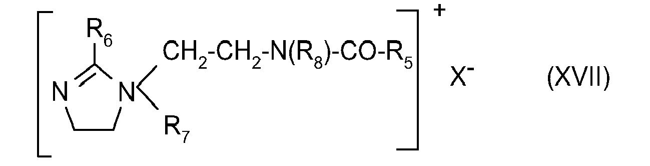 dans laquelle R₅ représente un radical alcényle ou alkyle comportant de 8 à 30 atomes de carbone par exemple dérivés des acides gras du suif, R₆ représente un atome d'hydrogène, un radical alkyle en C₁-C₄ ou un radical alcényle ou alkyle comportant de 8 à 30 atomes de carbone, R₇ représente un radical alkyle en C₁-C₄ , R₈ représente un atome d'hydrogène, un radical alkyle en C₁-C₄, X est un anion choisi dans le groupe des halogénures, phosphates, acétates, lactates, alkylsulfates, alkyl-ou-alkylarylsulfonates. De préférence, R₅ et R₆ désignent un mélange de radicaux alcényle ou alkyle comportant de 12 à 21 atomes de carbone par exemple dérivés des acides gras du suif, R₇ désigne méthyle, R₈ désigne hydrogène. Un tel produit est par exemple le Quaternium-27(CTFA 1997) ou le Quaternium-83 (CTFA 1997) commercialisés sous les dénominations "REWOQUAT" W 75, W90, W75PG, W75HPG par la société WITCO,
   C) - les sels de diammonium quaternaire de formule (XVIII) : 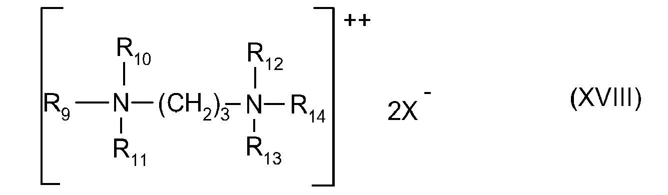 dans laquelle R₉ désigne un radical aliphatique comportant environ de 16 à 30 atomes de carbone, R₁₀, R₁₁, R₁₂, R₁₃ et R₁₄ , identiques ou différents sont choisis parmi l'hydrogène ou un radical alkyle comportant de 1 à 4 atomes de carbone, et X est un anion choisi dans le groupe des halogénures, acétates, phosphates, nitrates et méthylsulfates. De tels sels de diammonium quaternaire comprennent notamment le dichlorure de propanesuif diammonium.
   D) - les sels d'ammonium quaternaire comprenant au moins une fonction ester de formule (XIX ) suivante : 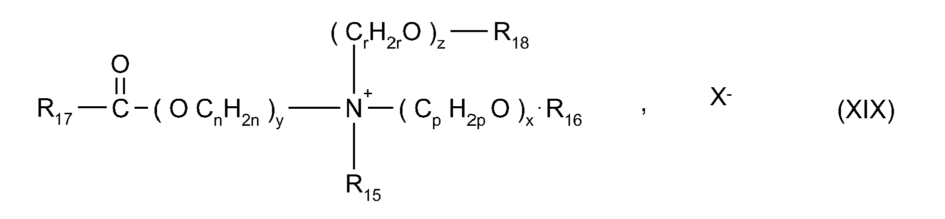 dans laquelle :
      - R15 est choisi parmi les radicaux alkyles en C1-C6 et les radicaux hydroxyalkyles ou dihydroxyalkyles en C1-C6 ;
      - R16 est choisi parmi l'hydrogène, le radical R₁₉-C(O)- et les radicaux R₂₀ hydrocarbonés en C1-C22 linéaires ou ramifiés, saturés ou insaturés,
      - R18 est choisi parmi l'hydrogène, le radical R₂₁-C(O)- et les radicaux R₂₂ hydrocarbonés en C1-C22 linéaires ou ramifiés, saturés ou insaturés:

      - R17, R19 et R21, identiques ou différents, sont choisis parmi les radicaux hydrocarbonés en C7-C22, linéaires ou ramifiés, saturés ou insaturés ;
      - n, p et r, identiques ou différents, sont des entiers valant de 2 à 6 ;
      - y est un entier valant de 1 à 10 ;
      - x et z, identiques ou différents, sont des entiers valant de 0 à 10 ;
      - X- est un anion simple ou complexe, organique ou inorganique ;
      sous réserve que la somme x + y + z vaut de 1 à 15, et lorsque x vaut 0 alors R16 désigne R20 et lorsque z vaut 0 alors R18 désigne R22.

Parmi les émulsionnants et les co-émulsionnants éventuellement utilisés dans la composition notamment sous forme d'émulsion, on peut citer, selon le sens de l'émulsion (E/H ou H/E), les esters d'acide gras et de polyol tels que le stéarate de PEG-100, le stéarate de PEG-50 et le stéarate de PEG-40 ; le tristéarate de sorbitane, les stéarates de sorbitane oxyéthylénés comprenant par exemple de 20 à 100 OE, et par exemple ceux disponibles sous les dénominations commerciales Tween® 20 ou Tween® 60, et leurs mélanges tels que le mélange de monostéarate de glycéryle et de stéarate de polyéthylène glycol (100 OE) commercialisé sous la dénomination SIMULSOL 165 par la société SEPPIC.
On peut citer aussi les émulsionnants siliconés tels que les diméthicone copolyols et les alkyl diméthicone copolyols. On peut citer par exemple comme diméthicone copolyol, le mélange de diméthicone copolyol, de cyclométhicone et d'eau (10/88/2), commercialisé par la société Dow Corning sous la dénomination DC3225C ou DC2-5225C, et comme alkyl diméthicone copolyol, ceux ayant un radical alkyle comportant de 10 à 22 atomes de carbone, tels que le cétyl diméthicone copolyol comme le produit commercialisé sous la dénomination Abil EM-90 par la société Goldschmidt et le mélange de diméthicone copolyol et de cyclopentasiloxane (85/15) commercialisé sous la dénomination Abil EM-97 par la société Goldschmidt ; le lauryl diméthicone copolyol et par exemple le mélange d'environ 91 % de lauryl diméthicone copolyol et d'environ 9% d'alcool isostéarylique, commercialisé sous la dénomination Q2-5200 par la société Dow Corning, et leurs mélanges.
Ces émulsionnants et co-émulsionnants sont de préférence présents, dans la composition, en une proportion allant de 0,3 à 30% en poids, et de préférence de 0,5 à 20% en poids par rapport au poids total de la composition.

Parmi les gélifiants hydrophiles, on peut citer en particulier les polymères carboxyvinyliques (carbomer), les copolymères acryliques tels que les copolymères d'acrylates/alkylacrylates, les polyacrylamides, les polysaccharides, les gommes naturelles et les argiles.
Comme gélifiants lipophiles, on peut citer les argiles modifiées comme les bentones, les sels métalliques d'acides gras et la silice hydrophobe.

Comme actifs cosmétiques, on peut utiliser notamment les dépigmentants, les émollients, les hydratants, les oligo-éléments, les anti-séborrhéiques, les anti-acnéiques, les agents favorisant la repousse des cheveux, les agents kératolytiques et/ou desquamants, les agents antirides et tenseurs, les agents anti-irritants, les agents apaisants, les vitamines, les filtres UV, les absorbeurs d'odeur, les anti-oxydants, les agents anti-chutes des cheveux, les agents antipelliculaires, les agents propulseurs, les céramides, et leurs mélanges.

Les compositions selon l'invention peuvent se présenter sous toutes les formes galéniques classiquement utilisées pour une application topique et notamment sous forme d'une solution, dispersion ou suspension aqueuse, alcoolique ou hydroalcoolique ou d'une solution huileuse, éventuellement épaissies ou gélifiées; d'une émulsion huile-dans-eau, eau-dans-huile, ou multiple, de consistance liquide ou semi liquide du type lait ou bien de consistance molle de type crème; d'un gel aqueux ou anhydre, d'une mousse; un gel huileux ou émulsionné; une dispersion de vésicules notamment lipidiques; une lotion biphase ou multiphase; d' un spray; ou de toute autre forme cosmétique.
L'homme du métier pourra choisir la forme galénique appropriée, ainsi que sa méthode de préparation, sur la base de ses connaissances générales, en tenant compte d'une part de la nature des constituants utilisés, notamment de leur solubilité dans le support, et d'autre part de l'application envisagée pour la composition.

La composition cosmétique selon l'invention peut se présenter sous la forme d'un produit de soin, de nettoyage et/ou de maquillage de la peau du corps ou du visage, des lèvres et des cheveux, d'un produit solaire ou autobronzant, d'un produit d'hygiène corporelle, d'un produit capillaire, notamment de soin, de nettoyage, de coiffage ou de coloration des cheveux.

Elle trouve notamment une application particulièrement intéressante dans le domaine capillaire, notamment pour le maintien de la coiffure ou la mise en forme des cheveux, ou encore le nettoyage des cheveux. Les compositions capillaires sont de préférence des shampooings, des après-shampooings, des gels de coiffage ou de soin, des lotions ou crèmes de soin, des conditionneurs, des lotions de mise en plis, des lotions pour le brushing, des compositions de fixation et de coiffage telles que les laques ou spray. Les lotions peuvent être conditionnées sous diverses formes, notamment dans des vaporisateurs, des flacons-pompe ou dans des récipients aérosol afin d'assurer une application de la composition sous forme vaporisée ou sous forme de mousse.

Elle peut également se présenter sous la forme d'un produit de coloration capillaire; ou sous forme de composition pour permanente, défrisage, ou décoloration, ou encore sous forme de compositions à rincer, à appliquer avant ou après une coloration, une décoloration, une permanente ou un défrisage ou encore entre les deux étapes d'une permanente ou d'un défrisage.

La composition selon l'invention peut également se présenter sous la forme d'une composition de soin, notamment hydratant, pour la peau, les lèvres et/ou les phanères, ou sous forme d'une composition de nettoyage de la peau, par exemple un produit démaquillant ou un gel pour le bain ou la douche.
Elle peut aussi se présenter sous forme d'un produit de soin, non coloré, destiné à traiter la peau et notamment à l'hydrater, la lisser, la dépigmenter, la nourrir, la protéger des rayons solaires, ou lui conférer un traitement spécifique. A cet effet, elle contient avantageusement au moins un actif de soin choisi parmi les dépigmentants, les émollients, les hydratants, les anti-séborrhéiques, les anti-acnéiques, les agents favorisant la repousse des cheveux, les agents kératolytiques et/ou desquamants, les agents antirides et tenseurs, les agents anti-irritants, les agents apaisants, les vitamines, les filtres, les absorbeurs d'odeur et leurs mélanges.
Elle peut également se présenter sous forme d'une composition d'hygiène corporelle notamment sous forme de produit déodorant, anti-transpirant, ou encore sous forme d'une composition dépilatoire.

Elle peut encore se présenter sous la forme d'un produit de maquillage, en particulier coloré, de la peau du corps ou du visage, ou des cheveux, en particulier un fond de teint, présentant éventuellement des propriétés de soin, un blush, un fard à joues ou à paupières, un produit anti-cerne, un eye-liner; un produit de maquillage des lèvres comme un rouge à lèvres, présentant éventuellement des propriétés de soin, un brillant à lèvres, les crayons à lèvres; un produit de maquillage des phanères comme les ongles, les cils en particulier sous forme d'un mascara pain, les sourcils et les cheveux; un produit de tatouage temporaire de la peau du corps.

L'invention a aussi pour objet un procédé cosmétique de traitement, notamment de maquillage, de soin, de nettoyage ou de coloration, des matières kératiniques, notamment de la peau du corps ou du visage, des ongles, des cheveux, des poils et/ou des cils, comprenant l'application sur lesdites matières d'une composition cosmétique telle que définie précédemment.
L'application peut éventuellement être suivie d'un rinçage à l'eau. Ainsi, ce procédé selon l'invention permet le maintien de la coiffure, le traitement cosmétique, le soin, le maquillage, le lavage ou le démaquillage, entre autre, de la peau, des cheveux et/ou de toute autre matière kératinique.

L'invention a également pour objet un procédé pour améliorer à la fois la persistance d'au moins un effet apporté après dépôt par une composition cosmétique et l'adhésion de la composition appliquée sur les matières kératiniques, et pour permettre également une élimination rapide, totale et sélective du dépôt, qui consiste à ajouter à la composition une quantité efficace d'au moins un (co)polymère tel que défini précédemment.
L'élimination du dépôt peut consister notamment en le rinçage d'une composition nettoyante ou en un démaquillage d'un dépôt de maquillage (rouge à lèvres, fond de teint, mascara, eye-liner notamment). Cette élimination peut être réalisée en utilisant un rupteur des interactions hydrogène.

L'invention est illustrée plus en détail dans les exemples suivants, dans lesquels les quantités sont données en pourcentage en poids.

### Exemple 1 : copolymère polyuréthanne-polyester à motif ionisable amine

1/ Préparation d'un monomère (I) de structure G1-L1-A-L2-G1 de formule :

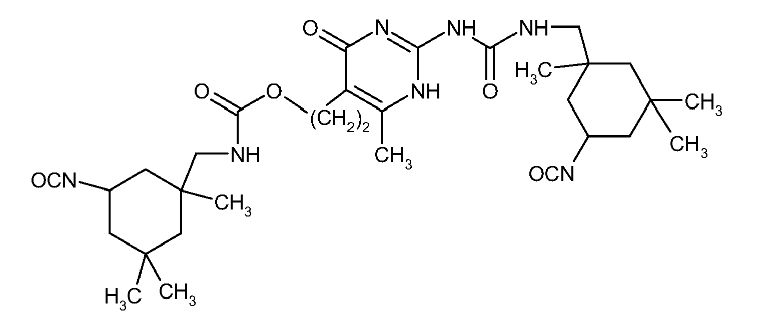

On met en suspension 12 g de 5-(2-hydroxyéthyl)-6-méthyl-isocytosine dans 150 ml d'isophorone diisocyanate (IPDI), et on laisse sous agitation sous argon pendant 12 heures, à 90°C.
On refroidit la solution limpide résultante et on la précipite dans l'hexane. On lave le précipité à l'hexane puis on l'isole par filtration, lavage et séchage.
On obtient 46 g de solide, soit un rendement de 93%.

2/ Préparation d'un copolymère polyuréthane-polyester, portant une fonction ionisable
On mélange 16,3 g (1 eq.) de poly(2-méthyl-1,3-propylène glutarate) de poids moléculaire moyen Mn=1,0 kDa (polymère téléchélique fonctionnalisé OH) dans 250 ml de chloroforme, avec 7,1 g (1 eq.) d'isophorone, 8,4 g (6/7 eq.) de monomère (I) préparé à l'étape précédente et 3,6 g (1 eq.) de N-méthyldiéthanolamine (motif ionisable).
On ajoute quelques gouttes de dilaurate de dibutyle étain (DBTDL), puis on chauffe à 60°C pendant 16 heures.
On précipite le polymère dans l'hexane puis on le sèche sous pression réduite.
On obtient le polymère recherché avec un rendement de 96%. La masse en nombre (Mn) du polymère obtenu est d'environ 13 000 Da.

### Exemple 2 : vernis à ongles

On solubilise 4,8 g de polymère préparé à l'exemple 1 dans 30 ml de THF; on ajoute 22 g d'acétate de butyle, puis on évapore le THF.
On obtient ainsi une solution visqueuse contenant 17,9% en poids de polymère. La viscosité peut être diminuée si nécessaire par addition de 2 g d'éthanol.

Après application sur l'ongle de cette solution, on obtient un film transparent et brillant.

### Exemple 3 : composition capillaire

On dissout 16 g de polymère préparé à l'exemple 1 dans 64 g de THF : le milieu est visqueux.
On ajoute 10 g d'éthanol, puis 14,4 g de HCl 1 N et 76,3 g d'eau, sous agitation avec un barreau magnétique, à température ambiante. On évapore le THF et l'éthanol à 50°C, à l'évaporateur rotatif.
On obtient une solution transparente de faible viscosité, légèrement jaunâtre.

Extrait sec final : 18,4% (en poids)
Taille de particule mesurée par diffusion de la lumière (via un appareil Coulter N4-SD) (après forte dilution) : 30 nm
pH=2,3

La solution peut ensuite être placée dans un flacon pompe et vaporisée sur les cheveux.
On observe une fixation de la chevelure, qui tient dans le temps; la chevelure est brillante.

### Exemple 4 : polymère (méth)acrylate à motif ionisable amine

1/ Préparation d'un monomère (I) de structure G1-L-A de formule : (appelé "acrylate de UPY") 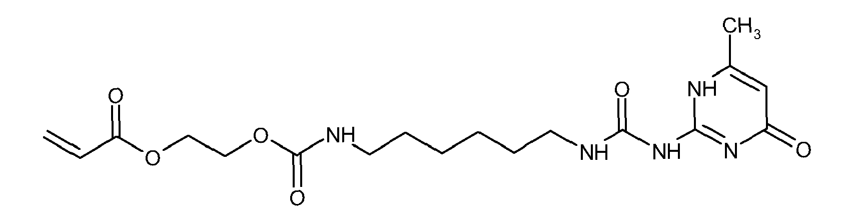 Le monomère peut être préparé par réaction de l'acrylate d'hydroxyéthyle sur OCN-(CH₂)₆-uréidopyrimidone, comme décrit dans le document US2004-0034190 et peut être schématisé ainsi : 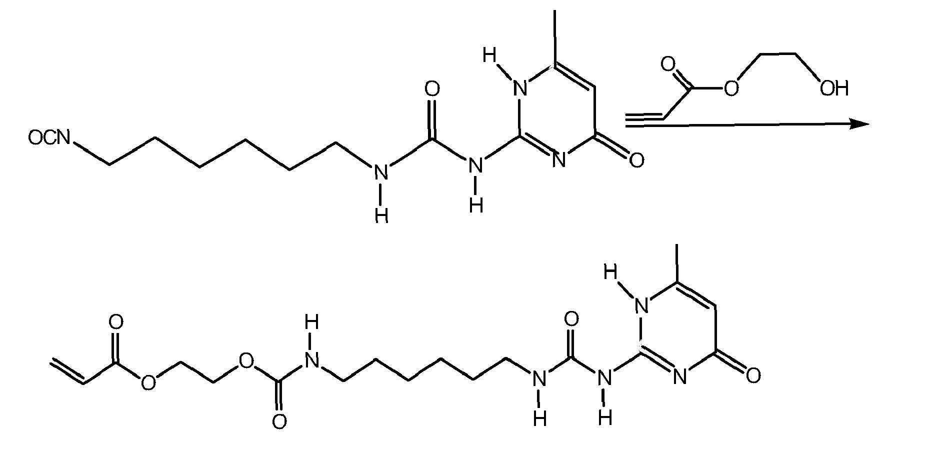 On met 46 g d'isocyanate en suspension dans 1 litre de chloroforme, on ajoute ensuite 36 ml d'acrylate d'hydroxyéthyle et 10 gouttes de dilaurate de dibutyl étain (DBTDL). Le mélange est agité à une température de bain d'huile de 90°C pendant 4 heures, et est ensuite refroidi et filtré. Le filtrat est concentré et on ajoute un excès de diéthyléther. On filtre et l'on obtient un précipité blanc, qui est lavé avec du diéthyléther, puis séché sous pression réduite.
On obtient 75 g d'un produit solide blanc, soit un rendement de 91%.

2/ Préparation d'un copolymère à base méthacrylate de diméthylaminoéthyle / acrylate de butyle / acrylate de "UPY"

On fait réagir, dans un mélange toluène 59,5 ml/DMSO 10,5 ml, les constituants suivants, à 60°C sous argon, pendant 8 heures.

| | |
|---|---|
| - Méthacrylate de diméthylaminoéthyle | 10,5 ml |
| - Acrylate de butyle | 24,5 ml |
| - Acrylate de UPY préparé ci-dessus | 6,24 g |
| - amorceur AIBN (azoisobutyronitrile) | 140 mg |
| - agent de transfert (dodécanethiol) | 1,4 ml |

Après réaction, on précipite la solution de polymère dans l'eau, puis on récupère le polymère qui est séché à l'étuve durant 12 heures.
On obtient 3,94 g de polymère soit un rendement de 96%.

### Exemple 5 : mise en dispersion dans l'eau/composition capillaire

On dissout 10 g de polymère préparé à l'exemple 4 dans 50 g de THF : le milieu est visqueux.
On ajoute 16 ml de HCl 1 N et 50 g d'eau, sous agitation avec un barreau magnétique, à température ambiante. On évapore le THF à 50°C, à l'évaporateur rotatif.
On obtient une solution transparente de faible viscosité, légèrement jaunâtre.

Extrait sec final : 20% (en poids)
Taille de particule mesurée par diffusion de la lumière (via un appareil Coulter N4-SD) (après forte dilution) : 100 nm
pH=2,5

La solution peut ensuite être placée dans un flacon pompe et vaporisée sur les cheveux.
On observe une fixation de la chevelure, qui tient dans le temps; la chevelure est brillante.

### Exemple 6 : copolymère polyuréthanne-polyester à motif ionisable acide

On sèche pendant 2 heures, sous pression réduite, 20,7 g de polymère téléchélique dihydroxy : poly(2-méthyl-1,3-propyène adipate) de Mn = 2.0 kD, puis on le met en solution dans 100 ml d'un mélange 1vol/1vol THF/Méthyléthylcétone, avec 0,69 g d'acide dimethylolpropionique (motif ionisable).
Le mélange est chauffé à 80°C sous argon. Dès qu'on obtient une solution limpide, on ajoute 1,15 g d'isophorone diisocyanate (IPDI) et 4 gouttes de dibutyl dilaurate d'étain. Le mélange est agité et chauffé à 80°C, toujours sous argon durant 2 heures.
On ajoute alors 5,46 g de monomère (I) préparé à l'exemple 1, et le mélange est laissé à 80°C, sous agitation et sous argon pendant 16 heures. Le volume réactionnel est diminué de moitié par évaporation au rotavaporateur, dilué dans 50 ml de toluène et agité sous argon à 80°C pendant 16h additionnelles. Par spectroscopie IR, on vérifie la disparition de la bande de l'isocyanate.
On ajoute alors 10 ml de méthanol et on précipite le polymère désiré dans 1 litre d'hexane. On sèche sous pression réduite et on obtient 26,8 de polymère; soit un rendement de 96%.

### Exemple 7 : Application vernis à ongle

On met en solution 8,2 g de polymère préparé à l'exemple 6, dans un mélange de 40 g d'acétate de butyle et de 9 g d'éthanol pour obtenir une viscosité adéquate pour une application vernis à ongle. Après agitation à température ambiante, on obtient une solution limpide contenant 13% en poids de polymère.
Après application sur l'ongle, on obtient un film brillant.

### Exemple 8 : copolymère polyuréthane-PDMS à motif ionisable amine

On met en solution 31 g de polymère PDMS téléchélique di-OH, vendu sous la dénomination KF003 par Shin-Etsu (Mn= 5 kDa) dans 120 ml de toluène sec, en présence de 1,5 g de N-méthyl-diéthanolamine (3,94% en poids de motif ionisable), de dilaurate de dibutyle étain en quantité catalytique et 2,8 g d'isophorone diisocyanate. Cette solution est chauffée à 80°C sous argon pendant 2 heures.
On y ajoute 30 ml de pyridine et 2,80 g de monomère (I) préparé dans l'exemple 1, et on chauffe sous argon pendant 16 heures supplémentaires.
Le milieu réactionnel est concentré à 50% de son volume initial, et strippé successivement avec 2x50ml de toluène. Le milieu réactionnel est ensuite dissout dans un mélange de solvant chloroforme/méthanol (9 volume/1volume) et précipité dans le méthanol (volume du méthanol 10 fois en excès par rapport au volume du milieu réactionnel).
On obtient 38,5 g de produit final après filtration et séchage sous pression réduite.
Ce produit est caractérisé par GPC (THF) Mn = 14 kDa avec un PD de 1,7.

### Exemple 9 : Application maquillage (fond de teint et rouge à lèvre)

Le polymère de l'exemple 8 est mis en solution à 20% dans la décaméthylcyclopentasiloxane (D5).
Après application sur la peau , on obtient un film confortable.

### Exemple 10 : Application Maquillage (fond de teint et rouge à lèvre)

Le polymère de l'exemple 8 peut aussi être partiellement protoné, afin de diminuer la viscosité d'une solution d'huile silicone le comprenant.
Ainsi, on prépare une solution à 10% de polymère de l'exemple 8 dans la décaméthylcyclopentasiloxane (D5). On y ajoute une solution HCl (1M) dans l'éther (0,32 ml) pour 1 g de polymère), afin de protoner partiellement l'amine présente sur le squelette polymérique. La solution est agitée pendant 3 heures à température ambiante, flacon ouvert. La viscosité de la solution disparaît.
Cette solution huileuse peut être appliquée sur la peau. Elle forme un film confortable.

## Revendications

1. Composition cosmétique ou pharmaceutique comprenant, dans un milieu physiologiquement acceptable, au moins un (co)polymère comportant :
(a) un squelette polymérique -POL- comprenant au moins deux motifs de répétition,
(b) au moins un groupe de jonction (A) lié audit squelette polymérique et capable d'établir des liaisons H avec un ou plusieurs groupes de jonction partenaires, de nature chimique identique ou différente, chaque appariement d'un groupe de jonction faisant intervenir au moins 3 liaisons H, de préférence au moins 4 liaisons H, préférentiellement 4 liaisons H,
**caractérisé en ce que** ledit (co)polymère comporte au moins un groupe ionisable.

2. Composition selon la revendication 1, dans laquelle ledit (co)polymère résulte de la polymérisation de monomères de formule (I) et facultativement de monomères de formule (II) selon le schéma réactionnel : 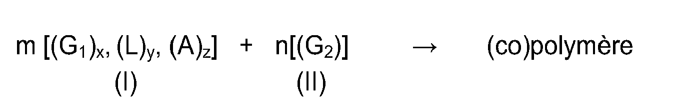 dans lequel :
- les groupements G₁, identiques ou différents, sont des groupements (co)polymérisables capables de former un lien covalent avec un autre groupement (co)polymérisable G₁ d'un autre monomère (I) et/ou avec un groupement (co)polymérisable porté par un monomère G2;
- les groupes A, identiques ou différents, sont des groupes de jonction capables de former au moins trois liaisons H, de préférence au moins 4 liaisons H, préférentiellement 4 liaisons H;
- les bras L, identiques ou différents, sont des bras de liaison divalents, y compris une liaison covalente simple, reliant un groupe de jonction A à un groupement G₁ ;
- x est un entier supérieur ou égal à 1, de préférence variant de 1 à 12, et mieux x est égal à 1 ou 2 ;
- y est un entier supérieur ou égal à 1, de préférence variant de 1 à 12, et mieux y est égal à 1 ou 2 ;
- z est un entier supérieur ou égal à 1, de préférence variant de 1 à 6, et mieux z est égal à 1 ;
- G₂, identiques ou différents, sont des monomères exempts de groupe de jonction A et comportant au moins un groupement (co)polymérisable capable de former un lien covalent avec un groupement (co)polymérisable G₁ d'un monomère (I) et/ou avec un groupement (co)polymérisable porté par un autre monomère G2, identique ou différent;
- m est le nombre de moles de monomères, identiques ou différents, de formule (I), homopolymérisés ou copolymérisés et est un entier allant de 1 à 12, de préférence compris entre 2 et 8, mieux entre 2 et 6 ;
- n est le nombre de moles de monomères, identiques ou différents, de formule (II), homopolymérisés ou copolymérisés, et est un entier allant de 0 à 20000, de préférence de 1 à 10000 et mieux de 1 à 5000 ; et m+n ≥ 2, de préférence ≥ 4;
étant entendu que l'un au moins des groupes A et/ou groupements G1 et/ou bras L et/ou monomère G2, comporte au moins un groupe ionisable.

3. Composition selon l'une des revendications précédentes, dans laquelle le (co)polymère est formé par réaction d'au moins un monomère de formule G₁-L-A et/ou G₁-L-A-L-G₁ et/ou G₁-L(A)-G₁ avec au moins un monomère de formule G₂, les groupements G₁, les bras de liaison L, les groupes de jonction A et les monomères G2 pouvant être identiques ou différents et étant définis comme dans la revendication 2.

4. Composition selon l'une des revendications précédentes, dans laquelle les (co)polymères comportent au moins 2, de préférence au moins 4, et mieux au moins 6 groupes de jonction.

5. Composition selon l'une des revendications précédentes, dans laquelle le groupe de jonction A est un groupe chimique, notamment carboné, comportant au moins 3 hétéroatomes, identiques ou différents, de préférence au moins 4 hétéroatomes, voire 4 hétéroatomes, identiques ou différents, choisis dans le groupe constitué de O, N, S, P et F, de préférence parmi O, S et N.

6. Composition selon l'une des revendications précédentes, dans laquelle le groupe de jonction comprend au moins 3 groupes fonctionnels, de préférence au moins 4, voire 4 groupes fonctionnels, choisis parmi :

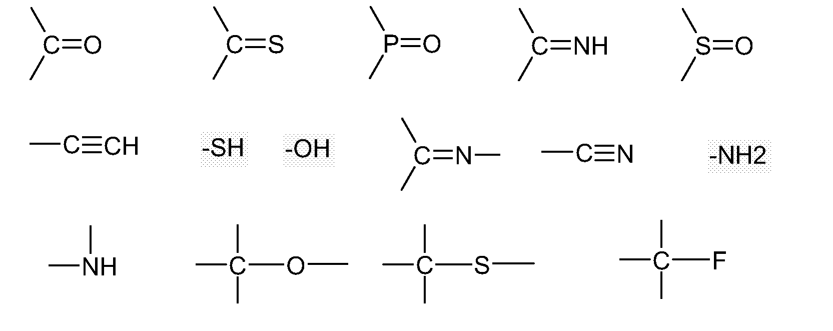

7. Composition selon l'une des revendications précédentes, dans laquelle le groupe de jonction comporte des cycles à 5 ou 6 atomes (cycles aromatiques
ou hétérocycles insaturés) constitués d'atomes de C et/ou N, avec des doubles liaisons conjuguées.

8. Composition selon l'une des revendications précédentes, dans laquelle les groupes de jonction (A) sont choisis parmi les familles suivantes, étant entendu que toutes les formes tautomériques sont inclues :
- (i) les aminopyrimidones de formule : 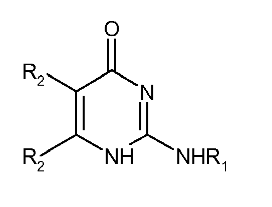
- (ii) les ureïdopyrimidones de formule : 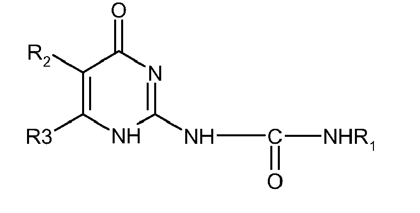
- (iii) les acylaminopyridines et notamment :
- les monoacylaminopyridines de structure : 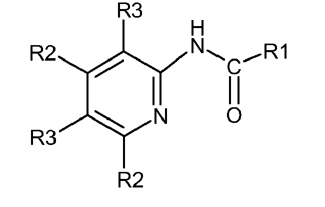
- les di(acylamino)pyridines et plus particulièrement les 2,6-di(acylamino)pyridines de structure : 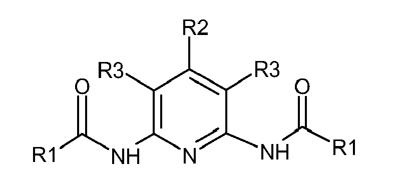
- (iv) les aminopyrimidines, et notamment :
- les composés aminopyrimidines : 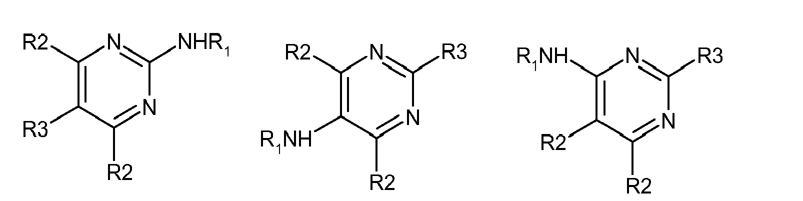
- les composés diaminopyrimidines de structure : 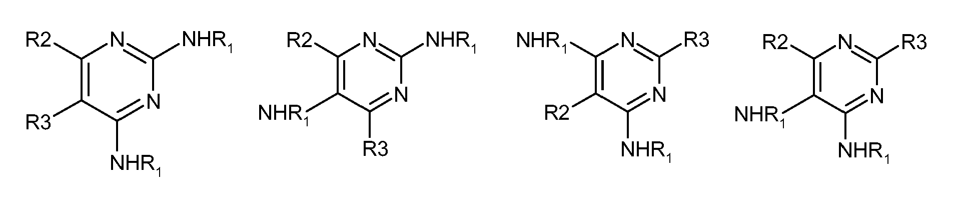
- les composés triaminopyrimidines;
- (v) les uréïdotriazines, et notamment les mono-, di- et tri-uréïdotriazines, et en particulier les uréïdoaminotriazines de structure : 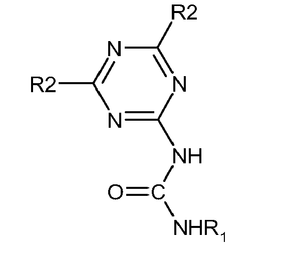
- (vi) les (acylamino)triazines, et notamment les mono-, di- et tri-acylamino triazines, éventuellement amino (mono-, di- ou tri-amino) et en particulier :
- les di(acylamino)triazines de structure : 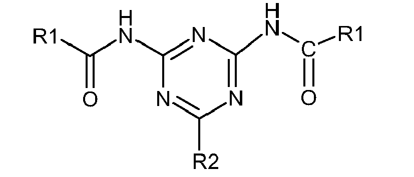
- les acylamino, amino-triazines, (mono ou di- acylamino, et mono- ou di-amino) et notamment les composés de structure : 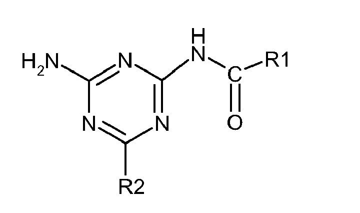
- les acylaminotriazines de structure : 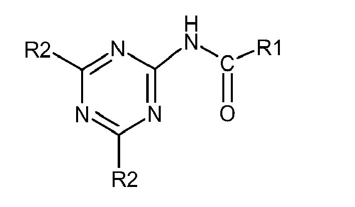
- les tri-acylamino triazines,
- (vii) les aminotriazines et notamment :
- les monoaminotriazines,
- les 2,6-diamino-s-triazines de structure : 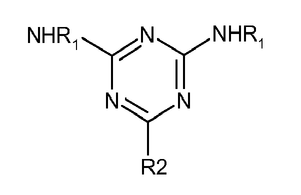
- les composés triamino-s-triazines de structure : 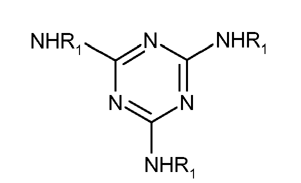
- (viii) les acylaminotriazoles de structure : 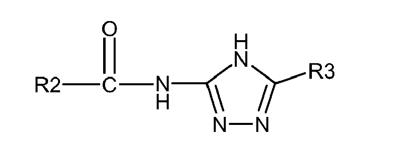
- (ix) les composés de la famille de l'acide urazoyl benzoïque de structure : 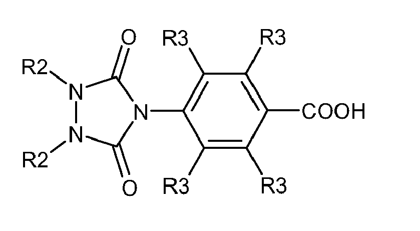
- (x) les phtalhydrazides de structure : 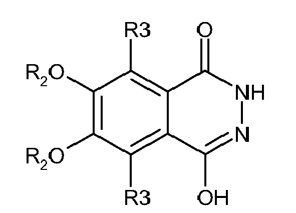
- (xi) les uraciles de structure : 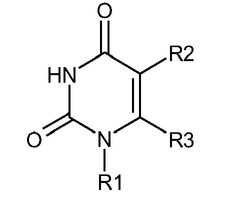
- (xii) les thymines de structure : 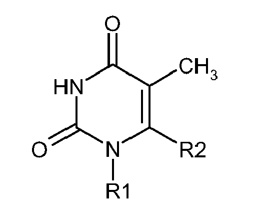
- (xiii) les succinimides de structure : 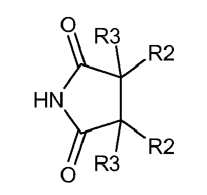
- (xiv) les glutarimides de structure : 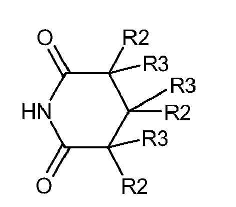
- (xv) les composés de la famille de l'acide cyanurique de structure : 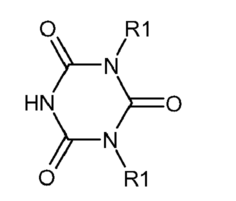
- (xvi) les maléimides : 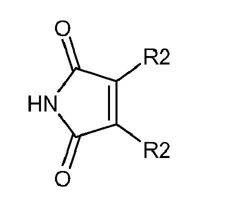
- (xvii) les composés de la famille de l'acide barbiturique, de structure : 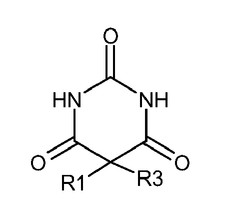
- (xviii) les composés de structure : 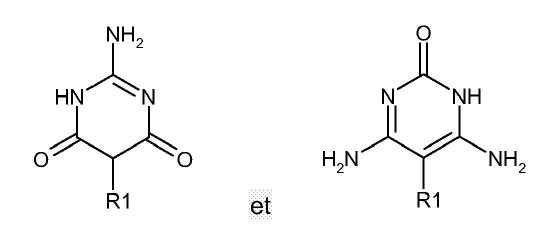
- (xix) les composés de la famille de l'acide triméllitique, de formule : 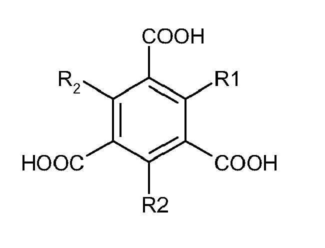
- (xx) les uréïdopyridines, notamment les mono- ou di-uréïdopyridines, et en particulier ceux de formule : 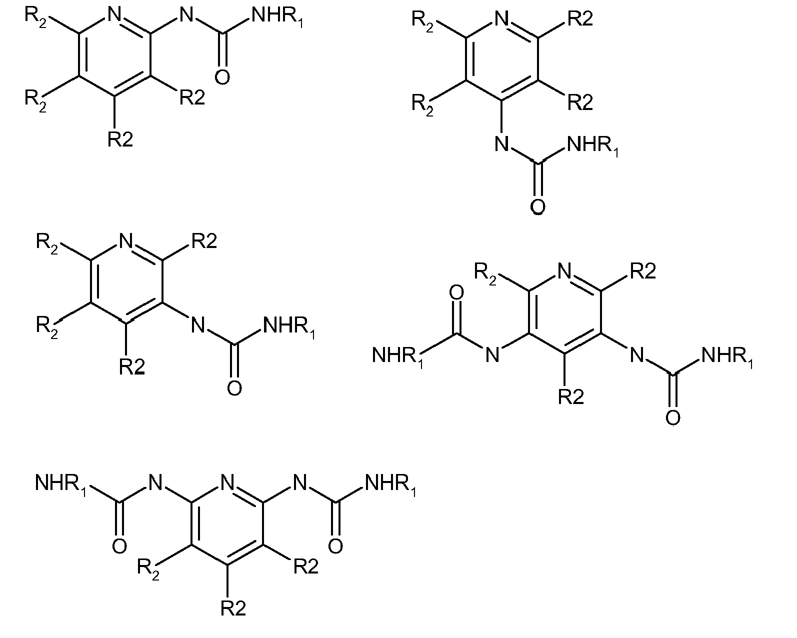
- (xxi) les carbamoylpyridines, de formule : 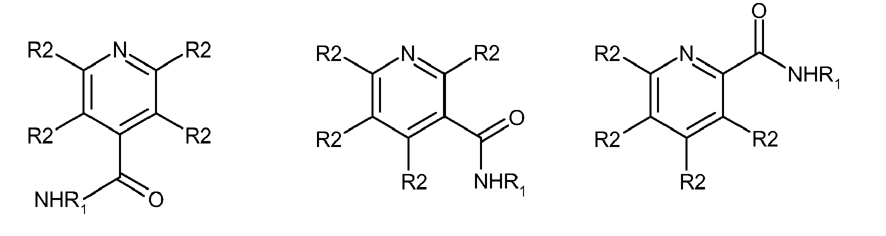
- (xxii) les adénines de formule : 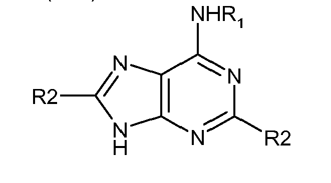
- (xxiii) les guanines de formule : 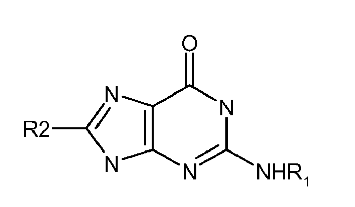
- (xxiv) les cytidines de formule : 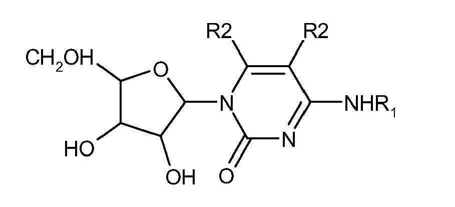 dans lesquelles la signification des radicaux est la suivante :
- (a) les radicaux R¹, identiques ou différents, représentent H, halogène et/ou un groupe carboné (notamment alkyle) monovalent, linéaire, ramifié ou cyclique, en C₁-C₆₀₀₀, saturé ou non, éventuellement aromatique, pouvant contenir un ou plusieurs hétéroatomes tels que O, S, N, P, Cl, Br, Cl, Br, F; ou une combinaison de ces significations; en particulier R¹ peut être un groupe cycloalkyle en C₄-C₁₂ ; un groupe alkyle linéaire ou ramifié en C₁-C₃₀ ou un groupe aryle en C₄-C₁₂ ; éventuellement substitués par une fonction amino, ester et/ou hydroxy.
- (b) les radicaux R2, identiques ou différents au sein d'une même formule, représentent H, halogène (-BR, -Cl, -F), -OH, -N(R)2 (avec R étant H ou un radical alkyle, linéaire ou ramifié en C1-C12, de préférence en C1-C4 et mieux un radical méthyle ou éthyle); ou un groupe hydrocarboné monovalent, linéaire, ramifié ou cyclique, en C1-C6000, saturé ou non, éventuellement aromatique, pouvant contenir un ou plusieurs hétéroatomes tels que O, S, N, P, F; ou une combinaison de ces significations;
- (c) les radicaux R³, identiques ou différents au sein d'une même formule, représentent H ou un groupe hydrocarboné monovalent, linéaire, ramifié ou cyclique, en C₁-C₆₀₀₀, saturé ou non, éventuellement aromatique, pouvant contenir un ou plusieurs hétéroatomes tels que O, S, N, P, F; ou une combinaison de ces significations;
étant entendu qu'au moins un, notamment un ou deux, des groupes R¹ et/ou R² est le point d'accroche du groupe de jonction A sur le squelette polymérique - POL-.

9. Composition selon la revendication 8, dans laquelle :
-i) R₁ est un groupement C₄H₉, phényle, 1,4-nitrophényle, 1,2-éthylène, 1,6-héxylène, 1,4-butylène, 1,6-(2,4,4-triméthylhexylène), 1,4-(4-méthylpentylène), 1,5-(5-méthylhexylène), 1,6-(6-méthylheptylène), 1,5-(2,2,5-triméthylhexylène), 1,7-(3,7-diméthyloctylène); -isophorone-, 4,4'-méthylène biscyclohexylène, 2-méthyl-1,3-phénylène, 4-méthyl-1,3-phénylène, 4,4-biphénylèneméthylène; de préférence R1 est un groupement -isophorone-, -(CH₂)₂-, -(CH₂)₆-, - CH₂CH(CH₃)-CH₂-C(CH₃)₂-CH₂-CH₂, 4,4'-méthylène biscyclohexylène ou 2-méthyl-1,3-phénylène; et/ou
-ii) R2 est H, CN, NH₂, ou bien :
- un groupe alkyle en C1-C30, notamment CH₃, C₁₃H₂₇, C₇H₁₅
- un groupe cycloalkyle en C₄-C₁₂,
- un groupe aryle en C₄-C₁₂, notamment phényle
- un groupe aryl(C4-C12)alkyle en C1-C30,
- un groupe alcoxy en C₁-C₄ ;
- un groupe arylalcoxy, en particulier un groupe aryle (C₁-C₄) alcoxy ;
- un hétérocycle en C4-C12;
- un groupe thioalcoxy,
- un groupe sulfoxy,
- leurs mélanges/combinaisons;
ces groupes étant éventuellement substitués par une fonction amino, ester et/ou hydroxy; et/ou
-iii) R3 est un groupe cycloalkyle en C₄-C₁₂; un groupe alkyle linéaire ou ramifié en C1-C30 ou un groupe aryle en C₄-C₁₂; éventuellement substitué par une fonction amino, ester et/ou hydroxy; et/ou
-iv) ledit point d'accroche est porté par R₁ et/ou R₂ et de préférence, lorsque le point d'accroche est unique, il est porté par le groupe R₁.

10. Composition selon l'une des revendications 8 à 9, dans laquelle le groupe de jonction est choisi parmi :
(a) les groupes de jonction (A) complémentaires et identiques c'est-à-dire auto-complémentaires, et notamment :
- les aminopyrimidones, les uréïdopyrimidones,
- les composés de la famille de l'acide triméllitique, ou de l'acide urazoyl benzoïque,
- les acylaminopyridines, les uréïdopyridines, les carbamoylpyridines,
- les acylaminotriazines, les uréidotriazines et notamment les uréïdoaminotriazines, les diamino-triazines,
- les acylaminotriazoles,
- les phtalhydrazides,
- les composés de formule : 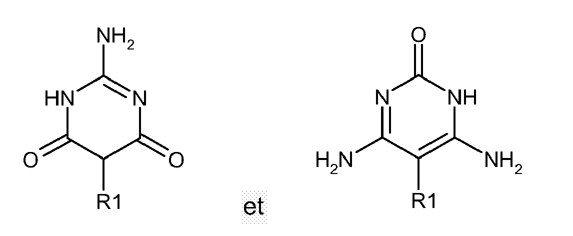
dans lesquelles R¹ est H ou un groupe hydrocarboné monovalent, linéaire, ramifié ou cyclique, en C₁-C₆₀₀₀, saturé ou non, éventuellement aromatique, pouvant contenir un ou plusieurs hétéroatomes tels que O, S, N, P, F.
(b) les groupes de jonction (A) complémentaires mais différents, et notamment:
- adénine complémentaire de guanine,
- cytidine complémentaire de thymine,
- triamino-s-triazine complémentaire de uracile ou de succinimide ou de glutarimide ou d'acide cyanurique ou de thymine ou de maléimide ou de (di)aminopyrimidine ou d'acide barbiturique;
- acylamino-amino-s-triazine complémentaire de uracile ou de succinimide ou de glutarimide ou d'acide cyanurique ou de thymine ou de maléimide ou de (di)aminopyrimidine ou d'acide barbiturique.

11. Composition selon l'une des revendications 8 à 10, dans laquelle les groupes de jonction (A) sont choisis parmi les groupes capables d'établir au moins trois liaisons H avec eux-mêmes (auto-complémentaire), notamment au moins quatre liaisons H avec eux-mêmes, et en particulier parmi :
- les uréïdopyrimidones;
- les uréïdopyridines, les carbamoylpyridines;
- les acylamino-s-triazines et notamment les acyl-diamino-s-triazines;
- les uréidotriazines ;
- les phtalhydrazides;
- les composés de formule : 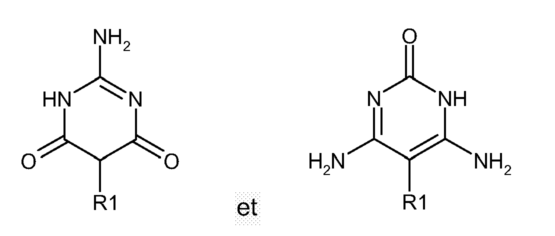 Dans lesquels les radicaux R1, R2 et R3 ont les significations données ci-dessus.

12. Composition selon l'une des revendications précédentes, dans laquelle les groupes de jonction (A) sont choisis parmi les groupes suivants:
- la 2-uréïdopyrimidone;
- la 6-méthyl, 2-uréïdopyrimidone;
- la diacyl de 2,6-diamino-s-triazine;
- l'uréido-s-triazine;
- les composés de formule : 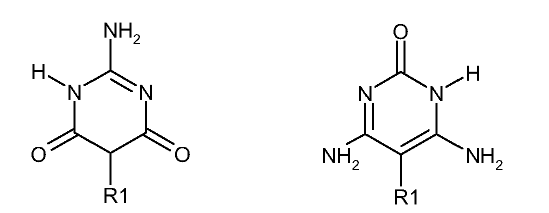
dans lesquelles R¹ est H ou un groupe hydrocarboné monovalent, linéaire, ramifié ou cyclique, en C₁-C₆₀₀₀, saturé ou non, éventuellement aromatique, pouvant contenir un ou plusieurs hétéroatomes tels que O, S, N, P, F.

13. Composition selon l'une des revendications précédentes, dans laquelle le groupe de jonction (A) porte au moins un groupe ionisable.

14. Composition selon l'une des revendications 2 à 13, dans laquelle ledit bras de liaison L est une liaison covalente simple ou bien est choisi parmi les groupes carbonés divalents, saturés ou insaturés, linéaires, ramifiés ou cycliques (y compris aromatiques), notamment alkyles, ainsi que leurs combinaisons, ayant de 1 à 6000 atomes de carbone, notamment 1 à 30, et pouvant éventuellement comprendre un ou plusieurs, de préférence 1 à 5, hétéroatomes identiques ou différents choisis parmi O, N, S, P, F, de préférence O, N et S.

15. Composition selon la revendication 14, dans laquelle les bras de liaison L sont des groupes carbonés, notamment alkylènes (alkyles divalents) en C1-C30, pouvant comprendre des groupements fonctionnels choisis parmi :

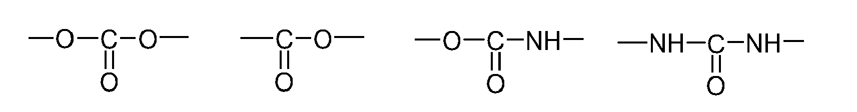

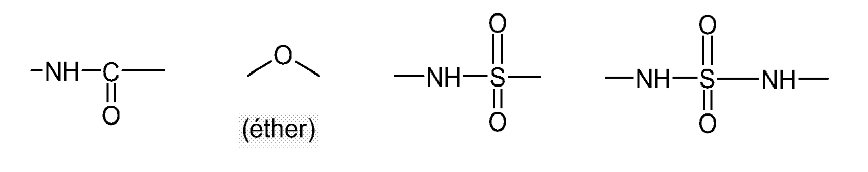

16. Composition selon l'une des revendications 14 à 15, dans laquelle le bras de liaison L, lorsqu'il n'est pas une liaison simple, porte au moins un groupe ionisable.

17. Composition selon l'une des revendications 2 à 16, dans laquelle ledit groupement (co)polymérisable G₁ est choisi parmi :
- les groupements (co)polymérisables comportant au moins une double liaison éthylénique capable de (co)polymériser par voie radicalaire, anionique ou cationique, par exemple un groupement CH₂=C(CH3)- ou CH₂=CH- ;
- les groupements (co)polymérisables par réaction de substitution ou d'addition, nucléophile ou électrophile, ou d'addition radicalaire et de préférence par polyaddition ou polycondensation, choisis par exemple parmi les groupements comportant une fonction hydroxyle (OH) ou hydroxyle activé tel que tosylate, thiol (SH), halogénure (Br, Cl), amine primaire ou secondaire (NH₂ ou NHR), ester (COOR), acide carboxylique (COOH), acide activé tel que COHal, isocyanate (NCO) protégé ou non, isothiocyanate (NCS), -C=C- , -C(O)H, -SiH, succinimide, oxazoline, acétal, hemiacétal, chlorotriazine, -SO₂Cl, époxyde; le radical R étant un alkyle en C1-6, de préférence méthyle.
- les groupements (co)polymérisables par ouverture, anionique ou cationique, de cycles, tels que des groupements comportant au moins un groupe éther cyclique, ester cyclique, amide cyclique ou carbonate cyclique.

18. Composition selon l'une des revendications 2 à 17, dans laquelle le groupement G1 porte au moins un groupe ionisable.

19. Composition selon l'une des revendications 2 à 18, dans laquelle le monomère de formule (I) est choisi parmi :
- i) les monomères (co)polymérisables par voie radicalaire, anionique ou cationique qui peuvent être représentés par la formule : 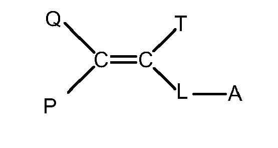 où P, Q et T représentent, indépendamment les uns des autres, un atome d'hydrogène, un radical hydrocarboné, linéaire ou ramifié, cyclique ou non, saturé ou non, ou aromatique, de préférence alkyle, comportant 1 à 12 atomes de carbone ou un groupement -L-A, L et A étant tels que définis précédemment.
-ii) les monomères (co)polymérisables par addition ou polycondensation, comportant au moins un groupement G₁, de préférence deux groupements G₁, pouvant (co)polymériser par addition ou polycondensation; de préférence comportant 1 ou 2 groupements A-L- ou -L-A-L-, de préférence un groupement A-L- ou -L-A-L-.
-iii) les monomères (co)polymérisant par ouverture de cycle, et comportant des groupes G₁ choisis parmi :
- des éthers cycliques de formule : 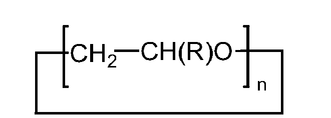 où R est H ou un radical hydrocarboné, linéaire ou ramifié, cyclique ou non, saturé ou non, ou aromatique, de préférence un alkyle, en C₁-C₁₂ et n est un entier de 1 à 3. De préférence R est H ou CH₃ et n = 1.
- des amides cycliques de formule : 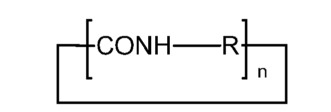 dans laquelle R est -(CH₂)ₘ- et m est un entier de 3 à 12, de préférence égal à 3 ou 5 et n est un entier de 1 à 3, de préférence égal à 1.
- des esters cycliques de formule : 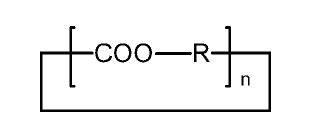 dans laquelle R est -(CH₂)ₘ- et m est un entier de 3 à 12, de préférence égal à 3 ou 5 et n est un entier de 1 à 3, de préférence égal à 1.
- des carbonates cycliques de formule : 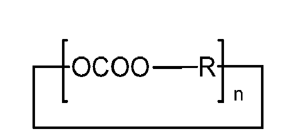 dans laquelle R est -(CH₂)ₘ- et m est un entier de 3 à 12, de préférence égal à 3 ou 5 et n est un entier de 1 à 3, de préférence égal à 1.
- des perfluoroéthers cycliques, des lactides, des oxazolines, le norbonène, ainsi que leurs dérivés;
- leurs combinaisons.

20. Composition selon la revendication 19, dans laquelle le monomère (I) est choisi parmi :
- les monomères (co)polymérisables par voie radicalaire, anionique ou cationique, de formule : 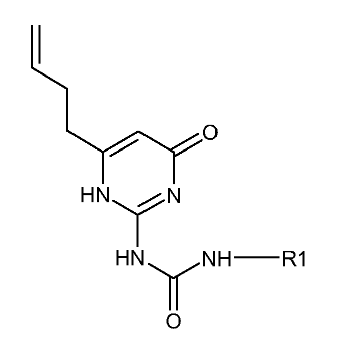
dans laquelle R₁ est tel que défini précédemment,
- l'acrylate dérivé du 6-méthyl 2-uréïdo-pyrimidone de formule : 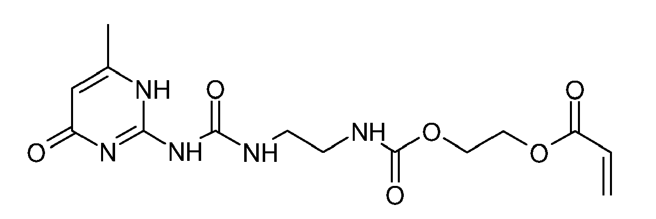
- les monomères de formule :
A-(CH₂)₂-CH=CH₂ 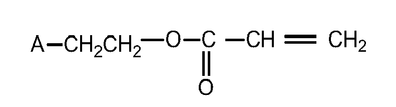 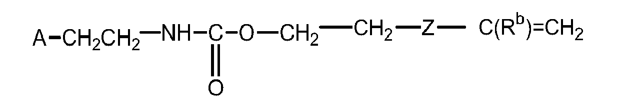 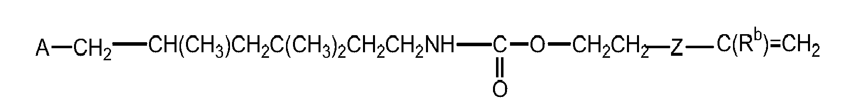 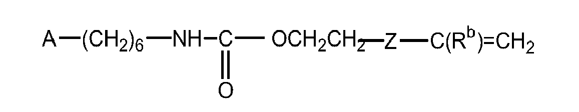 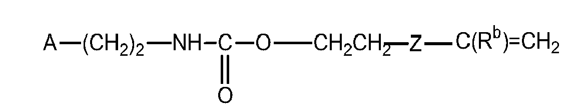 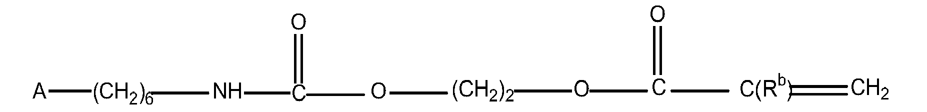 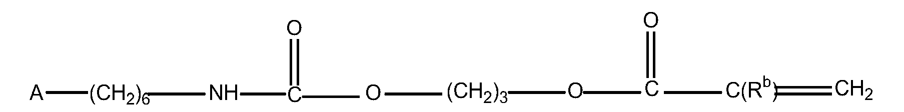 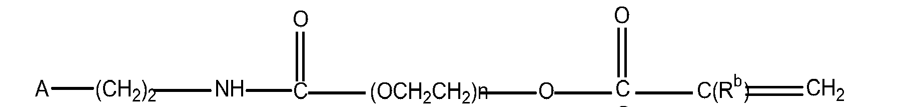 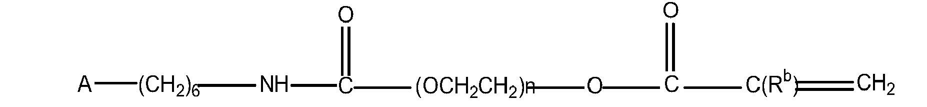 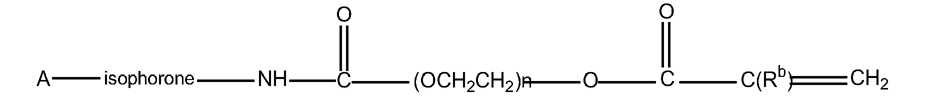
dans lesquelles :
- Z représente -O-C(O)- ou -NH-C(O)-
- n est un entier variant de 1 à 500
- R^{b} est H ou CH3 et
- A est le groupe : 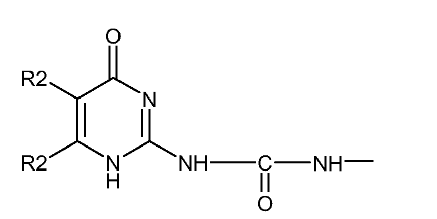
où les R₂, identiques ou différents, sont tels que définis ci-dessus.
- les monomères (co)polymérisables par polyaddition ou polycondensation de formule: 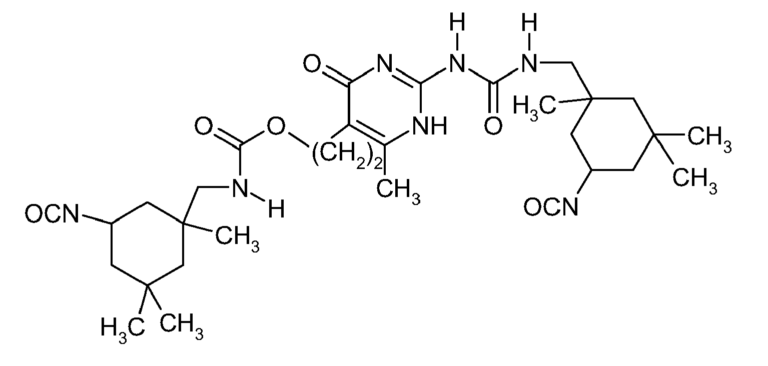
ou encore :

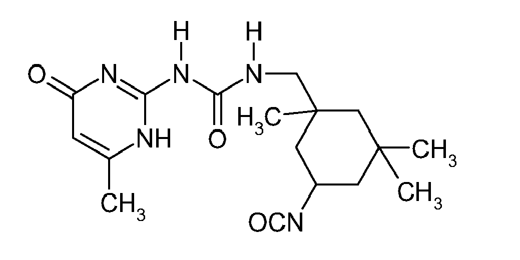

21. Composition selon l'une des revendications 2 à 20, dans laquelle ledit monomères (II) G₂ est choisi parmi les monomères éthyléniques, et notamment parmi:
-i) les (méth)acrylates de formule CH₂=CHCOOR⁴ ou CH₂=C(CH₃)COOR⁴ dans laquelle R⁴ représente :
- un hydrogène,
- un groupe alkyle, linéaire, cyclique ou ramifié, (notamment cycloalkyle ou alkylcycloalkyle), de 1 à 30 atomes de carbone, dans lequel se trouve(nt) éventuellement intercalé(s) un ou plusieurs hétéroatomes choisis parmi O, N, S et P, ledit groupe alkyle pouvant, en outre, être éventuellement substitué par un ou plusieurs substituants choisis parmi les groupes hydroxyle, les atomes d'halogène (Cl, Br, 1 et F), et les groupes Si (R₇)(R₈), où R₇ et R₈, identiques ou différents, représentent un groupe alkyle en C₁ à C₆ ou un groupe phényle.
- un groupe aryle en C₃ à C₂₀ tel que le groupe phényle ;
- un groupe aralkyle ou alkylaryle, en C₄ à C₃₀ (groupe alkyle en C₁ à C₈) tels que 2-phényl-éthyle ou benzyle ;
- un groupe hétérocycloalkyle en C4-C12 contenant un ou plusieurs hétéroatomes choisis parmi O, N, P et S, le cycle étant aromatique ou non; tel qu'imidazole;
- un groupe alkylhétérocycloalkyle en C4-C30, (alkyle C₁-C₈), tels que furfurylméthyle ou tétrahydrofurfurylméthyle,
lesdits groupes aryles, aralkyles pouvant comprendre éventuellement intercalés un ou plusieurs hétéroatomes choisis parmi O, N, S et P, et/ou pouvant être substitués par un ou plusieurs substituants choisis parmi les groupes hydroxyle, les atomes d'halogène et les groupes alkyles C₁-C₄, linéaires ou ramifiés qui eux-mêmes peuvent comprendre intercalés un ou plusieurs hétéroatomes choisis parmi O, N, S et P et/ou qui peuvent être substitués par un ou plusieurs substituants choisis parmi les groupes hydroxyle, les atomes d'halogène (Cl, Br, I et F), et les groupes Si (R₇)(R₈), où R₇ et R₈, identiques ou différents, représentent un groupe alkyle en C₁ à C₆, ou un groupe phényle.
-ii) les (méth)acrylamides de formule CH₂=CHCONR⁶R⁵ ou CH₂=C(CH₃)CONR⁶R⁵ dans laquelle R⁵ et R⁶, identiques ou différents, ont les mêmes significations que pour les groupes R⁴ ci-dessus.
-iii) les monomères vinyliques de formules : CH2=CH-R⁹, CH2=CH-CH2-R⁹ ou CH2=C(CH3)-CH2-R⁹ dans lesquelles R⁹ est un groupe hydroxyle, halogène (Cl
ou F), NH₂, acétamide (-NHCOCH3), -OR₁₀ où R₁₀ représente un groupe phényle ou un groupe alkyle en C1-C12 (éther de vinyle); -OCOR₁₁ (ester de vinyle)
où R₁₁ représente:
(i) un groupe alkyle de C₂ à C₁₂, linéaire ou ramifié,
(ii) un groupe cycloalkyle en C₃ à C₁₂ tel que isobornyle, cyclohexyle,
(iii) un groupe aryle en C₃ à C₂₀ tel que phényle,
(iv) un groupe aralkyle en C₄ à C₃₀ (groupe alkyle en C₁ à C₈) tel que 2-phényléthyle et benzyle,
(v) un groupe hétérocycloalkyle saturé ou non, aromatique ou non, de 4 à 12 chaînons contenant un ou plusieurs hétéroatomes choisis parmi O, N, et S, tel que furfuryle ou tétrahydrofurfuryle,
(vi) un groupe alkylhétérocycloalkyle de C₁ à C₄, tel que furfurylméthyle ou tétrahydrofurfurylméthyle,
lesdits groupes alkyles, cycloalkyles, aryles, aralkyles, hétérocycloalkyles ou alkylhétérocycloalkyles pouvant être éventuellement substitués par un ou plusieurs substituants choisis parmi les groupes hydroxyle, les atomes d'halogène, et les groupes alkyles de C₁ à C₄, linéaires ou ramifiés dans lesquels se trouvent éventuellement intercalés un ou plusieurs hétéroatomes choisis parmi O, N, S et P, lesdits groupes alkyles pouvant, en outre, être éventuellement substitués par un ou plusieurs substituants choisis parmi les groupes hydroxyle, les atomes d'halogène (Cl, Br, I et F) et les groupes Si(R₇)(R₈) où R₇ et R₈, identiques ou différents, représentent un groupe alkyle en C₁ à C₆, ou un groupe phényle.
-iv) les monomères (méth)acrylates, (méth)acrylamides ou vinyliques à groupe fluoré ou perfluoré, tels que le méthacrylate d'éthylperfluorooctyle et les monomères (méth)acryliques, (méth)acrylamides ou vinyliques siliconés.

22. Composition selon la revendication 21, dans laquelle R⁴ est choisi parmi les groupes méthyle, éthyle, propyle, n-butyle, isobutyle, tertiobutyle, hexyle, éthylhexyle, octyle, lauryle, stéaryle, isooctyle, isodécyle, dodécyle; des groupes dérivés d'alkyle (c'est à dire alkyles substitués et/ou interrompus) tels que les groupes hydroxyalkyles en C1-C4 tels que 2-hydroxyéthyle, 2-hydroxybutyle et 2-hydroxypropyle; les groupes alcoxy(C1-4)alkyle(C1-4) tels que méthoxyéthyle, éthoxyéthyle et méthoxypropyle; les groupes cycloalkyles en C3 à C12 tels que isobornyle, t-butylcyclohexyle ou cyclohexyle; ou encore les groupes t-butylbenzyle, phényle, furfurylméthyle, tétrahydrofurfurylméthyle, éthyl-2-perfluorohexyle; les groupes -(OC₂H₄)_{q}-OR, avec q = 5 à 500 et R = H ou alkyle de C₁ à C₃₀, par exemple -POE-méthoxy ou -POE-béhényle.

23. Composition selon l'une des revendications 21 à 22, dans laquelle le monomère (II) est choisi parmi le (méth)acrylamide, le N-éthyl(méth)acrylamide, le N-butylacrylamide, le N-t-butylacrylamide, le N-isopropylacrylamide, le N,N-diméthyl(méth)acrylamide, le N,N-dibutylacrylamide, le N-octylacrylamide, le N-dodécylcacrylamide, l'undécylacrylamide, et le N(2-hydroxypropylméthacrylamide); le vinylcyclohexane, le styrène, la N-vinylpyrrolidone et le N-vinylcaprolactame; l'acétate de vinyle, le propionate de vinyle, le butyrate de vinyle, l'éthylhexanoate de vinyle, le néononanoate de vinyle, le néododécanoate de vinyle; le vinyl méthyl éther, le vinyl éthyl éther, et le vinyl isobutyl éther.

24. Composition selon l'une des revendications 21 à 23, dans laquelle le monomère (II) G2 porte au moins un groupe ionisable.

25. Composition selon l'une des revendications 2 à 24, dans laquelle, dans le monomère (I), :
- le groupement G-L-A-L-G est du type : 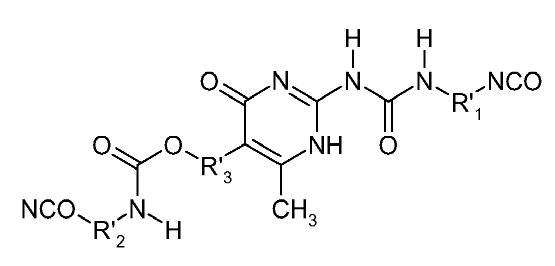 Et/ou
- le groupement A-L-G est du type : 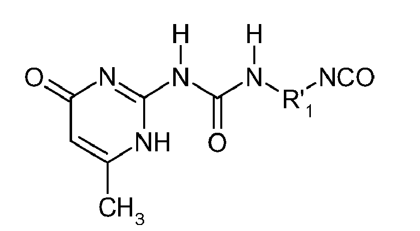 les radicaux R'₁, R'₂ et R'₃, identiques ou différents, représentant un groupe carboné divalent, notamment susceptible d'être choisi parmi 1,2-éthylène, 1,6-héxylène, 1,4-butylène, 1,6-(2,4,4-triméthylhexylène), 1,4-(4-méthylpentylène), 1,5-(5-méthylhexylène), 1,6-(6-méthylheptylène), 1,5-(2,2,5-triméthylhexylène), 1,7-(3,7-diméthyloctylène); -isophorone-, 4,4'-méthylènebiscyclohexylène, tolylène, 2-méthyl-1,3-phénylène, 4-méthyl-1,3-phénylène, 4,4-bisphénylèneméthylène.

26. Composition selon la revendication 25, dans laquelle les radicaux R'₁, R'₂ et R'₃, identiques ou différents, représentent, indépendamment l'un de l'autre, isophorone, -(CH₂)₂-, -(CH₂)₆-, -CH₂CH(CH₃)-CH₂-C(CH₃)₂-CH₂-CH₂, 4,4'-méthylènebiscyclohexylène, 2-méthyl-1,3-phénylène; de préférence R'₁ = R'₂ = -isophorone- et R'₃ = -(CH₂)₂- .

27. Composition selon l'une des revendications précédentes, dans laquelle le groupe ionisable est porté par un bras de liaison L du monomère (I) lorsque L n'est pas une liaison simple, ou par le monomère (II) G₂ lorsqu'il est présent dans le (co)polymère.

28. Composition selon l'une des revendications précédentes, dans laquelle :
- le groupe ionisable représente 0,1 à 50% en poids du poids total du copolymère selon l'invention, de préférence 0,5 à 35% en poids, et notamment 1 à 15% en poids, par rapport au poids total du polymère; et/ou
- le ou les monomères portant le/les groupes ionisables représentent 3 à 20% en poids, de préférence 6 à 17% en poids, voire 8 à 15% en poids, du poids total du copolymère final.

29. Composition selon l'une des revendications précédentes, dans laquelle les groupes ionisables sont choisis parmi :
-i) les groupes anionisables, et leurs sels, tels que les groupes comportant une fonction acide choisie parmi :
- le radical carboxylique : -COOH,
- le radical sulfonique : -SO₃H,
- le radical : -OSO₃H
- le radical phosphonique : -(O)P(OH)₂
- le radical phosphorique : -OP(O)(OH)₂
- ou leurs formes salifiées organiques ou minérales.
-ii) les groupes cationisables, et leurs sels, tels que les groupes comportant une fonction choisie parmi :
-a) les radicaux amine de formule -N(R₁₅)(R₁₆) ou leurs sels organiques ou minéraux, avec R₁₅ et R₁₆ représentant, indépendamment l'un de l'autre :
(i) un atome d'hydrogène,
(ii) un groupement alkyle linéaire, ramifié ou cyclique, saturé ou insaturé, éventuellement aromatique, comprenant de 1 à 30 atomes de carbone et pouvant comprendre 1 à 10 hétéroatomes choisis parmi O, N, S, et P, notamment un groupement méthyle, éthyle, propyle, isopropyle, n-butyle, t-butyle, isobutyle, lauryle, stéaryle;
(iii) un groupement oxyde d'alkylène de formule -(R₁₇O)ᵣR₁₈ avec R₁₇ représentant un alkyle linéaire ou ramifié en C₂-C₄, R₁₈ est l'hydrogène ou un radical alkyle, linéaire ou ramifié, en C₁ à C₃₀ et r est compris entre 1 et 250 inclus ;
(iv) R₁₅ et R₁₆ peuvent former avec l'atome d'azote un cycle saturé ou insaturé, éventuellement aromatique, comprenant au total 5, 6, 7 ou 8 atomes, et notamment 4, 5 ou 6 atomes de carbone et/ou 2 à 4 hétéroatomes choisi parmi O, S et N; ledit cycle pouvant en outre être fusionné avec un ou plusieurs autres cycles, saturés ou insaturés, éventuellement aromatiques, comprenant chacun 5, 6 ou 7 atomes, et notamment 4, 5, 6 ou 7 atomes de carbone et/ou 2 à 4 hétéroatomes choisi parmi O, S et N ;
-b) un groupement -R'₁₅-N-R'₁₆- dans lequel R'₁₅ et R'₁₆ forment avec l'atome d'azote un cycle, saturé ou insaturé, éventuellement aromatique, comprenant au total 5, 6, 7 ou 8 atomes, et notamment 4, 5 ou 6 atomes de carbone et/ou 2 à 4 hétéroatomes choisi parmi O, S et N; ledit cycle pouvant être fusionné avec un ou plusieurs autres cycles, saturés ou insaturés, éventuellement aromatiques, comprenant chacun 5, 6 ou 7 atomes, et notamment 4, 5, 6, 7 ou 8 atomes de carbone et/ou 2 à 4 hétéroatomes choisi parmi O, S et N, par exemple, le groupe ionisable peut constituer un cycle aromatique ou non comportant un groupement amine tertiaire cationisable ou peut représenter un hétérocycle aromatique ou non, contenant un azote tertiaire, cationisable.
-c) un groupement guanidino ou amidino;
-d) les radicaux ammonium quaternaire de formule : -N⁺(R¹²)₃, Z⁻ où R¹² est un radical alkyle, identique ou différent, linéaire ou ramifié, en C₁ à C₂₀ et Z est un atome d'halogène tel que Br, Cl ou bien -OSO₃CH₃ ;
-e) et leurs mélanges.

30. Composition selon l'une des revendications précédentes, dans laquelle les groupes ionisables sont choisis parmi :
- les groupes anionisables suivants : les groupes monovalents -COOH, - CH₂COOH, -(CH₂)₂COOH, -(CH₂)₃COOH, -(CH₂)SO₃H -(CH₂)₂SO₃H, - (CH₂)₃SO₃H, -O(CH₂)₃SO₃H; les groupes divalents -C(COOH)(CH₃)- et -CH₂-C(COOH)(CH₃)-CH₂- ;
- les groupes cationisables suivants : les groupes monovalents -(CH₂)₂-N(CH₃)₂, -N(CH₃)₂, -(CH₂)₃-N(CH₃)₂, -O-(CH₂)₃-N(CH₂CH₃)₂, -(CH₂)₂-N(CH₂CH₃)₂; les groupes divalents -(CH₂)₂-N(CH₃)-(CH₂)₂-et -(CH₂)₃-N(CH₃)-(CH₂)₃-.

31. Composition selon l'une des revendications 2 à 30, dans laquelle le groupement G₁ est porteur de groupes ionisables et est choisi parmi les groupements CH₂=C(COOH)-COO- et HOC(CH₃)(COOH)-.

32. Composition selon la revendication 31, dans laquelle le groupement G1-L-A est choisi parmi :
- le groupement CH₂=C(COOH)-COO-CH₂OCONH(CH₂)₆uréidopyrimidone de formule : 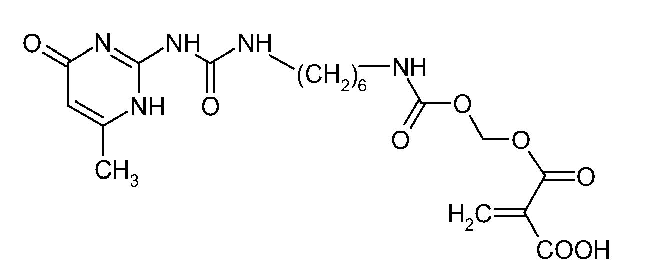
- le groupement HOC(CH₃)(COOH)-CH₂CH₂OC(O)-NH(CH₂)₆uréidopyrimidone de formule :

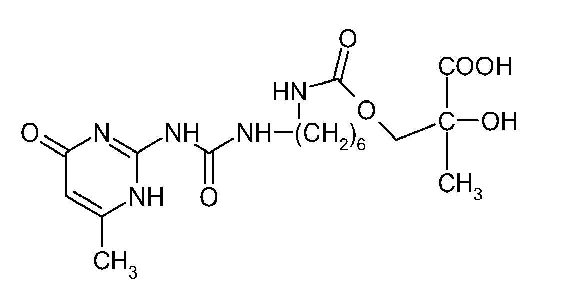

33. Composition selon l'une des revendications 2 à 32, dans laquelle le monomère (II) G₂ comporte un groupe ionisable et est choisi parmi :
- (i) les monomères à insaturation(s) éthylénique(s) comprenant au moins une fonction acide carboxylique (COOH), phosphonique (PO₃H₂) ou sulfonique (SO₃H) comme par exemple ceux de formule suivante :
CH₂=C(R₁₉)-(Z1)_{z1}-(Z2)_{z2}-Y
dans laquelle
- R₁₉ est un atome d'hydrogène ou un radical hydrocarboné, linéaire, cyclique ou ramifié, de type CₚH₂ₚ₊₁, avec p étant un entier compris entre 1 et 12, inclus; notamment R₁₉ peut représenter un radical méthyle, éthyle, propyle, butyle. De préférence, R₁₉ représente l'hydrogène ou un radical méthyle ou éthyle ;
- Z1 est un groupement divalent choisi parmi -COO-, -CONH-, -CONCH₃-, - OCO- et -O-, de préférence Z₁ est choisi parmi -COO- et -CONH- ;
- z₁ est 0 ou 1, de préférence 1 ;
- Z2 est un radical divalent carboné, saturé ou insaturé, éventuellement aromatique, linéaire, ramifié ou cyclique, de 1 à 30 atomes de carbone, pouvant comprendre 1 à 30 hétéroatomes choisis parmi O, N, S, et P;
- z₂ est 0 ou 1, de préférence 1 ; et
- Y est un groupement choisi parmi -COOH, -SO₃H -OSO₃H, -PO(OH)₂ et - OPO(OH)₂.
- (ii) les monomères à insaturation(s) éthylénique(s) comprenant au moins une fonction amine primaire, secondaire ou tertiaire, notamment ceux de formule suivante : 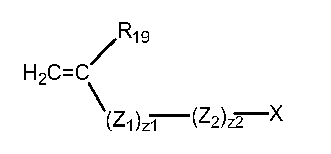 dans laquelle
- R₁₉, Z1, Z2, z₁ et z₂ ont les mêmes significations que dans la formule précédente; et
- X est un groupe de formule -N-R₁₇R₁₈ avec R₁₇ et R₁₈ représentant, indépendamment l'un de l'autre,
(i) un atome d'hydrogène ;
(ii) un groupement alkyle linéaire, ramifié ou cyclique, saturé ou insaturé, éventuellement aromatique, comprenant de 1 à 30 atomes de carbone, pouvant comprendre 1 à 10 hétéroatomes choisis parmi O, N, S, et P; notamment un groupement méthyle, éthyle, propyle, isopropyle, n-butyle, t-butyle, isobutyle, lauryle, stéaryle;
(iii) un groupement oxyde d'alkylène de formule -(R₂₀O)_{y}R₂₁ avec R₂₀ représentant un alkyle linéaire ou ramifié en C₂-C₄, R₂₁ est l'hydrogène ou un radical alkyle, linéaire ou ramifié, en C₂-C₃₀ et y est compris entre 1 et 250 inclus ;
(iv) R₁₇ et R₁₈ peuvent former avec l'atome d'azote un cycle saturé ou insaturé, éventuellement aromatique, comprenant au total 5, 6, 7 ou 8 atomes, et notamment 4, 5 ou 6 atomes de carbone et/ou 2 à 4 hétéroatomes choisi parmi O, S et N; ledit cycle pouvant en outre être fusionné avec un ou plusieurs autres cycles, saturés ou insaturés, éventuellement aromatiques, comprenant chacun 5, 6 ou 7 atomes, et notamment 4, 5, 6 ou 7 atomes de carbone et/ou 2 à 4 hétéroatomes choisi parmi O, S et N ;
ou bien X représente (b) un groupement -R'₁₅-N-R'₁₆- dans lequel R'₁₅ et R'₁₆ forment avec l'atome d'azote un cycle, saturé ou insaturé, éventuellement aromatique, comprenant au total 5, 6, 7 ou 8 atomes, et notamment 4, 5 ou 6 atomes de carbone et/ou 2 à 4 hétéroatomes choisi parmi O, S et N; ledit cycle pouvant être fusionné avec un ou plusieurs autres cycles, saturés ou insaturés, éventuellement aromatiques, comprenant chacun 5, 6 ou 7 atomes, et notamment 4, 5, 6, 7 ou 8 atomes de carbone et/ou 2 à 4 hétéroatomes choisi parmi O, S et N .
- (iii) les monomères de formule : 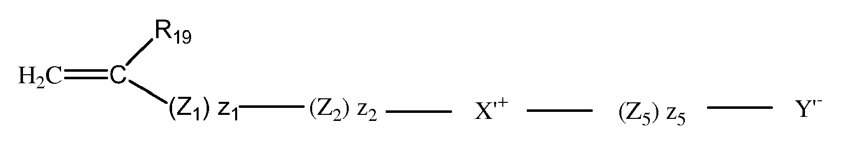 dans laquelle :
- R₁₉, Z₁, Z₂, z₁, z₂, ont la même signification que dans la formule précédente,
- Z₅ a la même signification que celle donnée pour Z₂, mais peut être différent de Z2
- z₅ =0 ou 1,
- X'⁺ est un groupe divalent de formule -N⁺(R₆)(R₇)- avec R₆ et R₇ représentant, indépendamment l'un de l'autre, soit (i) un atome d'hydrogène, soit (ii) un groupement alkyle linéaire, ramifié ou cyclique, éventuellement aromatique, comprenant de 1 à 25 atomes de carbone, pouvant comporter de 1 à 20 hétéroatomes choisis parmi O, N, S et P ; soit (iii) R₆ et R₇ peuvent former avec l'atome d'azote un premier cycle, saturé ou insaturé, éventuellement aromatique, comprenant au total 5, 6, 7 ou 8 atomes, et notamment 4, 5, 6 ou 7 atomes de carbone et/ou 2 ou 3 hétéroatomes choisis parmi O, N et S; ledit premier cycle pouvant être fusionné avec un ou plusieurs autres cycles, saturés ou insaturés, éventuellement aromatiques, comprenant chacun 5, 6, 7 ou 8 atomes, et notamment 4, 5, 6 ou 7 atomes de carbone et/ou 2 ou 3 hétéroatomes choisis parmi O, N et S ; R₆ et R₇ étant notamment choisis parmi l'hydrogène, un groupement méthyle, éthyle, propyle, isopropyle, n-butyle, t-butyle ou isobutyle ; et
- Y'⁻ est un groupement choisi parmi -COO⁻, -SO₃⁻ , -OSO₃⁻, -PO₃²⁻, -OPO₃²⁻.
- (iv) les monomères de formule HX"-B-X"H, dans laquelle :
- X", identique ou différent, représente O, S, NH ou NR, avec R représentant un groupe alkyle en C₁₋₆,
- B représente un radical alkylène ayant de 1 à 6000 atomes de carbone, linéaire, ramifié ou cyclique, saturé ou insaturé, éventuellement aromatique, comprenant éventuellement un ou plusieurs hétéroatomes choisis parmi O, S, P et N, et/ou éventuellement substitué par un ou plusieurs atomes de fluor et/ou silicium;
sous réserve qu'au moins un des radicaux B porte un groupe ionisable.

34. Composition selon la revendication 33, dans laquelle :
-i) Z2 est :
- un radical alkylène tel que méthylène, éthylène, propylène, n-butylène, isobutylène, tertiobutylène, n-hexylène, n-octylène, n-dodécylène, n-octadécylène, n-tétradécylène, n-docosanylène;
- un radical phénylène -C₆H₄-(ortho, méta ou para) éventuellement substitué, par un radical alkyle en C₁-C₁₂ comprenant éventuellement 1 à 8 hétéroatomes choisis parmi O, N, S, et P; ou bien un radical benzylène -C₆H₄-CH₂- éventuellement substitué, par un radical alkyle en C₁-C₁₂ comprenant éventuellement 1 à 8 hétéroatomes choisis parmi O, N, S, et P;
- un radical de formule -CH₂-CH(OH)-, -CH₂-CH₂-CH(OH)-, -CH₂-CH₂-CH(NH₂)-, -CH₂-CH(NH₂)-, -CH₂-CH₂-CH(NHR')-, -CH₂-CH(NHR')-, -CH₂-CH₂-CH(NR'R")-, -CH₂-CH(NR'R")-, -CH₂-CH=CH- avec R' et R" représentant un alkyle linéaire ou ramifié en C₁-C₁₈, notamment méthyle ou éthyle; et/ou
-ii) X constitue un cycle aromatique ou non comportant un groupement amine tertiaire cationisable ou peut représenter un hétérocycle aromatique ou non, contenant un azote tertiaire, cationisable; notamment X est choisi parmi les radicaux de type pyridine, indolyle, isoindolinyle, imidazolyle, imidazolinyle, piperidinyle, pyrazolynyle, pyrazolye, quinoline, pyrazolinyle, pyridinyle, piperazinyle, pyrrolidinyle, quinidinyle, thiazolinyle, morpholine, guanidino, amidino, et leurs mélanges; et/ou
-iii) Z₂ et Z₅ sont sélectionnés parmi les groupements alkylènes linéaires, saturés ou non, ramifiés ou cycliques (aromatiques ou non) comprenant 1-30 atomes de carbone, incluant ou non un ou plusieurs hétéroatomes tels que O, N, S et P. De préférence, Z₂ et Z₅ sont sélectionnés parmi les groupes alkylènes en C₁-C₃₀, phénylène, benzylène, -(CH₂-CH=CH)-, -(CH₂-CH₂-CHOH)- ou -CH₂-CH₂-CH(NR₂₀R₂₁)- avec R₂₀ et R₂₁ sélectionnés parmi H et alkyles en C₁-C₁₈, de préférence, R₂₀ et R₂₁ étant choisis parmi H, CH₃ et C₂H₅. Préférentiellement, Z₂ et Z₅ sont sélectionnés parmi les groupes alkylènes en C₁-C₆, phénylène, benzylène; et/ou
-iv) B est :
- un radical alkylène ayant 1 à 40 atomes de carbone ou cycloalkylène ayant 3 à 16 atomes de carbone, éventuellement substitué par un radical alkyle en C1-C12 et/ou comprenant éventuellement 1 à 8 hétéroatomes choisis parmi O, N, S, F, Si et P; tel que méthylène, éthylène, propylène, n-butylène, isobutylène, tertiobutylène, n-hexylène, n-octylène, n-dodécylène, n-octadécylène, n-tétradécylène, n-docosanylène; ethyl-2-hexyl, cyclohexylène, cyclohexyleméthylène, isophorone ;
- un radical arylène en C1-C30 tel qu'un radical phénylène -C₆H₄-(ortho, méta ou para);
- un radical alkylarylène ou arylalkylène en C₁ à C₃₀, de préférence en C₂ à C₁₂, éventuellement substitué par un radical alkyle en C₁-C₁₂ comprenant éventuellement 1 à 25 hétéroatomes choisis parmi O, N, S, F, Si et P; tel qu'un radical benzylène -C₆H₄-CH₂- éventuellement substitué, par un radical alkyle en C₁-C₁₂ comprenant éventuellement 1 à 8 hétéroatomes choisis parmi O, N, S, F, Si et P;
et B peut comprendre :
- un radical de formule -O-CO-O-, -CO-O-, -OCO -, -O-CO-NH-, anhydride, - NH-CO-NH-, NHCO;
- un radical -Si(R₄)(R₅)O- où R₄ et R₅, identiques ou différents représentent H
ou un radical hydrocarboné, linéaire ou ramifié, cyclique ou non, saturé ou non,
ou aromatique, de préférence un alkyle en C₁-C₁₂ pouvant éventuellement comporter un ou plusieurs, de préférence 1 à 5, hétéroatomes identiques ou différents choisis parmi O, N, S, P, F et Si, de préférence O, N et S ; et/ou
- un radical oxyalkylène ou aminoalkylène, notamment un radical oxyde d'alkylène de formule -(R"O)_{y}R"₁ avec R" représentant un alkyle linéaire ou ramifié en C₂-C₄, R"₁ représentant H ou un radical alkyle, linéaire ou ramifié, en C₁ à C₃₀ et y est compris entre 1 et 500 inclus.

35. Composition selon l'une des revendications 33 à 34, dans laquelle le monomère (II) G2 est choisi parmi, seul ou en mélange, :
a)
l'acide acrylique, l'acide méthacrylique, l'acide crotonique, l'acide itaconique, l'acide fumarique, l'acide maléique, l'acide diacrylique, l'acide diméthylfumarique, l'acide citraconique, l'acide acrylamidopropanesulfonique, l'acide 2-acrylamido 2-méthylpropanesulfonique, l'acide styrène sulfonique, l'acide vinylbenzoïque, l'acide vinylphosphorique, l'acide vinylsulfonique, l'acide vinylbenzène sulfonique, l'acide acrylamidoglycolique de formule CH2=CH-CONHCH(OH)COOH, l'acide vinylphosphonique; le (méth)acrylate de 2-carboxyéthyle, le méthacrylate ou l'acrylate de sulfopropyle (CH₂=C(CH₃)CO₂(CH₂)₃SO₃H), le méthacrylate ou l'acrylate de sulfoéthyle et la vinylméthylsulfone, le 2-(méthacryloyloxy)éthylphosphate de formule CH₂=C(CH₃)COOC₂H₄OP(O)(OH)₂; le maléate de diallyle de formule C₃H₅-CO₂-CH=CH-CO₂-C₂H₅ ; les anhydrides carboxyliques porteurs d'une liaison vinylique tels que l'anhydride maléique, ainsi que leurs sels;
b)
- la 2-vinylpyridine, la 4-vinylpyridine, l'allylamine et l'allylpyridine;
- les (méth)acrylates d'aminoalkyles, tels que les (méth)acrylates de [N,N-di(C₁-C₄) alkylamino](C₁-C₆)alkyle ou les (méth)acrylates de [N-(C₁-C₄)alkylamino](C1-C6)alkyle et notamment le (méth)acrylate de N,N-diméthylaminoéthyle, le (méth)acrylate de N,N-diéthylaminoéthyle, le (méth)acrylate de 2-aminoéthyle, le (méth)acrylate de 2-(N-tertbutylamino)éthyle;
- les (méth)acrylamides d'aminoalkyles, tels que les (méth)acrylamides de [N,N-di(C₁-C₄)alkylamino](C₁-C₆)alkyle ou les (méth)acrylamides de [N-(C₁-C₄)alkyle amino](C₁-C₆)alkyle, et notamment le (méth)acrylamide de N,N-diméthylaminopropyle, le (méth)acrylamide de N,N-diméthylaminoéthyle; le (méth)acrylamide de 3-aminopropyle;
- la vinylamine, la vinylimidazole, le 2-(diéthylamino)éthylstyrène;
- la N-vinylimidazole, la N-vinyl-2-méthylimidazole, la N-vinylcarbazole;
ainsi que leurs sels et/ou leurs formes quaternisées.
c)
la N,N-dimethyl-N-(2-methacryloyloxyethyl)-N-(3-sulfopropyl) ammonium bétaine, la N,N-dimethyl-N-(3-methacrylamidopropyl)-N-(3-sulfopropyl) ammonium bétaine et la 1-(3-sulfopropyl)-2-vinylpyridinium bétaine.
d)
l'acide diméthylolpropionique, l'acide diméthylaminopropionique, la N-éthylsulfoniquediméthanolamine, la N-éthylsulfoniquediéthanolamine, l'acide diol benzène sulfonique, la diaminopyridine, la N-méthyldiéthanolamine, la N-éthyldiéthanolamine, la N-tert-butyldiéthanolamine, et leurs mélanges.

36. Composition selon l'une des revendications précédentes, dans laquelle les (co)polymères se présentent sous l'une des structures suivantes:
- (co)polymère linéaire et fonctionnalisé en α, ω avec des groupes de jonction (A);
- (co)polymère linéaire comportant plus de deux groupes de jonction, situés dans la chaîne et/ou à l'une ou aux deux extrémités et/ou en ramifications; et/ou
- (co)polymère ramifié avec des groupes de jonction dans la chaîne et/ou en ramification et/ou à l'une ou aux deux extrémités.

37. Composition selon l'une des revendications précédentes, dans laquelle le (co)polymère est choisi parmi, sous réserve qu'il comprenne un groupe ionisable :
- les polyuréthanes, polyurées, polyesters aliphatiques ou aromatiques, polyamides aliphatiques ou aromatiques, ou leurs copolymères, comme par exemple des polyuréthanne/urée, polyester/amide, polyester/polyuréthanne/urée.
- les (co)polymères éthyléniques et notamment vinyliques, comme par exemple les copolymères (méth)acryliques, les copolymères (méth)acrylamides, les copolymères allyliques, les copolyoléfines tels que les polydiènes hydrogénés ou non et leurs mélanges; et en particulier les copolymères vinyliques/(méth)acrylates, vinyliques/(méth)acrylamides, vinyliques/(méth)acrylates/méthacrylamides, oléfiniques/vinyliques et les (méth)acrylates /(méth)acrylamides.
- les (co)polymères obtenus par ouverture de cycle, comme les polyéthers de type polyoxyde d'éthylène, polyoxyde de propylène, et leurs copolymères polyoxyde d'éthylène/polyoxyde de propylène; les polylactides, les polyesters comme la polycaprolactone; les polyoxazolines tels que la poly(2-méthyloxazoline), ou la poly(2-éthyloxazoline);
- les polycaprolactames et les polypyrrolidones.
- les (co)polymères de siloxane, tels que par exemple les polydiméthylsiloxanes (PDMS), et polyméthylphénylsiloxanes ;
- les polythioéthers, les polycarbonates, les polyacétals, les perfluoropolyéthers,
- les (co)polymères obtenus par métathèse comme le poly(norbornène) et ses copolymères,
- les copolymères de ces différents types de polymères, comme par exemple les copolymères polysiloxane/polyoxyde d'éthylène, polysiloxane/polyuréthanne/urée, poly(éthylène-butylène)/polyuréthanne et polybutadiène hydrogéné/polyuréthanne;
- leurs sels et leurs dérivés ; et leurs mélanges.

38. Composition selon l'une des revendications précédentes, dans laquelle le (co)polymère présente une masse moléculaire moyenne en nombre (Mn) comprise entre 1000 et 3 000 000, notamment 5000 et 1 000 000, et de préférence entre 8000 et 500 000.

39. Composition selon l'une des revendications précédentes, dans laquelle les (co)polymères sont solubles et/ou dispersibles dans le milieu de la composition et notamment dans les milieux aqueux, les huiles carbonées, les huiles de silicone et/ou les solvants cosmétiques, tels que les alcanols, notamment l'éthanol et les esters d'alkyle tels que les acétates d'alkyle.

40. Composition selon l'une des revendications précédentes, dans laquelle les (co)polymères sont présents à raison de 0,01 à 90% en poids de matière sèche, notamment 0,05 à 70% en poids, voire 0,1 à 60% en poids, encore mieux 1 à 50% en poids, par rapport au poids total de la composition.

41. Composition selon l'une des revendications précédentes, dans laquelle le milieu physiologiquement acceptable comprend un composé choisi parmi l'eau, les alcools, les polyols, les esters, les cétones liquides, les éthers de propylène glycol liquides à température ambiante, les éthers liquides à 25°C, les alcanes liquides à 25°C, les aldéhydes liquides à 25°C, les composés cycliques aromatiques liquides à 25°C, les huiles carbonées, les huiles de silicone, les huiles de silicone fluorées, les cires, les gommes, les tensioactifs, les épaississants, les gélifiants hydrophiles ou lipophiles, les actifs cosmétiques hydrophiles ou lipophiles, les conservateurs, les antioxydants, les parfums, les agents nacrants, les charges, les neutralisants, les polymères autres que ceux définis préalablement et notamment filmogènes, les émulsionnants et les co-émulsionnants, les pigments, les matières colorantes, les nacres, les émulsionnants et les co-émulsionnants, et leurs mélanges.

42. Composition selon l'une des revendications précédentes, se présentant sous la forme d'une solution, dispersion ou suspension aqueuse, alcoolique ou hydroalcoolique ou d'une solution huileuse, éventuellement épaissies ou gélifiées; d'une émulsion huile-dans-eau, eau-dans-huile, ou multiple, de consistance liquide ou semi liquide du type lait ou bien de consistance molle de type crème; d'un gel aqueux ou anhydre, d'une mousse; un gel huileux ou émulsionné; une dispersion de vésicules notamment lipidiques; une lotion biphase ou multiphase; d' un spray; ou de toute autre forme cosmétique.

43. Composition selon l'une des revendications précédentes, se présentant sous la forme d'un produit de soin, de nettoyage et/ou de maquillage de la peau du corps ou du visage, des lèvres et des cheveux, d'un produit solaire ou autobronzant, d'un produit d'hygiène corporelle, d'un produit capillaire, notamment de soin, de nettoyage, de coiffage ou de coloration des cheveux.

44. Composition selon l'une des revendications précédentes, se présentant :
- sous le forme d'une composition capillaire choisie parmi les shampooings, des après-shampooings, des gels de coiffage ou de soin, des lotions ou crèmes de soin, des conditionneurs, des lotions de mise en plis, des lotions pour le brushing, des compositions de fixation et de coiffage telles que les laques ou spray; les produits de coloration capillaire; ou les compositions pour permanente, défrisage, ou décoloration; les compositions à rincer, à appliquer avant ou après une coloration, une décoloration, une permanente ou un défrisage ou encore entre les deux étapes d'une permanente ou d'un défrisage.
- sous la forme d'une composition de soin, notamment hydratant, pour la peau, les lèvres et/ou les phanères, ou sous forme d'une composition de nettoyage de la peau, par exemple un produit démaquillant ou un gel pour le bain ou la douche.
- sous la forme d'un produit de soin, non coloré, destiné à traiter la peau et notamment à l'hydrater, la lisser, la dépigmenter, la nourrir, la protéger des rayons solaires, ou lui conférer un traitement spécifique.
- sous forme d'une composition d'hygiène corporelle notamment sous forme de produit déodorant, anti-transpirant, ou d'une composition dépilatoire.
- sous la forme d'un produit de maquillage, en particulier coloré, de la peau du corps ou du visage, ou des cheveux, en particulier un fond de teint, présentant éventuellement des propriétés de soin, un blush, un fard à joues ou à paupières, un produit anti-cerne, un eye-liner; un produit de maquillage des lèvres comme un rouge à lèvres, présentant éventuellement des propriétés de soin, un brillant à lèvres, les crayons à lèvres; un produit de maquillage des phanères comme les ongles, les cils en particulier sous forme d'un mascara pain, les sourcils et les cheveux; un produit de tatouage temporaire de la peau du corps.

45. Procédé cosmétique de traitement, notamment de maquillage, de soin, de nettoyage ou de coloration, des matières kératiniques, notamment de la peau du corps ou du visage, des ongles, des cheveux, des poils et/ou des cils, comprenant l'application sur lesdites matières d'une composition cosmétique telle que définie selon l'une quelconque des revendications 1 à 44.

46. Procédé pour améliorer à la fois la persistance d'au moins un effet apporté après dépôt par une composition cosmétique et l'adhésion de la composition appliquée sur les matières kératiniques, et pour permettre également une élimination rapide, totale et sélective du dépôt, qui consiste à ajouter à la composition une quantité efficace d'au moins un (co)polymère tel que défini dans l'une quelconque des revendications 1 à 38.
